Europäisches Patentamt

European Patent Office

Office européen des brevets

(11) Publication number: **0 235 112 B1**

# EUROPEAN PATENT SPECIFICATION

(45) Date of publication of patent specification: **13.12.95**  (51) Int. Cl.⁶: **C12N 15/76**, C12N 1/20,
C12N 15/31

(21) Application number: **87870026.9**

(22) Date of filing: **26.02.87**

(54) **The gal operon of streptomyces**

(30) Priority: **28.02.86 US 834706**
**30.01.87 US 9419**

(43) Date of publication of application:
**02.09.87 Bulletin 87/36**

(45) Publication of the grant of the patent:
**13.12.95 Bulletin 95/50**

(84) Designated Contracting States:
**AT BE CH DE ES FR GB GR IT LI LU NL SE**

(56) References cited:
**EP-A- 0 187 630**

**GENE, vol. 40, no. 2/3, 1985, pages 191-201,
Elsevier Science Publishers, Amsterdam,
NL; M.E. BRAWNER et al.: "Characterization
of Streptomyces promoter sequences using
the Escherichia coli galactokinase gene"**

**NUCLEIC ACIDS RESEARCH, vol. 13, no. 6,
1985, pages 1841-1853, IRL Press Ltd, Oxford,
GB; C. DEBOUCK et al.: "Structure of the
galactokinase gene of Escherichia coli, the
last (?) gene of the gal operon"**

(73) Proprietor: **SMITHKLINE BEECHAM CORPORA-
TION**
**P.O. Box 7929**
**1 Franklin Plaza**
**Philadelphia**
**Pennsylvania 19101 (US)**

(72) Inventor: **Adams, Craig W.**
**1773 South Buena Vista**
**Corona**
**California 92720 (US)**
Inventor: **Fornwald, James Allan**
**104 Burnside Avenue**
**Norristown**
**Pennsylvania 19403 (US)**
Inventor: **Brawner, Mary Ellen**
**126 Valley Stream Circle**
**Wayne**
**Pennsylvania 19087 (US)**
Inventor: **Schmidt, Francis John**
**1404 Doris Drive**
**Columbia**
**Missouri 65201 (US)**

ABSTRACTS OF THE ANNUAL MEETING OF THE AMERICAN SOCIETY FOR MICROBIOLOGY, vol. 84, no. 0, 1984, abstract no. H98; W. BURNETT et al.: "Transcriptional and transletional regulatory elements in the streptomyces - lividans beta-gal operon"

(74) Representative: **Giddings, Peter John, Dr.**
**SmithKline Beecham plc**
**Corporate Intellectual Property**
**SB House**
**Great West Road**
**Brentford, Middlesex TW8 9BD (GB)**

**Description**

CROSS REFERENCE TO RELATED APPLICATIONS

This application is a continuation-in-part of Serial Number 834,706, filed February 28, 1986, which is pending.

BACKGROUND OF THE INVENTION

This invention relates to a recombinant DNA molecule comprising the Streptomyces gal operon.

Hodgson, J. Gen. Micro., 128, 2417-2430 (1982), report that Streptomyces coelicolor A3(2) has a glucose repression system which allows repression at the level of transcription of the arabinose uptake system, one of the glycerol uptake systems, and also repression of the galactose uptake system in wild type strains. There is no report in Hodgson of actual galactose metabolism by S. coelicolor A3(2).

Okeda et al. Mol. Gen. Genet., 196, 501-507 (1984), report that glucose kinase activity, 2-deoxyglucose-sensitivity, glucose utilization and glucose repression were all restored to S. coelicolor A3(2) glk (glucose kinase) mutants transformed by a 3.5 kb DNA fragment which contained the glk gene cloned from S. coelicolor into a phage vector.

Seno et al., Mol. Gen. Genet., 193, 119-128 (1984), report the glycerol (gyl) operon of Streptomyces coelicolor, and state that such operon is substrate-inducible and catabolite-repressible.

Debouck et al., Nuc. Acids. Res., 13(6), 1841-1853 (1985), report that the gal operon of E. coli consists of three structurally contiguous genes which specify the enzymes required for the metabolism of galactose, i.e., galE (uridine diphosphogalactose-4-epimerase), galT (galactose-1-phosphate uridyltransferase) and galK (galactokinase); that such genes are expressed from a polycistronic mRNA in the order E, T, K; that the expression of the promotor distal gene of the operon, galK, is known to be coupled translationally to the galT gene immediately preceding it; that such transnational coupling results from a structural overlap between the end of the galT coding sequence and the ribosome binding region of galK; and that the translational coupling of galT and galK ensures the coordinate expression of these genes during the metabolism of galactose.

SUMMARY OF THE INVENTION

This invention relates to a recombinant DNA molecule comprising a Streptomyces gal operon P2 promotor expression unit, or P2 promotor or any functional derivative thereof as well as a recombinant DNA molecule comprising a Streptomyces gal operon P1 promotor, P1 promotor regulated region or the entire gal operon or any regulatable and functional derivative thereof.

This invention provides a recombinant DNA molecule comprising the Streptomyces lividans gal operon located on a ca. 9kb HindIII-SphI fragment of S. lividans 1326 chromosomal DNA or any Streptomyces derivative thereof which functions in substantially the same way as the naturally occurring Streptomyces gal operon in terms of regulatable production of the galT, galE and galK gene products; a foreign functional DNA molecule operatively linked to such operon; a recombinant DNA vector comprising such DNA molecule, and, optionally, additionally comprising a replicon; a method of preparing a host cell transformed with such vector; the transformed host prepared by such method; a method of expressing such functional DNA sequence which comprises cultivating such transformed host under suitable conditions such that the functional DNA sequence is expressed; and to a method of regulating the expression of such functional DNA sequence which comprises cultivating such transformed host under conditions which regulate such expression.

This invention also provides a recombinant DNA molecule comprising the Streptomyces lividans gal operon P2 promotor expression unit located on a ca. 9kb HindIII-SphI fragment of S. lividans 1326 chromosomal DNA having an NruI site, where the P2 transcription start site is located approximately 1.25 Kb downstream of the NruI site, or any Streptomyces derivative thereof which functions in substantially the same way as the naturally occurring unit in terms of production of the galE and galK gene products; a foreign functional DNA molecule operatively linked to such unit; a recombinant DNA vector comprising such DNA molecule, and, optionally, additionally comprising a replicon; a method of preparing a host cell transformed with such vector; the transformed host prepared by such method; and to a method of expressing such functional DNA sequence which comprises cultivating such transformed host under suitable conditions such that the functional DNA sequence is expressed.

3

This invention also provides a recombinant DNA molecule comprising the Streptomyces lividans gal operon P1 promotor regulated region located on a ca. 9kb HindIII-SphI fragment of S. lividans 1326 chromosomal DNA having an NruI site, where the P1 transcription start site is located approximately 0.10 Kb downstream of the NruI site, or any Streptomyces derivative thereof which functions in substantially the same way as the naturally occurring region in terms of regulatable production of the galT, galE and galK gene products; a foreign functional DNA molecule operatively linked to such DNA molecule; a recombinant DNA vector comprising such DNA molecule, and, optionally, additionally comprising a replicon; a method of preparing a host cell transformed with such vector; the transformed host prepared by such method; a method of expressing such functional DNA sequence which comprises cultivating such transformed host under suitable conditions such that the functional DNA sequence is expressed; and to a method of regulating the expression of such functional DNA sequence which comprises cultivating such transformed host under conditions which regulate such expression.

This invention also provides a recombinant DNA molecule comprising the Streptomyces lividans gal operon P1 promotor located on a ca. 9kb HindIII-SphI fragment of S. lividans 1326 chromosomal DNA having an NruI site, where the P1 transcription start site is located approximately 0.10 Kb downstream of the NruI site, or any Streptomyces derivative thereof which functions in substantially the same way as the naturally occurring promoter in terms of enabling the binding of RNA polymerase thereto and transcription of a functional DNA sequence operatively linked to such promoter; a foreign functional DNA molecule operatively linked to such DNA molecule; a recombinant DNA vector comprising such molecule, and, optionally, additionally, comprising a replicon;a method of preparing a host cell transformed with such vector; the transformed host prepared by such method; a method of expressing such functional DNA sequence which comprises cultivating such transformed host under suitable conditions such that the functional DNA sequence is expressed; and to a method of regulating the expression of such functional DNA sequence which comprises cultivating such transformed host under conditions which regulate such expression.

This invention also relates to a recombinant DNA molecule comprising the Streptomyces lividans gal operon P2 promotor located on a ca. 9kb HindIII-SphI fragment of S. lividans 1326 chromosomal DNA having an NruI site, where the P2 transcription start site is located approximately 1.25 Kb downstream of the NruI site, or any Streptomyces derivative thereof which functions in substantially the same way as the naturally occurring promoter in terms of enabling the binding of RNA polymerase thereto and transcription of a functional DNA sequence operatively linked to such promoter; a foreign functional DNA molecule operatively linked to such region; a recombinant DNA vector comprising such DNA molecule, and optionally, additionally comprising a replicon; a method for preparing a host cell transformed with such vector; the transformed host prepared by such method; and to a method of expressing such functional DNA sequence which comprises cultivating such transformed host under suitable conditions such that the functional DNA sequence is expressed.

BRIEF DESCRIPTION OF THE DRAWINGS

Figure 1 represents a restriction endonuclease map of the Streptomyces lividans 1326 galactose (gal) operon and indicates approximate locations for structural genes and promoters within the operon.

Figure 2 represents a restriction endonuclease map of plasmid pK21.

Figure 3 represents a comparison of the restriction endoninclease maps of the S. lividans gal operon and a restriction fragment containing the S. coelicolor galK gene.

DETAILED DESCRIPTION OF THE INVENTION

It has now been discovered that the Streptomyces genome contains a operon for the metabolism of galactose (i.e., a gal operon) which comprises three structural genes (galT, galE and galK) and two promoters (P1 and P2). The galT gene product is known as galactose-1-phosphate uridyltransferase (transferase), the galE gene product is known as uridine diphosphogalactose-4-epimerase (epimerase), and the galK gene product is known as galactose-1-kinase (galactokinase). The function of the gene products of galT, galE and galK in galactose metabolism in Streptomyces is explained by the following diagram:

1. galactose + ATP galactokinase
galactose-1-phosphate + ADP
2. galactose-1-phosphate + UDP-glucose transferase
UDP-galactose + glucose-1-phosphate

3. UDP-galactose epimerase UDP-glucose

By the term "promoter" is meant any region upstream of a structural gene which permits binding of RNA polymerase and transcription to occur.

By the term "structural gene" is meant a coding sequence for a polypeptide which serves to be the template for the synthesis of mRNA.

By the term "operon" is meant a group of closely linked genes responsible for the synthesis of one or a group of enzymes which are functionally related as members of one enzyme system. An operon comprises an operator gene, a number of structural genes (equivalent to the number of enzymes in the system) and a regulator gene. By "operator" or "operator gene is meant a DNA sequence which controls the biosynthesis of the contiguous structural gene(s) within an operon. By "regulator gene" is meant a gene which controls the operator gene in an operon through the production of a repressor which can be either active (enzyme induction) or inactive (enzyme repression). The transcription of the structural gene(s) in an operon is switched on or off by the operator gene which is itself controlled in one or more of three ways: 1) in inducible enzyme systems, the operator is switched off by a repressor produced by the regulator gene and which can be inactivated by some metabolite or signal substance (an inducer) coming from elsewhere in the cell or outside the cell, so that the presence of the inducer results in the operon becoming active; or 2) in repressed enzyme systems, the operator is switched off by a repressor-corepressor complex which is a combination of an inactive repressor produced by the regulator gene with a corepressor from elsewhere, so that the presence of the corepressor renders the operon inactive; or 3) in activated gene systems, the promoter is switched on by an activator produced by a regulator gene which can be activated by some metabolic or signal substance.

The Streptomyces gal operon is naturally present in the Streptomyces genome.

By the term "Streptomyces gal operon" is meant that region of the Streptomyces genome which comprises the P1 promoter, P2 promoter, galT, galE and galK structural genes and any other regulatory regions required for transcription and translation of such structural genes.

By the term "regulatory region" is meant a DNA sequence, such as a promoter or operator, which regulates transcription of a structural gene.

The following model is suggested for gene expression within the Streptomyces gal operon. The P1 promoter is a galactose inducible promoter (i.e., it is induced in the presence of galactose and repressed in the presence of glucose). According to S1 data, the P2 promoter is constitutive, i.e., it is "turned on" regardless of the presence or absence of galactose or any other carbon source.

A cosmid library was constructed for Streptomyces lividans 1326 DNA by using cosmid pJW357 (which encodes the ability to replicate in both Streptomyces and E. coli). This library was then transfected into E. coli K21 which is a derivative of the E. coli strain MM294 which contained a bacteriophage P1 transduced galactokinase (galK) mutation. Transfected cells were plated under media conditions which select for both the presence of the cosmid and the presence of an active galK gene. Weakly positive colonies were isolated and the cosmid DNA derived from these colonies was transformed into the K21 strain. These transformations yielded two cosmids which consistently produced positive growth with galactose as the only carbon source. These galK$^+$ cosmids were then transformed into a Streptomyces host (i.e., Streptomyces lividans 1326-12K) which had been isolated by the inventors of the subject invention as unable to grow on medium in which galactose was the only carbon source by using 2-deoxy-galactose selection [see, Brawner et al., Gene, 40 191 (1985), in press]. Under conditions which differentiate strains able and unable to produce galactokinase, only one of the cosmids caused the Streptomyces lividans 1326-12K host to become galK$^+$. Further studies have demonstrated that this cosmid encodes a gene with galactokinase activity. Additional studies, including DNA sequence analysis and protein studies demonstrate that this Streptomyces gene shares homology with the E. coli and yeast galactokinase genes. Regulation studies indicate that the cosmid encoded galactokinase gene regulated in the same manner as the chromosome encoded gene.

A. S. lividans gal operon was originally isolated from a ca. 9 kilobase (Kb) region of Streptomyces lividans 1326. The ca. 9 Kb region of Streptomyces lividans 1326 containing the Streptomyces gal operon has been mapped substantially as follows in Table A. By "substantially" is meant (i) that the relative positions of the restriction sites are approximate, (ii) that one or more restriction sites can be lost or gained by mutations not otherwise significantly affecting the operon, and (iii) that additional sites for the indicated enzymes and, especially for enzymes not tested, may exist. The restriction enzymes used herein are commercially available. All are described by Roberts, Nuc. Acids. Res., 10(5): p117 (1982).

5

TABLE A

| Map Position | Restriction Enzyme | Location (kb) |
|---|---|---|
| 1 | HindIII | -.40 |
| 1a | NruI | 0 |
| 2 | BglII | .75 |
| 3 | EcoRI | 1.05 |
| 4 | PvuII | 1.15 |
| 5 | MluI | 2.30 |
| 6 | PvuII | 2.80 |
| 7 | EcoRI | 4.00 |
| 8 | PvuII | 4.10 |
| 8a | SacI | 4.25 |
| 9 | PvuII | 5.00 |
| 10 | XhoI | 5.50 |
| 11 | BamHI | 5.80 |
| 12 | BamHI | 6.50 |
| 13 | MluI | 6.90 |
| 13a | PvuII | 7.20 |
| 14 | MluI | 7.80 |
| 15 | BamHI | 8.00 |
| 16 | SphI | 8.30 |

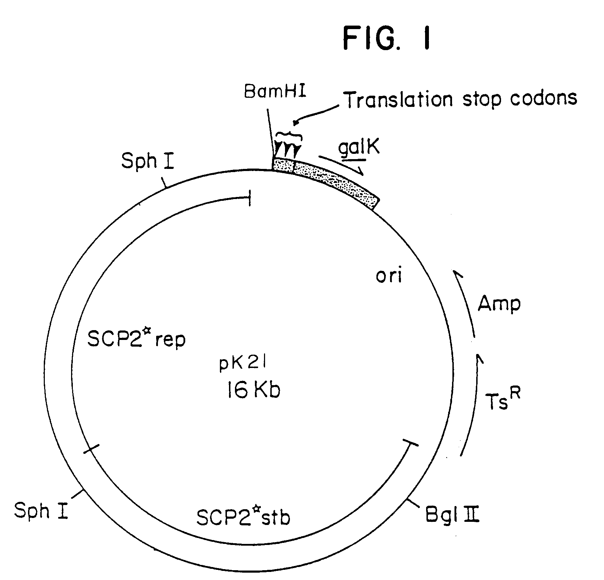

Figure 1 represents a restriction endonuclease map of the Streptomyces lividans 1326 gal operon and indicate locations for structural genes (galT, galE and galK) and promoters (P1 and P2) comprised within the operon.

Referring to Table A and Figure 1, the location of the promoters and structural genes of the Streptomyces lividans 1326 gal operon are mapped substantially as follows in Table B:

TABLE B

| | Location (Kb) |
|---|---|
| P1 transcription start site | .10 |
| galT translation initiation codon | .15 |
| P2 transcription start site | 1.25 |
| galE translation initiation codon | 1.50 |
| galK translation initiation codon | 2.40 |
| 3' end of galK message | 3.60 |

Microorganisms of the genus Streptomyces have historically been used as a source of antibiotics for the pharmaceutical industry. Consequently, the technical skills necessary to scale-up the production of biological products using Streptomyces as the vehicle for the production of such products are presently available. However, before Streptomyces can be used as a vehicle for the production of bioactive molecules using the new recombinant DNA technologies, there is a need to define regulatory elements in Streptomyces analogous to those which have proved useful in E. coli. These regulatory elements include ribosomal binding sites and regulated transcriptional elements.

The existence of a galE, galT or galK gene or gene product or gal operon in Streptomyces has not been previously reported. The instant invention, i.e., the cloning of the Streptomyces gal operon, enables construction of regulatable expression/cloning vectors in Streptomyces, other actinomycetes, and other host organisms. Furthermore, the instant invention led to the discovery that the Streptomyces gal operon is polycistronic. Perhaps the most important feature of the cloning of the Streptomyces gal operon is the observation that there are sequences essential for regulation of the Streptomyces galK gene. Direct analogy to the initial use of the lac promoter from E. coli as an expression system can be made. In fact, Brosius et al., Proc. Natl. Acad. Sci. USA, 81, 6929-6933 (1984), utilized the regulatory elements of the E. coli lac promoter to regulate the exceptionally strong E. coli ribosomal promoters. Because it is likely that the Streptomyces gal operon ribosomal promoters are also exceptionally strong, such promoters enable the construction of regulatable expression vectors which will be very useful in Streptomyces, other actinomycetes, and other host organisms. The instant invention also enabled the unexpected discovery that the 2-deoxygalactose selection which has been used in E. coli to select for galK mutants also operates in Streptomyces to select for galK mutants [see, Brawner et al., Gene 40, 191 (1985), in press]. This observation, combined with the ability to clone the Streptomyces galK gene and the promoter and regulatory regions required for its transcription and translation on a cosmid, as described herein, allows the direct insertion of any structural gene into the chromosomally located galK gene of Streptomyces by homologous recombination. This manipulation will allow molecular biologists to stably insert DNA fragments of interest into the Streptomyces chromosome. Such an approach will allow researchers to tag or mark a Streptomyces strain of interest or to insert expression cassettes into the organism without the need of maintaining an antibiotic selection such as that presently required by most Streptomyces expression vectors.

This invention relates to a recombinant DNA molecule comprising the Streptomyces lividans gal operon or any regulatable and functional Streptomyces derivative thereof.

By "regulatable and functional derivative" is meant any derivative of the Streptomyces gal operon which functions in substantially the same way as the naturally occurring Streptomyces gal operon in terms of regulatable production of the galT, galE and galK gene products. Such derivatives include partial sequences of the gal operon, as well as derivatives produced by modification of the gal operon coding sequence. Techniques for modifying the gal operon which are known in the art include, for example, treatment with chemical mutagens, irradiation or direct genetic engineering, such as by inserting, deleting or substituting nucleic acids by the use of enzymes or recombination techniques. The naturally occurring Streptomyces gal operon can be isolated from any galactose utilizing Streptomyces strain by employing the techniques described herein. Numerous strains of various Streptomyces species are publicly available from many sources. For example, the American Type Culture Collection, Rockville, Maryland, U.S.A. has approximately 400 different species of Streptomyces available to the public. The ability of a particular strain of Streptomyces to utilize galactose can be readily determined by conventional techniques, such as by growing such strain on a medium containing galactose as the sole carbon source. The preferred Streptomyces species from which to isolate a gal operon include S. lividans, S. coelicolor, S. azureus and S. albus, S. carzinostaticus, S. antifibrinolyticus and S. longisporus. S. lividans is most preferred. The Streptomyces gal operon, and smaller portions thereof, is useful as a nucleic acid probe to obtain homologous sequences from other cells and organisms. The Streptomyces gal operon is also useful as a selection marker in an appropriate host mutant, and for providing regulatory elements. By "appropriate host mutant" is meant a host which does not utilize galactose because it (a) does not contain a gal operon or (b) contains a nonfunctional gal operon, or (c) contains a defect within a homologous structural gene or regulatory region comprised by the Streptomyces gal operon such as a defective P1 promoter, P2 promoter, galT gene, galK gene and/or galE gene. Thus, a recombinant DNA molecule (comprising the Streptomyces gal operon and a foreign functional DNA sequence operatively linked thereto), which can be prepared by conventional techniques, can be transformed into an appropriate host mutant by conventional techniques for incorporation into the host genome by homologous recombination to enable regulatable expression of the foreign functional DNA sequence without the need of maintaining an expensive antibiotic selection. Such operon may therefore also be incorporated on recombinant DNA expression vectors for regulatable expression of a foreign functional DNA sequence operatively linked to such operon in an appropriate host mutant transformed with such vector without the need of maintaining an expensive antibiotic selection. Such operon is also useful for transforming those cells, viruses and microorganisms, such as strains of Streptomyces, other actinomycetes, and other prokaryotic organisms, such as gal⁻ E. coli strains, which do not utilize galactose into galactose utilizing strains. Such transformation may have pleiotrophic effects on the transformed host. By the term "functional DNA sequence" is meant any discrete region of DNA derived directly or indirectly from Streptomyces or any other source which functions in a host organism transformed therewith as a gene expression unit, structural gene, promoter or a regulatory region. Preferred functional DNA sequences include those coding for polypeptides of pharmaceutical importance, such as, but not limited to, insulin, growth hormone, tissue plasminogen activator, alpha -1-antitrypsin or antigens used in vaccine production. By the term "foreign functional DNA sequence" is meant a functional DNA sequence not derived from the Streptomyces gal operon coding region.

This invention also relates to a recombinant DNA molecule comprising the Streptomyces gal operon P2 promoter expression unit or any functional derivative thereof. By the term "P2 promoter expression unit" is meant that region of the Streptomyces gal operon comprising the Streptomyces gal operon P2 promoter, galE and galK structural genes and any other regulatory regions required for transcription and translation of such structural genes. By "functional derivative" is meant any derivative of the Streptomyces gal operon P2 promoter expression unit which functions in substantially the same way as the naturally occurring region in terms of production of the Streptomyces gal operon galE and galK gene products. Such derivatives include partial sequences of the Streptomyces gal operon P2 promoter expression unit, as well as derivatives produced by modification of the Streptomyces gal operon P2 promoter expression unit coding sequence. Techniques for effecting such modification are known in the art, and some have been outlined above. The naturally occurring Streptomyces gal operon P2 promoter expression unit can be isolated from the naturally occurring Streptomyces gal operon by conventional techniques. The Streptomyces gal operon P2 expression unit is useful as a selection marker in an appropriate host mutant and for providing regulatory elements. By "appropriate host mutant" is meant a host which does not utilize galactose because it contains a defect within a homologous structural gene or regulatory region comprised by the Streptomyces P2 promoter expression unit such as a defective P2 promoter, galE gene aid/or galK gene. Thus, a recombinant DNA molecule (comprising the Streptomyces gal operon P2 promoter expression unit and a foreign functional DNA sequence operatively linked thereto), which can be prepared by conventional techniques, can be transformed into an appropriate host mutant by conventional techniques for incorporation into the host genome by homologous recombination to enable constitutive expression of the foreign functional DNA sequence without the need of maintaining an expensive antibiotic selection. Such expression unit may also be incorporated on recombinant DNA expression vectors for constitutive expression of foreign functional DNA sequences. The Stretomyces gal operon P2 promoter expression unit is also useful for complementation of an appropriate host mutant which can then be used for constitutive expression of a foreign functional DNA sequence operatively linked to such expression unit in an appropriate host mutant transformed with such vector without the need of maintaining an expensive antibiotic selection.

This invention also relates to a recombinant DNA molecule comprising the Streptomyces gal operon P1 promoter regulated region or any regulatable and functional derivative thereof. By the term "P1 promoter regulated region" is meant that region of the Streptomyces gal operon comprising the Streptomyces gal operon P1 promoter, galT, galE and galK structural genes and any other regulatory regions required for transcription and translation of such structural genes. By "regulatable and functional derivative" is meant any derivative of the Streptomyces gal operon P1 promoter regulated region which functions in substantially the same way as the naturally occurring region in terms of regulatable production of the Streptomyces gal operon galT, galE and galK gene products. Such derivatives include partial sequences of the Streptomyces gal operon P1 promoter regulated region, as well as derivatives produced by modification of the Streptomyces gal operon P1 promoter regulated region coding sequence. Techniques for effecting such modification are known in the art, and some have been outlined above. The naturally occurring Streptomyces gal operon P1 promoter regulated region can be isolated from the naturally occurring Streptomyces gal operon by conventional techniques, such as by excising the P2 promoter from the naturally occurring Streptomyces gal operon or inactivating the P2 promoter by a point mutation or by inserting a foreign DNA sequence within the promoter. The Streptomyces gal operon P1 promoter regulated region is useful for the utilities outlined above for the Streptomyces gal operon.

This invention also relates to a recombinant DNA molecule comprising the Streptomyces gal operon P2 promoter or any functional derivative thereof. By "functional derivative" is meant any derivative of the Streptomyces gal operon P2 promoter which functions in substantially the same way as the naturally occurring P2 promoter in terms of enabling the binding of RNA polymerase thereto and transcription of a functional DNA sequence operatively linked to such promoter. Such derivatives include partial sequences of the Streptomyces gal operon P2 promoter, as well as derivatives produced by modification of the gal operon P2 promoter coding sequence. Techniques for effecting such modification are known in the art, and some have been outlined above. The naturally occurring Streptomyces gal operon P2 promoter can be isolated from the naturally occurring Streptomyces gal operon by conventional techniques. A recombinant DNA molecule (comprising the Streptomyces gal operon P2 promoter and a foreign functional DNA sequence operatively linked thereto), which can be prepared by conventional techniques, can be transformed into an appropriate host mutant by conventional techniques for incorporation into the host genome by homologous recombination to enable constitutive expression of the foreign functional DNA sequence. The Streptomyces gal operon P2 promoter is also useful for incorporation into recombinant DNA expression vectors for constitutive expression of a foreign functional DNA sequence operatively linked thereto in viruses and eukaryotic or prokaryotic cells or organisms, especially in Streptomyces or other actinomycetes, transformed with such vector.

This invention also relates to a recombinant DNA molecule comprising the Streptomyces gal operon P1 promoter or any regulatable and functional derivative thereof. By "regulatable and functional derivative" is meant any derivative of the Streptomyces gal operon P1 promoter which functions in substantially the same way as the naturally occurring P1 promoter in terms of enabling the binding of RNA polymerase thereto and regulating the transcription of a functional DNA sequence operatively linked to such promoter. Such derivatives include partial sequences of the Streptomyces gal operon P1 promoter, as well as derivatives produced by modification of the gal operon P1 promoter coding sequence. Techniques for effecting such modification are known in the art, and some have been outlined above. The naturally occurring Streptomyces gal operon P1 promoter can be isolated from the naturally occurring Streptomyces gal operon by conventional techniques. A recombinant DNA molecule (comprising the Streptomyces gal operon P1 promoter and a foreign functional DNA sequence operatively linked thereto), which can be prepared by conventional techniques, can be transformed into an appropriate host mutant by conventional techniques for incorporation into the host genome by homologous recombination to enable regulatable expression of the foreign functional DNA sequence. The Streptomyces gal operon P1 promoter is also useful for incorporation into recombinant DNA expression vectors for regulatable expression of a foreign functional DNA sequence operatively linked thereto in viruses and eukaryotic or prokaryotic cells or organisms, especially Streptomyces or other actinomycetes, transformed with such vector.

This invention also relates to a recombinant DNA molecule comprising the Streptomyces gal operon galE, galT or galK gene, or any functional derivative thereof. By "functional derivative" is meant any derivative of the Streptomyces gal operon galE, galT or galK gene which functions in substantially the same way as the naturally occurring gene in terms of production of an active galE, galT, or galK type gene product. Such derivatives include partial sequences of the Streptomyces gal operon galE, galT, or galK gene, as well as derivatives produced by modification of the gal operon sequence. Techniques for effecting such modification are known in the art, and some have been outlined above. The naturally occurring Streptomyces gal operon galE, galT and/or galK gene can be isolated from the naturally occurring Streptomyces gal operon by conventional techniques. The Streptomyces gal operon galE, galT and/or galK gene can be used as a selection marker in an appropriate host mutant. By "appropriate host mutant is meant a host which does not utilize galactose because it contains a defect within a homologous galE, galT and/or galK gene. Thus, a recombinant DNA molecule (comprising the Streptomyces gal operon galE, galT and/or galK gene and a foreign functional DNA sequence, both of which are operatively linked to appropriate regulatory region), which can be prepared by conventional techniques, can be transformed into an appropriate host mutant by conventional techniques for incorporation into the host genome by homologous recombination to enable detection of transformants without the need of maintaining an expensive antibiotic selection. Likewise, a recombinant DNA vector comprising the Streptomyces gal operon galE, galT and/or galK gene and a foreign functional DNA sequence, both of which are operatively linked to appropriate regulatory regions, as well as a replicon, can be transformed into an appropriate host mutant by conventional techniques to enable detection of transformants without the need of maintaining an expensive antibiotic selection. The Streptomyces gal operon galE, galK and/or galT gene is also useful for complementation of an appropriate host mutant.

The Streptomyces gal operon galE gene is also useful for providing a ribosome binding site and initiation codon which can be fused to a foreign functional DNA sequence to enable the expression of such coding sequence when incorporated into an appropriate expression vector and transformed into an appropriate host. If such foreign functional DNA sequence is fused to the galE gene ribosome binding site and initiation codon in a recombinant DNA expression vector comprising the Streptomyces gal operon P2 promoter expression unit, or the entire gal operon, such DNA sequence will be constitutively expressed when such vector is transformed into an appropriate host organism. If such DNA sequence is fused to the galE gene ribosome binding site and initiation codon in a recombinant DNA expression vector comprising the Streptomyces gal operon P2 promoter regulated region, expression of such DNA sequence can be regulated when such vector is transformed into an appropriate host organism by controlling the presence or absence of galactose or glucose.

The Streptomyces gal operon galT gene is also useful for providing a ribosome binding site and initiation codon which can be fused to a foreign functional DNA sequence to enable the expression of such coding sequence when incorporated onto an appropriate expression vector and transformed into an appropriate host. If such DNA sequence is fused to the galT gene ribosome binding site and initiation codon in a recombinant DNA expression vector comprising the Streptomyces gal operon P1 promoter regulated region, or the entire gal operon, expression of such coding sequence can be regulated in a host transformed with such vector as outlined above.

This invention also relates to a recombinant DNA vector comprising a replicon, Streptomyces gal operon, or a functional and regulatable derivative thereof, and a foreign functional DNA sequence operatively linked to such operon. Such vector can be prepared by conventional techniques. The replicon employed should be one known for its ability to stably and extrachromosomally, maintain a vector in the

9

host organism which is to be the host transformed with the vector.

This invention also relates to a transformed host microorganism comprising a recombinant DNA vector wherein said vector contains a replicon, the Streptomyces gal operon, or a functional and regulatable derivative thereof, and a foreign functional DNA sequence operatively linked to such operon; and to the method of preparing such host which comprises transforming an appropriate host microorganism with such vector. Appropriate host microorganisms which may be employed in the method of this invention include viruses, and eukaryotic and prokaryotic cells or organisms, especially actinomycetes, such as those of the genus Streptomyces. The most preferred host microorganisms belong to the genus Streptomyces. Preferred species of Streptomyces include Streptomyces lividans, S. coelicolor, S. azuraeus and S. albus. Transformation of such host microorganism with such vector can be accomplished using conventional techniques such as the method of Chater et al., Curr. Top. Micro. Imm., 96, 69-95 (1982). This invention also related to a method of expressing the functional DNA sequence contained by such transformed host of this invention which comprises cultivating such transformed host under suitable conditions such that the functional DNA sequence is expressed. By "suitable conditions" is meant those conditions which will allow the host to grow and which enable the expression of the functional DNA sequence. Such suitable conditions can be determined by one of skill in the art using conventional techniques and will depend on various factors, such as the host organism employed and the functional DNA sequence to be expressed. This invention is also related to a method of regulating the expression of the functional DNA sequence contained by such transformed host which comprises cultivating a transformed host containing such functional DNA sequence under appropriate conditions such that its expression is regulatable. By "appropriate conditions" is meant those conditions which enable the Streptomyces gal operon (and thus the foreign functional DNA sequence) to be regulatable. By "regulatable" is meant responsive to the presence of galactose or its metabolites and the presence of glucose or its metabolites in the growth media of the transformed host cell. Such regulation can be carried out by addition or deletion of galactose or glucose to the transformed host's culture medium. The optimal levels of galactose and/or glucose for up or down-regulation of the expression of the foreign functional DNA coding sequence by the transformed host of this invention can be readily determined by one of skill in the art using conventional techniques.

This invention also relates to a recombinant DNA vector comprising a replicon, a Streptomyces gal operon P2 promoter expression unit, or a functional derivative thereof, and a foreign functional DNA sequence operatively linked to such unit. Such a vector can be prepared by conventional techniques. The replicon employed should be one known for its ability to stably, and extrachromosomally, maintain a vector in the host organism which is to be transformed with the vector.

This invention also relates to a transformed host microorganism comprising a recombinant DNA vector wherein said vector contains a replicon, the Streptomyces gal operon P2 promoter expression unit, or a functional derivative thereof, and a foreign functional DNA sequence operatively linked to such unit; and to the method of preparing such host which comprises transforming an appropriate host microorganism with such vector. By the term "operatively linked" is meant that a functional DNA sequence is transcriptionally or translationally linked to an expression control sequence (i.e., the Streptomyces gal operon, P2 promoter expression unit, P1 promoter regulated region, P1 promoter or P2 promoter) in such a way so that the expression of the functional DNA sequence is under control of the expression control sequence. Thus, for example, a foreign functional DNA sequence can be transcriptionally or translationally linked to the Streptomyces gal operon by inserting such operon within the Streptomyces gal operon P1 or P2 promoter transcript. By the term "replicon" is meant that region of DNA on a plasmid which functions to maintain, extrachromosomally, such plasmid in a host microorganism or cell transformed therewith. It has also been discovered that the Streptomyces gal operon, and smaller portions thereof, is useful as a nucleic acid probe to obtain homologous sequences from other cells and organisms. Appropriate host microorganisms which may be employed in the method of this invention include any virus or eukaryotic or prokaryotic cell or organism, especially any actinomycetes such as those of the genus Streptomyces. The most preferred host microorganisms belong to the genus Streptomyces. Preferred species of Streptomyces include Streptomyces lividans, S. coelicolor, S. azuraeus and S. albus. Transformation of such host microorganism with such vector can be accomplished using conventional techniques such as the method of Chater et al., Curr. Top. Micro. Imm., 96, 69-95 (1982). This invention also related to a method of expressing the functional DNA sequence contained by such transformed host of this invention which comprises cultivating such transformed host under suitable conditions such that the functional DNA sequence is expressed. By "suitable conditions" is meant those conditions which will allow the host to grow and which enable the expression of the functional DNA sequence. Such suitable conditions can be determined by one of skill in the art using conventional techniques and will depend on various factors, such as the host organism employed and the functional DNA sequence to be expressed.

This invention also relates to a recombinant DNA vector comprising a replicon, a Streptomyces gal operon P1 promoter regulated region, or a functional and regulatable derivative thereof, and a foreign functional DNA sequence operatively linked to such region. Such a vector can be prepared by conventional techniques. The replicon employed should be one known for its ability to stably and extrachromosomally maintain a vector in the host organism which is to be the host transformed with the vector.

This invention also relates to a transformed host microorganism comprising a recombinant DNA vector wherein said vector contains a replicon, a Streptomyces gal operon P1 promoter regulated region, or a functional and regulatable derivative thereof, and a foreign functional DNA sequence operatively linked to such region; and to the method of preparing such host which comprises transforming an appropriate host microorganism with such vector. Appropriate host microorganisms which may be employed include any virus or eukaryotic or prokaryotic cell or organism especially actinomycetes such as those of the genus Streptomyces. The most preferred host microorganisms belong to the genus Streptomyces. Preferred species of Streptomyces include Streptomyces lividans, S. coelicolor, S. azuraeus and S. albus. Transformation of such host microorganism with such vector can be accomplished using conventional techniques such as the method of Chater et al., Curr. Top. Micro. Imm., 96, 69-95 (1982). This invention also related to a method of expressing the foreign functional DNA sequence contained by such transformed host of this invention which comprises cultivating such transformed host under suitable conditions such that the functional DNA sequence is expressed. By "suitable conditions" is meant those conditions which will allow the host to grow and which enable the expression of the functional DNA sequence. Such suitable conditions can be determined by one of skill in the art using conventional techniques and will depend on various factors, such as the host organism employed and the functional DNA sequence to be expressed. This invention also related to a method of regulating the expression of the functional DNA sequence contained by such transformed host which comprises cultivating a transformed host containing such functional DNA sequence under appropriate conditions such that its expression is regulatable. By "appropriate conditions is meant those conditions which enable the Streptomyces gal operon P1 promoter regulated region (and thus the foreign functional DNA sequence) to be regulatable. By "regulatable" is meant responsive to the presence or absence of galactose or its metabolites and the presence or absence of glucose or its metabolites in the growth media of the transformed host cell. Such regulation can be carried out by addition or deletion of galactose or glucose to the transformed host's culture medium.

This invention also relates to a recombinant DNA vector comprising a replicon, a Streptomyces gal operon P2 promoter, or a functional derivative thereof, and a foreign functional DNA sequence operatively linked to such promoter. Such a vector can be prepared by conventional techniques. The replicon employed should be one known for its ability to stably and extrachromosomally maintain a vector in the host organism which is to be the host transformed with the vector.

This invention also relates to a transformed host microorganism comprising a recombinant DNA vector wherein said vector contains a replicon, a Streptomyces gal operon P2 promoter, or a functional derivative thereof, and a foreign functional DNA sequence operatively linked to such region; and to the method of preparing such host which comprises transforming an appropriate host microorganism with such vector. Appropriate host microorganisms which may be employed include actinomycetes such as those of the genus Streptomyces. The most preferred host microorganisms belong to the genus Streptomyces. Preferred species of Streptomyces include Streptomyces lividans, S. coelicolor, S. azuraeus and S. albus. Transformation of such host microorganism with such vector can be accomplished using conventional techniques such as the method of Chater et al., Curr. Top. Micro. Imm., 96, 69-95 (1982). This invention also related to a method of expressing the foreign functional DNA sequence contained by such transformed host of this invention which comprises cultivating such transformed host under suitable conditions such that the functional DNA sequence is expressed. By "suitable conditions" is meant those conditions which will allow the host to grow and which enable the expression of the functional DNA sequence. Such suitable conditions can be determined by one of skill in the art using conventional techniques and will depend on various factors, such as the host organism employed and the functional DNA sequence to be expressed.

This invention also relates to a recombinant DNA vector comprising a replicon, Streptomyces gal operon P1 promoter, or any regulatable and functional derivative thereof, and a foreign functional DNA sequence operatively linked to such region. Such a vector can be prepared by conventional techniques. The replicon employed should be one known for its ability to stably and extrachromosomally maintain a vector in the host organism which is to be the host transformed with the vector.

This invention also relates to a transformed host microorganism comprising a recombinant DNA vector wherein said vector contains a replicon, the Streptomyces gal operon P1 promoter, or any regulatable and functional derivative thereof, and a foreign functional DNA sequence operatively linked to such region; and to the method of preparing such host which comprises transforming an appropriate host microorganism with such vector. Appropriate host microorganisms which may be employed include viruses or prokaryotic or eukaryotic cells or organisms, especially actinomycetes such as those of the genus Streptomyces. The most preferred host microorganisms belong to the genus Streptomyces. Preferred species of Streptomyces include Streptomyces lividans, S. coelicolor, S. azuraeus and S. albus. Transformation of such host microorganism with such vector can be accomplished using conventional techniques such as the method of Chater et al., Curr. Top. Micro. Imm., 96, 69-95 (1982). This invention also relates to a method of expressing the foreign functional DNA sequence contained by such transformed host of this invention which comprises cultivating such transformed host under suitable conditions such that the functional DNA sequence is expressed. By "suitable conditions" is meant those conditions which will allow the host to grow and which enable the expression of the functional DNA sequence. Such suitable conditions can be determined by one of skill in the art using conventional techniques and will depend on various factors, such as the host organism employed and the foreign functional DNA sequence to be expressed. This invention also relates to a method of regulating the expression of the functional DNA sequence contained by such transformed host which comprises cultivating a transformed host containing such foreign functional DNA sequence under appropriate conditions such that its expression is regulatable. By "appropriate conditions" is meant those conditions which enable the gal operon P1 promoter (and thus the functional DNA sequence) to be regulatable. By "regulatable" is meant responsive to the presence or absence of galactose or its metabolites and the presence of glucose or its metabolites in the growth media of the transformed host cell. Such regulation can be carried out by addition or deletion of galactose or glucose to the transformed host's culture medium.

## EXAMPLES

In the following Examples, specific embodiments of the invention are more filly disclosed. These Examples are intended to be illustrative of the subject invention and should not be construed as limiting its scope. In all Examples, temperature is in degrees Centigrade (°C).

By utilizing conventional methods, such as those outlined in the following Examples, one of skill in the art can isolate the gal operon from any galactose utilizing strain of Streptomyces. Furthermore, by utilizing techniques similar to those employed herein to isolate the Streptomyces gal operon, one of skill in the art can attempt to use the Streptomyces gal operon to isolate a gal operon from other galactose utilizing other strains of Streptomyces, especially S. coelicolor, S. azuraeus, S. albus and other S. lividans strains.

Molecular genetic manipulations and other techniques employed in the following Examples are described in Hopwood et al., Genetic Manipulation of Streptomyces: A Laboratory Manual, John Innes Foundation, Norwich, England (1985).

## ABBREVIATIONS

In the following Examples, the following abbreviations may be employed:

LB: 10 grams (g) tryptone, 5 g yeast extract, 5g NaCl

MBSM (modified MBSM): See, Brawner et al., Gene, 40, 191 (1985) (in press) MOPS: (3)-N-morpholino-(proprane-sulfonic acid)

YEME + MgCl$_2$ + Glycine: [per liter(l)] 3 g yeast extract, 5 g peptone, 3 g malt extract, 10 g glucose, 10 g MgCl$_2$'' 62H$_2$O, 340 g sucrose.

SL: Mix together (NH$_4$)$_2$SO$_4$ (1g/l); L-asparagine (2 g/l); K$_2$HPO$_4$ (9 g/l); NaH$_2$PO$_4$ (1 g/l) for 0.2% agar and autoclave. Then mix with yeast extract (20 g/l), MgCl$_2$ (5 g/l); CuCl$_2$ (0.1 g/l); Trace elements [20 ml/l - include ZnCl$_2$-40 mg/l; FeCl$_3$''6H$_2$O (200 mg/l); CuCl$_2$''2H$_2$O (10 mg/l); NaB$_4$O$_7$''10H$_2$O (10 mg/l); (NH$_4$)-$_6$MO$_7$O$_{24}$'' 4 H$_2$O(10 mg/l)] filter and sterilize.

YEME (Ym base): (per liter) yeast extract (3g); peptone (5g); malt extract (3g); MgCl$_2$''6H$_2$O (2g)

Ymglu: YEME + glucose (10g)

Ymgal: YEME + galactose (10g)

BACTERIAL STRAINS

In the following Examples, the following strains of E. coli are employed:

| CGSC Strain #[a] | Strain Designation | Sex | Chromosomal Markers |
|---|---|---|---|
| 4473 (galE⁻) | W3109 | F⁻ | galE9,[b]g⁻;IN(rrnD-rrnE)I |
| 4467 (galT⁻) | W3101 | F⁻ | galT22[b]g⁻;IN(rrnD-rrnE)I |
| 4498 (galE⁻) | PL-2 | Hfr | thi-1, relA1, 921E28,g⁻,spoT1 |

[a]CGSC Strain # is the stock number designated for such strain by the E. coli Genetic Stock Center of the Department of Human Genetics, Yale University School of Medicine, 333 Cedar Street, P.O. Box 3333, New Haven, Connecticut, 06510, U.S.A.
[b]galE9 is the old Lederberg gal9; galT22 is the old Lederberg gal$_1$.

S1 ANALYSIS

S1 analysis is used to identify the 5' end of RNAs and the length of a RNA of interest. In the following Examples, S1 analysis refers to S1 experiments carried out according to the method of Weaver et al., Nucl. Acids Res., 7, 1175 (1979) and Berk et al., Proc. Natl. Acad. Sci. USA, 75, 1214 (1978).

EXAMPLE I

A. CLONING OF A STREPTOMYCES LIVIDANS GALACTOKINASE GENE.

Streptomyces lividans strain 1326 is described by Bibb et al., Mol. Gen. Genetics, 184, 230-240 (1981) and was obtained from D. A. Hopwood, John Innes Foundation, Norwich, England. Streptomyces lividans strain 1326 and S. lividans strain 1326 containing the pIJ6 plasmid were deposited in the Agricultural Research Culture Collection, Peoria, Illinois, U.S.A., on June 1, 1982, under accession numbers NRRL 15091 and 15092, respectively.

High molecular weight chromosomal DNA was isolated from Streptomyces lividans strain 1326 according to the method of Maniatis et al., "Molecular Cloning. A Laboratory Manual", Cold Spring Harbor Laboratory (1982) and was size fractionated on a 10-40% sucrose gradient (See, Maniatis et al., cited above, p. 284-285). Fractions of 18-24 kilobase (Kb) pairs were combined and dialyzed exhaustively against 10 mM Tris-HCl/1 mM EDTA (pH 8). Cosmid shuttle vector pJW357 was employed to clone such fractionated chromosomal DNA in its entirety. pJW357 was constructed by fusing pDPT6 cut with PstI to pIJ350 cut with PstI. pIJ350 is described in Kieser et al., Mol. Gen. Genet., 185, 223-238 (1982). pDPT6 is a tetracycline and chloramphenicol resistant, pBR322-based E. coli cosmid cloning vector described in Taylor et al., U.S. Patent No. 4,476,227. pJW357 has a unique EcoRI site in the chloramphenicol resistance gene and a unique BamHI site in the Tc$^R$ (tetracycline) resistance gene. pJW357 was digested with BamHI, dephosphorylated with alkaline phosphatase, and ligated to the fractionated chromosomal DNA described above.

The ligation product was packaged into bacteriophage heads (using the in vitro packaging system described by Maniatis et al., cited above, p. 264-265) and transfected into E. coli strain K21 which is a galK⁻ derivative of E. coli MM294. The transformation culture was grown for two hours in LB and for an additional two hours in LB with 25 ug/ml chloramphenicol, washed three times with equal volumes of M9 media [see, Miller, "Experiments in Molecular Genetics", Cold Spring Harbor Laboratory (1972)] without a carbon source, and plated onto M9 agar [supplemented with proline, histidine, arginine, isoleucine, leucine, saline and .5% galactose; See, Adams et al., Biochem. Biophys. Res. Comm., 89(2), 650-58 (1979)] with 30 mg/ml chloramphenicol. Twenty plates were spread with approximately 200 transformants per plate. After three days incubatiott at 37° C, no transformants were detected. The minimal plates were then sprayed with nicotinic acid to 5 ug/ml to supplement the nicotinic acid requirement of E. coli strain K21, and the incubation was continued for 3 more days at 37° C and for 2 additional days at room temperature. After such incubation, the surviving colonies were patched to both MacConkey galactose agar (MAC-GAL) [See, Miller et al., cited above] with 30 ug/ml chloramphenicol and to M63 minimal agar [See, Miller et al., cited above] supplemented with .5% galactose, 5 ug/ml nicotinic acid, 5 ug/ml thiamine and 30 ug/ml chloramphenicol. Only two colonies contained cosmid DNA that transformed E. coli K21 to a galK⁺ phenotype. Such cosmids were designated as pSLIVGAL-1 and pSLIVGAL-2. Both colonies were light red on MAC-GAL (i.e., they were galK+) and also grew on the M63 medium.

Plasmids pSLIVGAL-1 and pSLIVGAL-2 were isolated from the two galK+ colonies described above and were transformed, according to the method of Chater et al., Curr. Top. Micro. Imm., 96, 69-95 (1982), into Streptomyces lividans strain 1326-12K (a galK deficient strain isolated after UV mutagenesis of S. lividans strain 1326, See, Brawner et al., Gene, 40, 191 (1985), (in press). Plasmid encoded complementation of the S. lividans 1326-12K (galK−) host was tested by observing growth of spores plated on MBSM-gal-thiostrepton according to the method of Brawner et al., Gene, 40, 191 (1985) (in press). pSLIVGAL-2 showed no detectable complementation of the Streptomyces 1326-12K host.

Cell extracts were prepared from cultures grown in SL medium supplemented with 1% glucose or galactose and 10 )g/ml thiostrepton. The extracts were analyzed for galactokinase production by immunoblot analysis (see, Brawner et al., Gene, 40, 191 (1985), in press) using rabbit antisera prepared against E. coli galactokinase. The protein detected by immunoblot analysis was the approximate size of E. coli galK. Such protein appeared in galactose supplemented cultures of Streptomyces at levels several fold higher than in glucose cultures.

B. MAPPING OF THE S. LIVIVANS GALK REGION WITHIN A COSMID.

The galK region of the pSLIVGAL1 and pSLIVGAL2 cosmids, prepared as described above, was identified by cloning random fragments from the cosmids into a pUC18 derivative [See, Norrander et al., Gene, 26, 101-106 (1983)] and scoring complementation of E. coli strain MM294 (galK−) on MAC-GAL medium. The cosmid clone was partially digested with Sau3AI (using conditions which maximized the yield of 2 to 4 kilobase fragments), and the products of this reaction were ligated into the BglII site of pUC18-TT6, a derivative of pUC18 constructed by insertion of the following synthetic DNA sequence into the BamHI site of pUC18:

$$5'GATCAGATCTTGATCACTAGCTAGCTAG\ 3'$$

$$3'\quad\ \ \ TCTAGAACTAGTGATCGATCGATCCTAG\ 5'$$

Twelve galK+ clones (red on MAC-GAL) were screened for size. One clone, designated as plasmid pSAU10, was the smallest and had an insert size of approximately 1.4 Kb.

In contrast to colonies containing pSLIVGAL1, the pUC clones were very red on MAC-GAL medium, indicating an increased production of galactokinase. The most likely explanation for the increased enzyme level was that the S. lividans galK gene was now being transcribed by an E. coli promoter which was stronger than the upstream promoter on the cosmid.

The insert of pSAU10 was isolated as an EcoRI to HindIII fragment (these sites flank the insert region of pUC18-TT6) for use as a probe for the S. lividans galK gene. The chromosomal DNA used in the cloning was restricted with EcoRI plus MluI and BamHI plus BglII, and then blotted according to the method of Southern, J. Mol. Biol., 98, 503 (1975). The pSAU10 fragment was nick translated and hybridized to the blot. The probe identified a 1.3 kb EcoRI-MluI fragment and a 5 kb BamHI-BglII fragment in the chromosomal digests. When this data was compared to the map of the cosmid insert, the location of the galK gene (between map positions 5 and 7, See Table A) was confirmed.

C. DNA SEQUENCING OF THE S. LIVIDANS GAL OPERON.

The Streptomyces lividans gal operon was sequenced by chain termination [(See, Sanger et al., Proc. Nat'l Acad. Sci., U.S.A., 74, 5463 (1977)] and chemical cleavage [See, Maxam and Gilbert, Methods in Enzymology, 65, 499 (1980)]. The initial sequences of galK were derived from Sau3AI and SalI fragments of the insert of pSAU6 (a 2.3 Kb sibling of pSAU10) shotgun cloned into the BamHI and SalI sites (respectively) of M13 mp 10 [See, Messing, Methods in Enzymology, 101, 20 (1983)]. Amino acid sequences of the S. lividans galT, galE and galK genes were predicted by computer, and further analyzed by comparison with amino acid sequences of the E. coli and or S. cerevisiae galactokinase, gal-1-phosphate uridyltransferase and UDP-4-epimerase enzymes. The sequences of these proteins were predicted by computer analysis using the total or partial DNA sequence of the genes which encode the gal enzymes [see, Debouck et al., Nuc Acids. Res., 13(6), 1841-1853 (1985), and Citron and Donelson, J. Bacteriology, 158, 269 (1984)]. Some homology was found between the inferred protein sequence for the S. lividans galK, galT, galE gene products and their respective E. coli and/or S. cerevisiae gene products.

The complete DNA sequence of the S. lividans gal operon is shown in Table 1. Included in Table 1 are the transcription start sites for the operon's promoters and the predicted amino acid sequences of the galT, galE and galK gene products.

## TABLE 1
### TRANSLATED SEQUENCE OF STREPTOMYCES LIVIDANS GALACTOSE OPERON

```
        -120          -110          -100          -90           -80           -70
         •             •             •             •             •             •
  CTA CGC CTC CGC GTT CAG TAA TTG AAC ACT TTT GGT GAT GAA CTT TGT TTG ATT GTC


        -60           -50           -40           -30           -20
         •             •             •             •             •
  ATG TGA CAG CGG CGT GGT GGG TTG TGA TGT GTT ATG TTT GAT TGT GTT GGA TGA TTG
                                                              ˙galP1

       -10            1            10            20            30            40
         •             •             •             •             •             •
  ACG GGC GTC CTG GTG ACT CAT GGG TGG GTG CAG AGG AGT GCG GCA GTG AAG AAG ACC
                  Met Thr His Gly Trp Val Gln Arg Ser Ala Ala Val Lys Lys Thr
                  galT
        50            60            70            80            90           100
         •             •             •             •             •             •
  TCG ACC CGG CTG GCC GAC GGC CGT GAG CTG GTC TAC TAC GAC CTG CGC GAC GAC ACC
  Ser Thr Arg Leu Ala Asp Gly Arg Glu Leu Val Tyr Tyr Asp Leu Arg Asp Asp Thr

       110           120           130           140           150
         •             •             •             •             •
  GTG CGC GAC GCC CTG GAC CGC CGT CCG CTC GAG CGG ACC GTC ACC ACG TCC GAG GTG
  Val Arg Asp Ala Val Asp Arg Arg Pro Leu Glu Arg Thr Val Thr Thr Ser Glu Val

  160           170           180           190           200           210
   •             •             •             •             •             •
  CGA CGC GAC CCG CTG CTC GGC GAC TCC GCG CCG TCG CGC CTC GCA CCG GCA GGG GCG
  Arg Arg Asp Pro Leu Leu Gly Asp Ser Ala Pro Ser Arg Leu Ala Pro Ala Gly Ala

       220           230           240           250           260           270
         •             •             •             •             •             •
  CAC CTA CCA TCC GCC GGC CGA CCA GTG CCC GCT GTG CCc GTC GGA CGG GGA ACG GCT
  His Leu Pro Ser Ala Gly Arg Pro Val Pro Ala Val Pro Val Gly Arg Gly Thr Ala

       280           290           300           310           320           330
         •             •             •             •             •             •
  GAG CGA GAT CCG GCC TAT GAC GTG GTG GTC TTC GAC AAT CGC TTT CCC TCG CTC GCC
  Glu Arg Asp Pro Ala Tyr Asp Val Val Val Phe Glu Asn Arg Phe Pro Ser Leu Ala
```

Table 1 - (cont'd)

```
                340           350           360           370           380
                 •             •             •             •             •
 GGT GAC TCC GGG CGC TGC GAG GTC GTC TGC TTC ACC TCC GAC CAC GAC GCC TCC TTC
 Gly Asp Ser Gly Arg Cys Glu Val Val Cys Phe Thr Ser Asp His Asp Ala Ser Phe

     390           400           410           420           430           440
      •             •             •             •             •             •
 GCC GAC CTG AGC GAG GAG CAG GCC CGG CTC GTC GTC GAC GCC TGG ACG GAC CGC ACC
 Ala Asp Leu Ser Glu Glu Gln Ala Arg Leu Val Val Asp Ala Trp Thr Asp Arg Thr

         450           460           470           480           490           500
          •             •             •             •             •             •
 TCC GAG CTG TCC CAT CTG CCC TCC GTT GAA CAG GTG TTC TGC TTC GAG AAC CGG GGC
 Ser Glu Leu Ser His Leu Pro Ser Val Glu Gln Val Phe Cys Phe Glu Asn Arg Gly

             510           520           530           540           550
              •             •             •             •             •
 GCC GAG ATC GGG GTG ACG CTG GGT CAC CCC CAC GGG CAG ATC TAC GCC TAC CCG TTC
 Ala Glu Ile Gly Val Thr Leu Gly His Pro His Gly Gln Ile Tyr Ala Tyr Pro Phe

     560           570           580           590           600           610
      •             •             •             •             •             •
 ACC ACC CCC CGC ACC GCC CTG ATG CTC CGT TCA CTC GCC GCC CAC AAG GAC GCG ACG
 Thr Thr Pro Arg Thr Ala Leu Met Leu Arg Ser Leu Ala Ala His Lys Asp Ala Thr

         620           630           640           650           660           670
          •             •             •             •             •             •
 GGC GCG GGG AAC CTG TTC GAC TCC GTG CTG GAG GAG GAG CTG GCC GGT GAG CGG GTC
 Gly Gly Gly Asn Leu Phe Asp Ser Val Leu Glu Glu Glu Leu Ala Gly Glu Arg Val

             680           690           700           710           720
              •             •             •             •             •
 GTC CTG GAG GGT GAG CAC TGG GCC GCC TTC GTC GCG TAC GGC GCG CAC TGG CCG TAC
 Val Leu Glu Gly Glu His Trp Ala Ala Phe Val Ala Tyr Gly Ala His Trp Pro Tyr

 730           740           750           760           770           780
  •             •             •             •             •             •
 GAG GTG CAC CTC TAC CCG AAG CGG CGG GTG CCC GAT CTG CTC GGG CTC GAC GAG GCG
 Glu Val His Leu Tyr Pro Lys Arg Arg Val Pro Asp Leu Leu Gly Leu Asp Glu Ala

     790           800           810           820           830           840
      •             •             •             •             •             •
 GCT CGC ACA GAA TTC CCC AAG GTC TAC CTG GAG CTG CTG AGG CGT TTC GAC CGG ATC
 Ala Arg Thr Glu Phe Pro Lys Val Tyr Leu Glu Leu Leu Arg Arg Phe Asp Arg Ile
```

Table 1 - (cont'd)

```
              850         860         870         880         890         900
               •           •           •           •           •           •
    TTC GGC GAG GGC GAG CCC CCG ACC CCC TAC ATC GCG GCC TGG CAC CAG GCG CCC TTC
    Phe Gly Glu Gly Glu Pro Pro Thr Pro Tyr Ile Ala Ala Trp His Gln Ala Pro Phe

              910         920         930         940         950
               •           •           •           •           •
    GGC CAG CTC GAG TTC GAG GGT GTC ACG CGC GAC GAC TTC GCG CTC CAC CTG GAA CTT
    Gly Gln Leu Glu Phe Glu Gly Val Thr Arg Asp Asp Phe Ala Leu His Leu Glu Leu

       960         970         980         990        1000        1010
        •           •           •           •           •           •
    TTC ACT TCC GCC GTA CGT CCG GCA AGC TGA AGT TCC TCG CGG GCT CCG AAT CCG GCA
    Phe Thr Ser Ala Val Arg Pro Ala Ser ---                 ⁻galP2

        1020        1030        1040        1050        1060        1070
          •           •           •           •           •           •
         TGAACG TGTTCATCAA CGACGTACCC CCGGAGCGCC CGGCCGAGCG ACTGCGAGAG GTAGCGAG


       1080        1090        1100        1110        1120        1130
        •           •           •           •           •           •
    TTC ATG AGC GGG AAG TAC CTG GTG ACA GGT GGT GCC GGA TAC GTC GGC AGC GTC GTC
        Met Ser Gly Lys Tyr Leu Val Thr Gly Gly Ala Gly Tyr Val Gly Ser Val Val
        galE
        1140        1150        1160        1170        1180        1190
          •           •           •           •           •           •
    GCC CAG CAC TTG GTG GAG GCG GGC AAC GAG GTC GTG GTG CTC CAC AAT CTG TCG ACC
    Ala Gln His Leu Val Glu Ala Gly Asn Glu Val Val Val Leu His Asn Leu Ser Thr

            1200        1210        1220        1230        1240
             •           •           •           •           •
    GGC TTC CGT GAG GTG TGC CGG CGG GTG CCT CGT TCG TCG AGC CGA CAT CCG GGA CGC
    Gly Phe Arg Glu Val Cys Arg Arg Val Pro Arg Ser Ser Arg Arg His Pro Gly Arg

    1250        1260        1270        1280        1290        1300
     •           •           •           •           •           •
    CGC CAA GTG CGT GGA CGG CTC TCG TTC GAC.GGC GTG CTG CAC TTC GCC GCC TTC TCC
    Arg Gln Val Arg Gly Arg Leu Ser Phe Asp Gly Val Leu His Phe Ala Ala Phe Ser

       1310        1320        1330        1340        1350        1360
        •           •           •           •           •           •
    CAG GTC GGC GAG TCG GTC GTG AAG CCC GAG AAG TAC TGG GAC AAC AAC GTC GGT GGC
    Gln Val Gly Glu Ser Val Val Lys Pro Glu Lys Tyr Trp Asp Asn Asn Val Gly Gly
```

Tabel 1 - (cont'd)

```
         1370        1380        1390        1400        1410        1420
          •           •           •           •           •           •
ACC ATC GCG CTC CTC GAG GCC ATG CGC GGC GCC GGT GTG CGG CGG CTC GTC TTC TCC
Thr Met Ala Leu Leu Glu Ala Met Arg Gly Ala Gly Val Arg Arg Leu Val Phe Ser

              1430        1440        1450        1460        1470
               •           •           •           •           •
TCC ACG GCC GCC ACG TAC GGC GAG CCC GAG CAG GTT CCC ATC GTC GAG TCC GCG CCG
Ser Thr Ala Ala Thr Tyr Gly Glu Pro Glu Gln Val Pro Ile Val Glu Ser Ala Pro

     1480        1490        1500        1510        1520        1530
      •           •           •           •           •           •
ACG AGG CCC ACC AAT CCG TAC GGC GCC TCG AAG CTC GCC GTC GAC CAC ATG ATC ACC
Thr Arg Pro Thr Asn Pro Tyr Gly Ala Ser Lys Leu Ala Val Asp His Met Ile Thr

         1540        1550        1560        1570        1580        1590
          •           •           •           •           •           •
GGC GAG GCG GCG GCC CAC GGG CTG GGC GCG GTC TCC GTG CCC TAC TTC AAC GTC GCG
Gly Glu Ala Ala Ala His Gly Leu Gly Ala Val Ser Val Pro Tyr Phe Asn Val Ala

              1600        1610        1620        1630        1640
               •           •           •           •           •
GGC GCG TAC GGG GAG TAC GGC GAG CGC CAC GAC CCC GAG TCG CAT CTG ATT CCG CTG
Gly Ala Tyr Gly Glu Tyr Gly Glu Arg His Asp Pro Glu Ser His Leu Ile Pro Leu

     1650        1660        1670        1680        1690        1700
      •           •           •           •           •           •
GTC CTT CAA GTG GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

         1710        1720        1730        1740        1750        1760
          •           •           •           •           •           •
CCG ACG CCG GAC CGA CCT GTG TGC GCG ACT ACA TCC ACG TCG CCG ACC TGG CCG AGG
Pro Thr Pro Asp Arg Pro Val Cys Ala Thr Thr Ser Thr Ser Pro Thr Trp Pro Arg

              1770        1780        1790        1800        1810
               •           •           •           •           •
CCC ACC TGC TGG CCC TGC GCC GCC GCC CCG GGC GAG CAC CTC ATC TGC AAC CTG GGC
Pro Thr Cys Trp Pro Cys Ala Ala Ala Pro Gly Glu His Leu Ile Cys Asn Leu Gly

1820        1830        1840        1850        1860        1870
 •           •           •           •           •           •
AAC GGC AAC GGC TTC TCC GTC CGC GAG GTC GTC GAG ACC GTG CGG CGG GTG ACC GGC
Asn Gly Asn Gly Phe Ser Val Arg Glu Val Val Glu Thr Val Arg Arg Val Thr Gly
```

18

Table 1 - (cont'd)

```
        1880        1890        1900        1910        1920        1930
         •           •           •           •           •           •
CAT CCG ATC CCC GAG ATC ATG GCC CCG CGC CGC GCG CGC GAC CCG GCG GTC CTG GTC
His Pro Ile Pro Glu Ile Met Ala Pro Arg Arg Gly Arg Asp Pro Ala Val Leu Val


        1940        1950        1960        1970        1980        1990
         •           •           •           •           •           •
GCG TCC GCC GGC ACC GCC CGC GAG AAG CTG GGC TGG AAC CCG TCC CGC GCG GAC CTC
Ala Ser Ala Gly Thr Ala Arg Glu Lys Leu Gly Trp Asn Pro Ser Arg Ala Asp Leu


            2000        2010        2020        2030        2040
             •           •           •           •           •
GCC ATC GTG TCC GAC GCG TGG GAG TTG CCG CAG CGG CGC GCG GGC CAG TAG  TA
Ala Ile Val Ser Asp Ala Trp Glu Leu Pro Gln Arg Arg Ala Gly Gln ---


        2050        2060        2070        2080        2090        2100
         •           •           •           •           •           •
ACC GCA GTT ACC GGA AAG GCG ACC GGT CAG GGC ATG GGC GAG GCT GTC GGG GAA CCG
                                        Met Gly Glu Ala Val Gly Glu Pro
                                        galK
        2110        2120        2130        2140        2150
         •           •           •           •           •
TCG GCC AGC GGT TCC GGG AGC TGT ACG GGG CGG AGC CGG AGG GGG TGT GGG CGC CGA
Ser Ala Ser Gly Ser Gly Ser Cys Thr Gly Arg Ser Arg Arg Gly Cys Gly Arg Arg


    2160        2170        2180        2190        2200        2210
     •           •           •           •           •           •
GCG GGC CGG GAG AAC CTC ATC GGG GAG CAC ACC GAC TAC AAC GAC GGC TTC GTC ATG
Ala Gly Arg Glu Asn Leu Ile Gly Glu His Thr Asp Tyr Asn Asp Gly Phe Val Met


        2220        2230        2240        2250        2260        2270
         •           •           •           •           •           •
CCT TCG CCC TGC CGC ACC AGG TCG CGG CCC TCT CCC GGC GCG AAC GAC GGC ATC CTG
Pro Ser Pro Cys Arg Thr Arg Ser Arg Pro Ser Pro Gly Ala Asn Asp Gly Ile Leu


        2280        2290        2300        2310        2320
         •           •           •           •           •
CGC CTG CAC TCG GCC GAC GTC GAC GCC GAC CCG GTC GAG CTG CGC GTC GCC GAC CTG
Arg Leu His Ser Ala Asp Val Asp Ala Asp Pro Val Glu Leu Arg Val Ala Asp Leu


    2330        2340        2350        2360        2370        2380
     •           •           •           •           •           •
GCC CCC GCG TCG GAC AAG TCC TGG ACG GCG TAC CCC TCG GGC GTC CTG TGG GCG CTG
Ala Pro Ala Ser Asp Lys Ser Trp Thr Ala Tyr Pro Ser Gly Val Leu Trp Ala Leu
```

Table 1 - (cont'd)

```
          2390        2400        2410        2420        2430        2440
           •           •           •           •           •           •
CGC GAG GCC GGA CAC GAG CTG ACC GGC GCC GAC GTC CAC CTG GCC TCG ACC GTC CCG
Arg Glu Ala Gly His Glu Leu Thr Gly Ala Asp Val His Leu Ala Ser Thr Val Pro

               2450        2460        2470        2480        2490
                •           •           •           •           •
TCC GGG GCG GGG CTC TCC TCC TCC GCG GCC CTG GAG GTC CGT CCC CTG GCG ATG AAC
Ser Gly Ala Gly Leu Ser Ser Ser Ala Ala Leu Glu Val Arg Pro Leu Ala Met Asn

 2500        2510        2520        2530        2540        2550
  •           •           •           •           •           •
GAC CTG TAC GCC CTC GCG CTG CGC GGC TGG CAG CTG GCC CGG CTG TGC CAG CGC GCG
Asp Leu Tyr Ala Leu Ala Leu Arg Gly Trp Gln Leu Ala Arg Leu Cys Gln Arg Ala

      2560        2570        2580        2590        2600        2610
       •           •           •           •           •           •
GAG AAC GTC TAC GTC GGC GCC CCC GTC GGC ATC ATG GAC CAG ACG GCG TCC GCC TGC
Glu Asn Val Tyr Val Gly Ala Pro Val Gly Ile Met Asp Gln Thr Ala Ser Ala Cys

           2620        2630        2640        2650        2660        2670
            •           •           •           •           •           •
TGC GAG GCG GGC ACG CCC TCT TCC TCG ACA CCC GCG ACC TCT CCC AGC GGC AGA TCC
Cys Glu Ala Gly Thr Pro Ser Ser Ser Thr Pro Ala Thr Ser Pro Ser Gly Arg Ser

               2680        2690        2700        2710        2720
                •           •           •           •           •
CCT TCG ACC TCG CCG CCG AGG GGA TGC GCC TGC TGG TCG TCG ACA CCC GGG TCA AGC
Pro Ser Thr Ser Pro Pro Arg Gly Cys Ala Cys Trp Ser Ser Thr Pro Gly Ser Ser

 2730        2740        2750        2760        2770        2780
  •           •           •           •           •           •
ACT CCC ACA GCG AGG GCG AGT ACG GCA AGC GCC GCG CGG GCT GCG AGA AGG GCG CCG
Thr Pro Thr Ala Arg Ala Ser Thr Ala Ser Ala Ala Arg Ala Ala Arg Arg Ala Pro

           2790        2800        2810        2820        2830        2840
            •           •           •           •           •           •
CGC TGC TGG GCG TCG ACG CGC TGC GAC GTG CCG TAC GCC GAC CTG GAC GCG GCG CTG
Arg Cys Trp Ala Ser Thr Arg Cys Asp Val Pro Tyr Ala Asp Leu Asp Ala Ala Leu

               2850        2860        2870        2880        2890
                •           •           •           •           •
GAG CGG CTG GGC GAC GAG GAG GAG GTG CGC CGC CTG GTC CGG CAC GTC GTG ACC GAG
Glu Arg Leu Gly Asp Glu Glu Glu Val Arg Arg Leu Val Arg His Val Val Thr Glu
```

20

**Table 1 - (cont'd)**

```
        2900          2910          2920          2930          2940          2950
          •             •             •             •             •             •
        GAC GAG CGC GTC GAA CGG GTG GTC GCG CTC CTC GAG TCG GCG ACA CCC GGC GCA TCG
        Asp Glu Arg Val Glu Arg Val Val Ala Leu Leu Glu Ser Ala Thr Pro Gly Ala Ser

            2960          2970          2980          2990          3000          3010
              •             •             •             •             •             •
        GCG CCG TCC TGG TCG AGG GCC ACG CCT GCT GCG CGA CGA CTT CCG CAT CTC CTC CCC
        Ala Pro Ser Trp Ser Arg Ala Thr Pro Ala Ala Arg Arg Leu Pro His Leu Leu Pro

              3020          3030          3040          3050          3060
                •             •             •             •             •
        CGA GCT GGA CCT GGT CGT CGA CAC GGC CCT GGC CTC CGC GGC CCT CGG CGC CGG ATG
        Arg Ala Gly Pro Gly Arg Arg His Gly Pro Gly Leu Arg Gly Pro Arg Arg Arg Met

        3070          3080          3090          3100          3110          3120
          •             •             •             •             •             •
        ACC GGC GGC GGC TTC GGC GGC TCG GCG ATC GTC CTG GTG GAG GCC GCC GCG GTG GAC
        Thr Gly Gly Gly Phe Gly Gly Ser Ala Ile Val Leu Val Glu Ala Ala Ala Val Asp

          3130          3140          3150          3160          3170          3180
            •             •             •             •             •             •
        GCC GTC ACC AAG GCG GTC GAG GAC GCC TTC GCC GCG GCG GGC CTC AAG CGT CCG CGG
        Ala Val Thr Lys Ala Val Glu Asp Ala Phe Ala Ala Ala Gly Leu Lys Arg Pro Arg

            3190          3200          3210          3220          3230          3240
              •             •             •             •             •             •
        GTG TTC GAG GCG GTG CCT CGG CGC GGC GCG GCG CCT GGT CTG ACG GTC AGC CGA GCC
        Val Phe Glu Ala Val Pro Arg Arg Gly Ala Ala Pro Gly Leu Thr Val Ser Arg Ala

              3250          3260          3270          3280          3290
                •             •             •             •             •
        GCT TCA CCA GCG TGT ACT CCG TGA TCC CCG GCG GGT AGT CGG GGA TCA CGC ACA TGA
        Ala Ser Pro Ala Cys Thr Pro ---

        3300
          •
        GCT GCT AGC CGC
```

## EXAMPLE 2

PROMOTERS OF THE S. LIVIDANS GAL OPERON

a) P1 promoter

(i) Summary

This promoter is galactose inducible, glucose repressible and is the regulatable promoter for the entire Streptomyces gal operon. S1 data indicates that the Streptomyces lividans gal operon encodes a polycistronic transcript of approximately 3.4 kilobases (Kb). The transcript consists of approximately 1 Kb for galT, followed by approximately 1 Kb each for galE and galK. (See, Figure 1).

Galactose induction of P1 is mediated, at least in part, by an operator sequence whose 5' end is located 31 bp upstream of the transcription start site and a repressor protein which recognizes the operator.

(ii) Experimental: Isolation, Localization, and Characterization of the P1 promoter.

The sequences upstream of the Streptomyces lividans galK ATG were screened for promoters using the E. coli galK promoter probe system of Brawner, et al., Gene, 40, 191, (1985), in press. The HindIII-

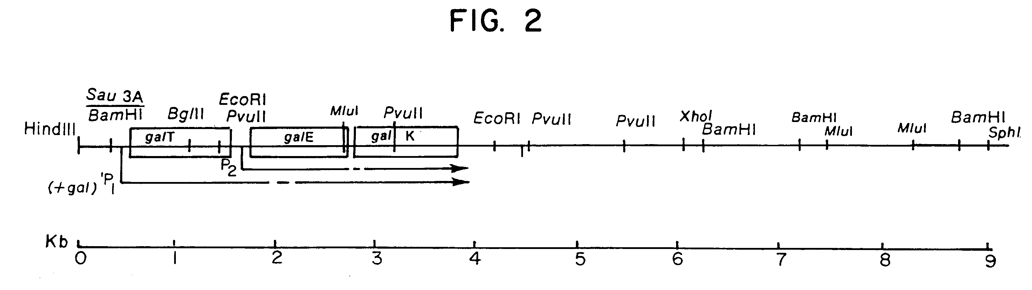

MluI fragment (See, Table A, map positions 1-5) was restricted with Sau3AI, ligated into the unique BamHI site of pK21 (Figure 2), and transformed into E. coli K21 (galK⁻) according to the method of Example 1. pK21 is a derivative of pSKO3 and is an E. coli-Streptomyces shuttle vector containing the E. coli galK gene (See, Figure 2). The construction of pSKO3 is described in Rosenberg et al., Genetic Engineering, 8, (1986), in press. The clones which expressed galK, i.e., those which had promoter activity, were identified on MacConkey - galactose plates. Two galK⁺ clones (designated as pK21 MH1 and 2) were transformed into Streptomyces 1326-12K (galK⁻). Extracts from transformants were cultured in Ymglu and Ymgal, and were analyzed by western blot analysis using anti-E. coli galactokinase antiserum. The blots showed significantly higher levels of galactokinase in the extracts from the galactose induced cultures.

pK21 MH1 and 2 were shown by restriction analysis to contain a 410 bp Sau3AI insert which is contained within the HindIII and BgIII sites (see Table A, map positions 1-2) by Southern blot analysis according to the method of Southern, J. Mol. Biol., 98, 503 (1975). The cloned fragment was analyzed by S1 analysis using RNA isolated from Streptomyces lividans 1326-12K and E. coli K21 cultures. The fragment yielded a 290 nucleotide protected fragment after S1 digestion (indicating the 5' end of an mRNA 290 bp upstream of the Sau3AI site). Hybridization experiments (using single stranded M13 clones of this region) have identified the direction of transcription as left to right as shown in Figure 2 (i.e., transcription is going toward galK).

Conventional DNA sequence analysis and additional S1 mapping analysis were used to define the 5'end of the mRNA.

The sequences responsible for regulating galactose induction of P1 were localized by removing sequences upstream of the transcription start site by nuclease Bal31. Any change in promoter function or galactose induction by removal of these sequences was assessed using the E. coli galK promoter probe plasmid used to identify P1.

(iii) Construction of Gal Promoter Deletions.

Plasmid pHL5 was constructed by cloning a DNA fragment containing 100 bp of sequences downstream from the start of P1 transcription and 216 bp upstream from the start of P1 transcription into plasmid pUC19TT1. Plasmid pUC19TT1 is described in Norrander et al., Gene, 26, 101-106 (1983) and has the linker as pUC18-TT6. See, Example IB. Deletions extending into the upstream sequence preceeding P1 were generated by iinearizing pHL5 with HindIII and treating the ends with nuclease Bal31. The uneven ends were subsequently repaired with the Klenow fragment of DNA polymerase I. Bal31-treated pHL5 was then digested with BamHI and run on a 5% acrylamide gel. DNA fragments in the molecular weight range of 100-300 bp were eluted from the gel and subcloned into M13 mp 10 that had been digested with HindII and BamHI. [See, Messing, Methods in Enzymology, 101, 20 (1983)]. Individual deletions were then sequenced from the single stranded phage DNA the dideoxy chain termination method of Sanger, et al., cited above.

(iv) Linking the P1 Promoter Deletions to the E. coli galK Gene.

The various mp 10 clones were digested with BamHI and HindIII. DNA fragments containing individual deletions were isolated from low-melting point agarose gels and then ligated to pK21 (see, Figure 2) that had been digested with BamHI and HindIII. After transformation into E. coli MM294, plasmid DNA was isolated for each of the deletion derivatives and transformed into Streptomyces Lividans 12K.

v) Functional Assessment of Bal 31-Generated Deletions in S. lividans

For each individual promoter deletion, a single thiostrepton resistant transformant was grown to late log in YM base (YEME) + 10 ug/ml thiostrepton. Cells were then pelleted, washed once in M56 media and resuspended in M56 media (see Miller, et al., cited above). The washed cells were then used to inoculate YM + 0 1M MOPS (pH 7.2) + 10 ug/ml thiostrepton supplemented with 1% galactose or 1% glucose. The cells were grown for 16 hours then assayed for galactokinase activity.

Ten individual pK21 derivatives containing either 120, 67, 55, 34, 31, 24, 20, 18, 10 or 8 bp of sequence upstream of the P1 transcription start site were analyzed for galactokinase expression. These results showed that all the information necessary for galactose induction of P1, (i.e., 10-20 fold greater levels of galactokinase produced in galactose grown cells versus glucose grown cells) is included in the 31 bp of sequence upstream of P1. A deletion which leaves 34 bp of sequence upstream of P1 is partially inducible by galactose since galactose induced 6-fold greater amounts of galactokinase. Thus, one end of the operator must be situated within the sequences between the -24 and -31 position. The remaining deletions which leave either 20, 18, 10 or 8 bp of upstream sequence result in a constitutive P1 promoter, that is the levels of galactokinase produced were equivalent when cells were grown in the presence of galactose or glucose. Although the promoter deletions which retained 8 and 10 bp of P1 were constitutive, the amount of galactokinase produced was reduced 10 fold in comparison to the promoter deletions which retained 18 to 120 bp of upstream sequence. This result indicates that sequences between the -10 and -18 positions of P1 are essential for promoter function.

This data supports a model in which galactose induction of P1 is mediated, at least in part, by an operator sequence. One end of this sequence is 24 to 31 bp upstream of the P1 transcription start site. Removing part or all of the operator results in a promoter which is partially or totally derepressed. The other end of this sequence has not been defined by these experiments but it most likely is contained within the 24 to 31 bp of sequence upstream of the P1 transcription start site. In addition we cannot eliminate the possibility that the 3' end of the operator is also within the 100 bp downstream of the transcription start site since these sequences were contained within the smallest region needed to achieve galactose induction. These data also suggest that the factor which interacts with the operator sequence is a repressor protein. Finally, we do not have any evidence which eliminates the possibility that P1 may be controlled by factors other than a repressor (i.e., positive activator such as lambda phage cII protein) to modulate galactose induction promoter transcript.

b) P2 promoter

(i) Summary

The P2 promoter of the Streptomyces gal operon is upstream of the galE gene and transcribes both galE and galK genes.

P2 promoter expression is constitutive (i.e., not glucose repressed/galactose induced) as shown by S1 analysis.

(ii) Experimental: Isolation, Localization, and Chararterization of the P2 promoter.

The existence of the Streptomyces gal operon P2 promoter became apparent when the BglIII-MluI fragment (see, Table A, map positions 2-5) of S. lividans 1326 DNA was inserted into plasmid pK21 (see, Figure 2) and galactokinase expression was observed in Streptomyces lividans 1326-12K transformed therewith.

DNA sequence analysis and S1 analysis were used to identify the 5' end of the S. lividans gal operon P2. The 5' end of the P2 promoter transcript is within 100 bp upstream of the predicted galE ATG.

EXAMPLE 3

EVIDENCE OF A POLYCISTRONIC MESSAGE IN THE STREPTOMYCES GAL OPERON

S1 analysis was used to map the transcripts upstream and downstream of the Streptomyces lividans gal operon galK gene. In general, overlapping DNA fragments of 1-2 Kb were isolated from subclones, further restricted, and end label led. The message was followed from the 3' end of galK to the upstream end at P1.

The 3' end of the Streptomyces lividans gal operon transcript probably occurs within the first hundred bases downstream of galK. Fragments 3' labelled at sites within the galK sequence were not protected to their full length (S1 analysis) if they extend into this downstream region. One experiment showed a possible protected region that terminated 50-100 bp downstream of the galK translation stop. The existence of a transcription terminator can be confirmed by conventional techniques by using a terminator probe system. The gal operon transcript clearly does not extend to the PvuII site (see, Table A, map position 8) because no full length protection of 5' labelled PvuII fragments occurs from that site.

5' end labelled fragments from two PvuII fragments, fragment I, (map positions 4-6, See, Table A), and fragment II, (map positions 6-8, See Table A), and the insert of pSau10 were used as sources of probes for S1 walking from the 3' to 5' end of the message. All fragments through this region are protected, except the fragment containing the P2 promoter which shows partial and full protection. The complete protection from S1 digest indicates a polycistronic message which initiates upstream at P1 and continues to approximately 100 bp downstream of galK.

The above data is indirect evidence of a polycistronic mRNA of the Streptomyces gal operon. S1 analysis using a long contiguous DNA fragment (e.g., the 4.5 kb HindIII-SacI fragment, see map position 7 of Table A) has been used to confirm the transcript size.

EXAMPLE 4

LOCALIZATION OF S. LIVIDANS GAL OPERON GALE AND GALT GENES

(i) Summary

The S. lividans gal operon galE gene was localized to 1.5 Kb PvuII fragment (map position, 4-6 of Table A) of pLIVGAL1 (Figure 1).

The S. lividans gal operon galE coding sequences extend through the MluI site (map position 5 of Table A).

The S. lividans gal operon galT gene was localized within the 1.15 Kb Nru-PvuII region (see, Table A, map positions 1a-4) of pSLIVGAL1.

Tee direction of S. lividans gal operon galE and galT transcription is the same as galK gene.

(ii) Experimental

It was necessary to identify the other functions contained on pLIVGAL1; specifically, does this plasmid encode for the enzyme galactose epimerase (galE) or the enzyme galactose transferase (galT). The Streptomyces gal operon galK gene was identified by its ability to complement an E. coli galK host. Thus, identification of the Streptomyces galT and galE genes was tested for by complementation of E. coli galE⁻ or galT⁻ hosts, respectively. An E. coli galT⁻ strain (CGSC strain #4467, W3101) and two galE⁻ strains (CGSC strain #4473; W3109 and CGSC strain #4498; PL-2) were obtained to test for complementation by the pSLIVGAL1 clone.

The ca. 9 Kb HindIII-SphI fragment (see, Table A, map positions 1-16) containing the Streptomyces lividans gal operon galK gene was inserted into pUC19. This fragment was situated within pUC19 such that transcription from the Plac promoter of pUC19 is in the same direction as the Streptomyces galK gene. pUC19 is described in Yanisch-Perrou, et al., Gene, 33, 103 (1983). Complementation was assayed by growth on MacConkey-galactose plates. Cells which can utilize galactose [galE⁺, galT⁺, galK⁺] will be red to pink on this medium. E. coli strain PL-2 (see, Example 2) containing pUC19 with the HindIII-SphI insert were pink on the indicator plate indicating that the HindIII-SphI fragment contains the Streptomyces lividans galE gene. The galE gene was later mapped to within the 4.5 Kb HindIII-SacI (the SacI site is near the region around map position 7-8 of Table A) fragment. If the sequences from the MluI site (map position 5 of Table A) to the SacI site were removed galE complementation of E. coli PL-2 was not detected. The 5' end of the galK gene is 70 base pairs (bp) from the MluI site. Therefore it seemed likely that the MluI site was contained within the 5' or 3' end of the galE gene. To determine the direction of galE transcription, the HindIII-SacI fragment was inserted into pUC18. In this configuration, the Streptomyces lividans galK gene is in the opposite orientation with respect to Plac. The pUC18 HindIII-SphI clone did not complement E. coli PL-2 indicating the galE is transcribed in the same direction as galK. In addition it was concluded that the MluI site is contained within the 3' end of the galE gene. DNA sequence analysis of the PvuII-MluI fragment (See, Table A, map position 4-5) has identified an open reading frame which encodes for a polypeptide of predicted molecular weight of 33,000 daltons. The 5' end of this reading frame is located approximately 176 bp from the PvuII site (See, Table A, map position 4). Therefore, the sequencing results support the conclusion that the 3' end of galE traverses the MluI site (see, Table A, map position 5).

Similar experiments to localize the galT gene on pSLIVGAL1 were attempted with the galT hosts.

The region between P1 and the 5' end of galE was sequenced to identify the galT gene. Translation of the DNA sequence to the amino acid sequence identified a reading frame which encodes a protein showing a region of homology to the yeast transferase.

EXAMPLE 5

GALACTOSE INDUCTION OF S. LIVIDANS GAL OPERON GALK GENE

(i) Summary

Galactokinase expression is induced within one hour after the addition of galactose to culture medium.

Galactokinase expression is 10 times higher in the presence of galactose versus glucose or no additional carbon source within 6 hours after addition of the sugar.

(ii) Experimental

Galactose induction of the Streptomyces lividans galK gene was examined by assaying for galactokinase activity at 1, 3, 6 and 24 hours after the addition of galactose. Two liters of YM + 0.1M MOPS (pH 7.2) were inoculated with $2\times10^7$ spores of Streptomyces lividans 1326. After 21 hours growth, galactose or glucose were added to a final concentration of 1%. One, three, six and twenty four hours after the addition of sugar, cells were isolated and assayed for galactokinase activity. Total RNA was prepared by procedures described in Hopwood et al., cited above.

An increase in galactokinase synthesis was observed one hour after the addition of galactose. The increase continued over time (1 to 24 hours). S1 analysis of RNA isolated from the induced cultures confirmed that the increase in galK activity was due to increased levels of the P1 promoter transcript.

The S1 data and the induction studies suggest the following model for gene expression within the Streptomyces gal operon. The P1 promoter is the galactose inducible promoter. The P1 transcript includes galT, galE and galK. The P2 promoter is constitutive and its transcript includes galE and galK.

It is interesting to note that the E. coli gal operon also has two promoters, P1 and P2. [See, Nusso et al., Cell, 12, 847 (1977)]. P1 is activated by cAMP-CRP binding whereas P2 is inhibited by cAMP-CRP. Translation of the E. coli gal operon galE coding sequence is more efficient when transcription initiates at P2 which serves to supply a constant source of epimerase even in the absence of galactose or the presence of glucose [See, Queen et al., Cell, 25, 241 (1981)]. The epimerase functions to convert galactose to glucose 1-phosphate during galactose utilization and convert UDP-glucose to UDP-galactose which is required for E. coli cell wall biosynthesis. It is possible that the P2 promoter of the Streptomyces galK operon also serves to supply epimerase and galactokinase in the absence of galactose or during secondary metabolism.

EXAMPLE 6

THE S. COELICOLOR GAL OPERON

(i) Summary

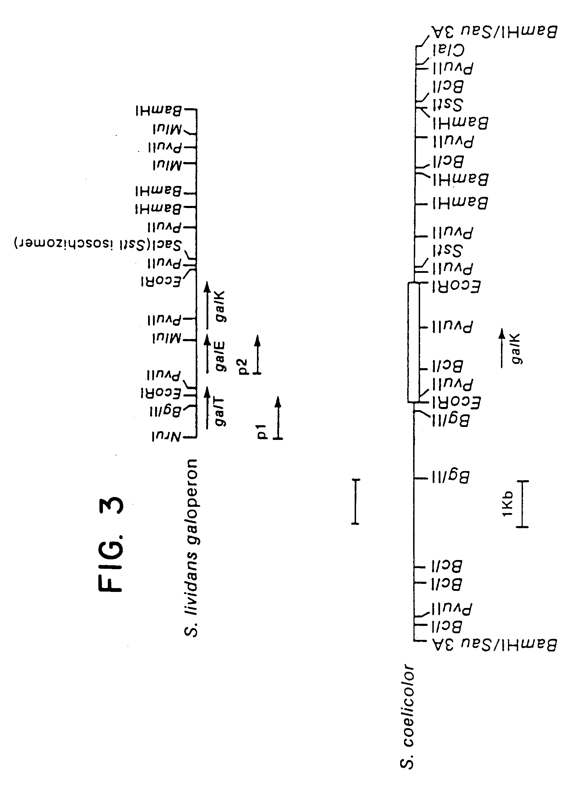

The restriction map of a fragment containing the S. coelicolor galK gene is identical to the restriction map of the S. lividans gal operon. (See, Figure 3).

S. coelicolor can grow on minimal media containing galactose as the sole carbon source.

Galactokinase expression in S. coelicolor is induced by the addition of galactose to the growth media.

A promoter analogous and most likely identical to P1 is responsible for galactose induction of the S. coelicolor gal operon.

(ii) Experimental

An approximately 14 kb partial Sau3A fragment containing the S. coelicolor galK gene was isolated by K. Kendall and J. Cullum at the University of Manchester Institute of Science and Technology, Manchester, UK (unpublished data; personal communication). They were able to localize the S. coelicolor galK gene within a 3 kb EcoRI fragment by complementation of a S. coelicolor galK mutant. The position of a number of restriction sites within the S. lividans gal operon are identical to those found within, upstream and downstream of the EcoRI fragment containing the S. coelicolor galK gene (Figure 3). Thus, it seems likely that the gene organization of the S. coelicolor gal operon is identical to the S. lividans gal operon.

Galactose induction of the S. coelicolor galK gene was examined by immunoblotting. S. coelicolor was grown in YM + 1% galactose or 1% glucose (Ymglu or Ymgal) for 20 hours at 28 C. Galactokinase expression was detected using rabbit antisera prepared against purified E. coli galactokinase. The protein detected was the approximate site of the E. coli and S. lividans galK gene product. Galactokinase expression is galactose induced since it was detected only when S. coelicolor was grown in Ym + galactose (Ymgal).

S1 nuclease protection studies were performed to determine if galactose induction of the S. coelicolor gal operon is directed by a promoter analogous to the S. lividans P1 promoter. RNA was isolated from S. coelicolor grown in Ym + 1% galactose or 1% glucose (Ymgal or Ymglu). The hybridization probe used for S1 analysis of this RNA was a 410 bp Sau3A fragment which contains the S. lividans P1 promoter, its transcription start site and the 5' end of the galT gene. The S1 protected fragment detected by this analysis co-migrated with the protected fragment detected when the probe was hybridized to RNA isolated from S.

lividans grown in the presence of galactose. Thus, this result shows that galactose induction of the S. coelicolor gal operon is directed by a sequence indistinguishable from the S. lividans P1 promoter.

It should be noted that the following strains of Streptomyces have been observed to be able to grow on medium containing galactose as the only carbon source:

S. albus J1074 (obtained from Dr. Chater, John Innes Foundation, Norwich, England)

S. carzinostaticus - ATCC accession number 15944

S. carzinostaticus - ATCC accession number 15945

S. antifibrinolyticus - ATCC accession number 21869

S. antifibrinolyticus - ATCC accession number 21870

S. antifibrinolyticus - ATCC accession number 21871

S. longisporus - ATCC accession number 23931

The abbreviation "ATCC" stands for the American Type Culture Collection, Rockville, Maryland, U.S.A.

While the above descriptions and Examples fully describe the invention and the preferred embodiments thereof, it is understood that the invention is not limited to the particular disclosed embodiments. Thus, the invention includes all embodiments coming within the scope of the following claims.

**Claims**
**Claims for the following Contracting States : BE, CH, DE, FR, GB, IT, LI, LU, NL, SE, GR**

1. A recombinant DNA molecule comprising a Streptomyces lividans gal operon located on a ca. 9kb HindIII-SphI fragment of S. lividans 1326 chromosomal DNA or any Streptomyces derivative thereof which functions in substantially the same way as the naturally occurring Streptomyces gal operon in terms of regulatable production of the galT, galE and galK gene products.

2. The molecule of Claim 1 wherein the operon is a S. coelicolor gal operon.

3. The molecule of Claim 1 which has the following coding sequence:

```
        -120          -110           -100            -90            -80           -70
          •             •              •              •              •             •
  CTA CGC CTC CGC GTT CAC TAA TTC AAC ACT TTT GCT GAT GAA CTT TCT TTG ATT CTC


          -60           -50            -40            -30            -20
           •             •              •              •              •
  ATC TGA CAC GCG GGT GGT GGC TTC TGA TGT GTT ATG TTT GAT TGT GTT GGA TGA TTG
                                                                   ˙galP1

    -10                          10             20             30             40
     •                            •              •              •              •
  ACG GGC GTC CTC GTC ACT CAT GGG TGG GTC CAG AGG AGT GCG GCA GTG AAG AAC ACC
                      Met Thr His Gly Trp Val Gln Arg Ser Ala Ala Val Lys Lys Thr
                      galT
        50            60             70             80             90            100
         •             •              •              •              •             •
  TCG ACC CGG CTG GCC GAC GGC CGT GAG CTC GTC TAC TAC GAC CTG CGC GAC GAC ACC
  Ser Thr Arg Leu Ala Asp Gly Arg Glu Leu Val Tyr Tyr Asp Leu Arg Asp Asp Thr


          110           120            130            140            150
           •             •              •              • •            •
  GTC CGC GAC GCC GTG GAC CGC CGT CCG CTG GAG CGG ACC GTC ACC ACG TCC GAG GTC
  Val Arg Asp Ala Val Asp Arg Arg Pro Leu Glu Arg Thr Val Thr Thr Ser Glu Val

  160           170            180            190            200            210
   •             •              •              •              •              •
  CGA CGC GAC CCG CTG CTC GGC GAC TCC GCG CCG TCG CGC CTC GCA CCG GCA GGG GCG
  Arg Arg Asp Pro Leu Leu Gly Asp Ser Ala Pro Ser Arg Leu Ala Pro Ala Gly Ala


        220           230            240            250            260            270
         •             •              •              •              •              •
  CAC CTA CCA TCC GCC GGC CGA CCA GTG CCC GCT GTG CCc GTC GGA CGG GGA ACG GCT
  His Leu Pro Ser Ala Gly Arg Pro Val Pro Ala Val Pro Val Gly Arg Gly Thr Ala


        280           290            300            310            320            330
         •             •              •              •              •              •
  GAG CGA GAT CCG GCC TAT GAC GTG GTG GTC TTC GAG AAT CGC TTT CCC TCG CTG GCC
  Glu Arg Asp Pro Ala Tyr Asp Val Val Val Phe Glu Asn Arg Phe Pro Ser Leu Ala
```

```
         340        350        360        370        380
          •          •          •          •          •
GGT GAC TCC GGG CCC TGC GAG GTC CTC TGC TTC ACC TCC GAC CAC GAC GCC TCC TTC
Gly Asp Ser Gly Arg Cys Glu Val Val Cys Phe Thr Ser Asp His Asp Ala Ser Phe

   390        400        410        420        430        440
    •          •          •          •          •          •
GCC GAC CTG AGC GAG GAG CAG GCC CGG CTC GTC GTC GAC GCC TGG ACG GAC CGC ACC
Ala Asp Leu Ser Glu Glu Gln Ala Arg Leu Val Val Asp Ala Trp Thr Asp Arg Thr

        450        460        470        480        490        500
         •          •          •          •          •          •
TCC GAG CTG TCC CAT CTC CCC TCC GTT GAA CAG GTG TTC TGC TTC GAG AAC CGC GGC
Ser Glu Leu Ser His Leu Pro Ser Val Glu Gln Val Phe Cys Phe Glu Asn Arg Gly

             510        520        530        540        550
              • •        •          •          •          •
GCC GAG ATC GGC GTG ACG CTG GGT CAC CCG CAC GGG CAG ATC TAC GCC TAC CCG TTC
Ala Glu Ile Gly Val Thr Leu Gly His Pro His Gly Gln Ile Tyr Ala Tyr Pro Phe

560        570        580        590        600        610
 •          •          •          •          •          •
ACC ACC CCC CGC ACC GCC CTG ATG CTC CGT TCA CTC GCC GCC CAC AAG GAC GCG ACG
Thr Thr Pro Arg Thr Ala Leu Met Leu Arg Ser Leu Ala Ala His Lys Asp Ala Thr

   620        630        640        650        660        670
    •          •          •          •          •          •
GGC GGG CGG AAC CTC TTC GAC TCC GTG CTG GAG GAG GAG CTG GCC GGT GAG CGG GTC
Gly Gly Gly Asn Leu Phe Asp Ser Val Leu Glu Glu Glu Leu Ala Gly Glu Arg Val

        680        690        700        710        720
         •          •          •          •          •
GTC CTG GAG GGT GAG CAC TGG GCC GCC TTC GTC GCG TAC GGC GCG CAC TGG CCG TAC
Val Leu Glu Gly Glu His Trp Ala Ala Phe Val Ala Tyr Gly Ala His Trp Pro Tyr

730        740        750        760        770        780
 •          •          •          •          •          •
GAG GTC CAC CTC TAC CCG AAG CGG CGG GTG CCC GAT CTG CTC GGG CTC GAC GAG GCG
Glu Val His Leu Tyr Pro Lys Arg Arg Val Pro Asp Leu Leu Gly Leu Asp Glu Ala

   790        800        810        820        830        840
    •          •          •          •          •          •
GCT CGC ACA GAA TTC CCC AAG GTC TAC CTG GAG CTG CTG AGG CGT TTC GAC CGC ATC
Ala Arg Thr Glu Phe Pro Lys Val Tyr Leu Glu Leu Leu Arg Arg Phe Asp Arg Ile
```

```
         850           860           870           880           890           900
          •             •             •             •             •             •
TTC GGC GAG GGC GAG CCC CCG ACC CCC TAC ATC GCC GCC TGG CAC CAG GCG CCC TTC
Phe Gly Glu Gly Glu Pro Pro Thr Pro Tyr Ile Ala Ala Trp His Gln Ala Pro Phe

              9:             920           930           940           950
               •             •             •             •             •
GGC CAG CTG GAG TTC GAG GGT GTC ACC CGC GAC GAC TTC GCG CTC CAC CTG GAA CTT
Gly Gln Leu Glu Phe Glu Gly Val Thr Arg Asp Asp Phe Ala Leu His Leu Glu Leu

     960           970           980           990          1000          1010
      •             •             •             •             •             •
TTC ACT TCC GCC GTA CGT CCG GCA AGC TGA AGT TCC TCG CGG GCT CCG AAT CCG GCA
Phe Thr Ser Ala Val Arg Pro Ala Ser ---      ⌐galP2

     1020          1030          1040          1050          1060          1070
      •             •             •             •             •             •
    TGAACG TGTTCATCAA CGACGTACCC CCGGAGCGCG CGGCCCGAGCG ACTGCGAGAG GTAGCGAG

1080          1090          1100          1110          1120          1130
 •             •             •             •             •             •
TTC ATG AGC GGG AAG TAC CTG GTG ACA GGT GGT GCC GGA TAC GTC GGC AGC GTC GTC
    Met Ser Gly Lys Tyr Leu Val Thr Gly Gly Ala Gly Tyr Val Gly Ser Val Val
    galE
    1140          1150          1160          1170          1180          1190
     •             •             •             •             •             •
GCC CAG CAC TTG GTG GAG GCG GGG AAC GAG GTC GTG GTG CTG CAC AAT CTG TCG ACC
Ala Gln His Leu Val Glu Ala Gly Asn Glu Val Val Val Leu His Asn Leu Ser Thr

        1200          1210          1220          1230          1240
         •             •             •             •             •
GGC TTC CGT GAG GTG TGC CGG CGG GTC CCT CGT TCG TCG ACG CGA CAT CCG GCA CGC
Gly Phe Arg Glu Val Cys Arg Arg Val Pro Arg Ser Ser Arg Arg His Pro Gly Arg

1250          1260          1270          1280          1290          1300
 •             •             •             •             •             •
CGC CAA GTG CGT GGA CGG CTC TCG TTC GAC GGC GTG CTC CAC TTC GCC GCC TTC TCC
Arg Gln Val Arg Gly Arg Leu Ser Phe Asp Gly Val Leu His Phe Ala Ala Phe Ser

   1310          1320          1330          1340          1350          1360
    •             •             •             •             •             •
CAG GTC GGC GAG TCG GTC GTG AAG CCC GAG AAG TAC TGG GAC AAC AAC GTC GGT GGC
Gln Val Gly Glu Ser Val Val Lys Pro Glu Lys Tyr Trp Asp Asn Asn Val Gly Gly
```

```
         1370          1380          1390          1400          1410          1420
          •             •             •             •             •             •
ACC ATC GCG CTC CTC GAG GCC ATC CGC GGC GCC GGT GTC CGG CGC CTC GTC TTC TCC
Thr Met Ala Leu Leu Glu Ala Met Arg Gly Ala Gly Val Arg Arg Leu Val Phe Ser

                1430          1440          1450          1460          1470
                 •             •             •             •             •
TCC ACG GCC GCC ACG TAC GGC GAG CCC GAG CAG GTT CCC ATC GTC GAG TCC GCG CCC
Ser Thr Ala Ala Thr Tyr Gly Glu Pro Glu Gln Val Pro Ile Val Glu Ser Ala Pro

     1480          1490          1500          1510          1520          1530
      •             •             •             •             •             •
ACG AGG CCC ACC AAT CCG TAC GGC GCC TCC AAG CTC GCC GTC GAC CAC ATG ATC ACC
Thr Arg Pro Thr Asn Pro Tyr Gly Ala Ser Lys Leu Ala Val Asp His Met Ile Thr

         1540          1550          1560          1570          1580          1590
          •             •             •             •             •             •
GGC GAG GCG GCC GCC CAC GGG CTG GGC GCG GTC TCC GTC CCG TAC TTC AAC GTC GCG
Gly Glu Ala Ala Ala His Gly Leu Gly Ala Val Ser Val Pro Tyr Phe Asn Val Ala

             1600          1610          1620          1630          1640
              •             •             •             •             •
GGC GCG TAC GGC GAG TAC GGC GAG CGC CAC GAC CCC GAG TCG CAT CTG ATT CCC CTG
Gly Ala Tyr Gly Glu Tyr Gly Glu Arg His Asp Pro Glu Ser His Leu Ile Pro Leu

  1650          1660          1670          1680          1690          1700
   •             •             •             •             •             •
GTC CTT CAA GTG GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

     1710          1720          1730          1740          1750          1760
      •             •             •             •             •             •
CCG ACC CCG GAC CGA CCT GTG TGC GCC ACT ACA TCC ACG TCC CCG ACC TGG CCG AGG
Pro Thr Pro Asp Arg Pro Val Cys Ala Thr Thr Ser Thr Ser Pro Thr Trp Pro Arg

             1770          1780          1790          1800          1810
              •             •             •             •             •
CCC ACC TGC TGG CCG TGC GCC GCC GCC CCG GGC GAG CAC CTC ATC TGC AAC CTG GGC
Pro Thr Cys Trp Pro Cys Ala Ala Ala Pro Gly Glu His Leu Ile Cys Asn Leu Gly

 1820          1830          1840          1850          1860          1870
  •             •             •             •             •             •
AAC GGC AAC GGC TTC TCC GTC CGC GAG GTC GTC GAG ACC GTG CGG CGG GTG ACG GGC
Asn Gly Asn Gly Phe Ser Val Arg Glu Val Val Glu Thr Val Arg Arg Val Thr Gly
```

```
        1880          1890          1900          1910          1920          1930
         •             •             •             •             •             •
CAT CCC ATC CCC GAG ATC ATG GCC CCG CGC CGC GGG CGC GAC CCG GCG CTC CTC GTC
His Pro Ile Pro Glu Ile Met Ala Pro Arg Arg Gly Arg Asp Pro Ala Val Leu Val

        1940          1950          1960          1970          1980          1990
         •             •             •             •             •             •
GCG TCC GCC GGC ACC GCC CGC GAG AAG CTC GGC TGG AAC CCG TCC CGC GCG GAC CTC
Ala Ser Ala Gly Thr Ala Arg Glu Lys Leu Gly Trp Asn Pro Ser Arg Ala Asp Leu

        2000          2010          2020          2030          2040
         •             •             •             •             •
GCC ATC GTC TCC GAC GCG TGG GAG TTG CCG CAG CGG CGC GCC GGC CAG TAG  TA
Ala Ile Val Ser Asp Ala Trp Glu Leu Pro Gln Arg Arg Ala Gly Gln ---


        2050          2060          2070          2080          2090          2100
         •             •             •             •             •             •
ACC GCA GTT ACC GGA AAG GCG ACC GGT CAG GGC ATG GGC GAG CCT GTC GGG GAA CCG
                                            Met Gly Glu Ala Val Gly Glu Pro
                                            galK
        2110          2120          2130          2140          2150
         •             •             •             •             •
TCG GCC AGC GGT TCC GGG AGC TGT ACG GGG CGG AGC CGG AGG GGC TGT GGG CGC CGA
Ser Ala Ser Gly Ser Gly Ser Cys Thr Gly Arg Ser Arg Arg Gly Cys Gly Arg Arg

  2160          2170          2180          2190          2200          2210
   •             •             •             •             •             •
GCG GGC CGC GAG AAC CTC ATC GGG GAG CAC ACC GAC TAC AAC GAC GGC TTC GTC ATG
Ala Gly Arg Glu Asn Leu Ile Gly Glu His Thr Asp Tyr Asn Asp Gly Phe Val Met

     2220          2230          2240          2250          2260          2270
      •             •             •             •             •             •
CCT TCG CCC TGC CGC ACC AGG TCG CGG CCG TCT CCC GGC GCG AAC GAC GGC ATC CTC
Pro Ser Pro Cys Arg Thr Arg Ser Arg Pro Ser Pro Gly Ala Asn Asp Gly Ile Leu

        2280          2290          2300          2310          2320
         •             •             •             •             •
CGC CTG CAC TCG GCC GAC GTC GAC GCC GAC CCG GTC GAG CTG CGC GTC GCC GAC CTG
Arg Leu His Ser Ala Asp Val Asp Ala Asp Pro Val Glu Leu Arg Val Ala Asp Leu

2330          2340          2350          2360          2370          2380
 •             •             •             •             •             •
GCC CCC GCG TCG GAC AAG TCC TGC ACG GCG TAC CCC TCG GGC GTC CTG TGG GCG CTG
Ala Pro Ala Ser Asp Lys Ser Trp Thr Ala Tyr Pro Ser Gly Val Leu Trp Ala Leu
```

```
          2390          2400          2410          2420          2430          2440
           •             •             •             •             •             •
CGC GAG GCC GGA CAC GAG CTG ACC GGC GCC GAC GTC CAC CTG GCC TCG ACC GTC CCG
Arg Glu Ala Gly His Glu Leu Thr Gly Ala Asp Val His Leu Ala Ser Thr Val Pro

                2450          2460          2470          2480          2490
                 •             •             •             •             •
TCC GGG GCG GGG CTC TCC TCC TCC GCG GCC CTG GAG GTC CCT CCC CTG GCG ATG AAC
Ser Gly Ala Gly Leu Ser Ser Ser Ala Ala Leu Glu Val Arg Pro Leu Ala Met Asn

     2500          2510          2520          2530          2540          2550
      •             •             •             •             •             •
GAC CTG TAC GCC CTC GCG CTG CGC GGC TGG CAG CTG GCC CGG CTG TGC CAG CGC GCG
Asp Leu Tyr Ala Leu Ala Leu Arg Gly Trp Gln Leu Ala Arg Leu Cys Gln Arg Ala

          2560          2570          2580          2590          2600          2610
           •             •             •             •             •             •
GAG AAC GTC TAC GTC GGC GCC CCC GTC GGC ATC ATG GAC CAG ACG GCG TCC GCC TGC
Glu Asn Val Tyr Val Gly Ala Pro Val Gly Ile Met Asp Gln Thr Ala Ser Ala Cys

               2620          2630          2640          2650          2660          2670
                •             •             •             •             •             •
TGC GAG GCG GGC ACG CCC TCT TCC TCG ACA CCC GCG ACC TCT CCC ACC GGC AGA TCC
Cys Glu Ala Gly Thr Pro Ser Ser Ser Thr Pro Ala Thr Ser Pro Ser Gly Arg Ser

               2680          2690          2700          2710          2720
                •             •             •             •             •
CCT TCG ACC TCG CCG CCG AGG GGA TGC GCC TGC TGG TCC TCG ACA CCC GGG TCA AGC
Pro Ser Thr Ser Pro Pro Arg Gly Cys Ala Cys Trp Ser Ser Thr Pro Gly Ser Ser

2730          2740          2750          2760          2770          2780
 •             •             •             •             •             •
ACT CCC ACA GCG AGG GCG AGT ACG GCA AGC GCC GCG CGG GCT GCG AGA AGC GCG CCG
Thr Pro Thr Ala Arg Ala Ser Thr Ala Ser Ala Ala Arg Ala Ala Arg Arg Ala Pro

     2790          2800          2810          2820          2830          2840
      •             •             •             •             •             •
CGC TGC TGG GCG TCG ACG CGC TGC GAC GTG CCG TAC GCC GAC CTG GAC GCG GCG CTG
Arg Cys Trp Ala Ser Thr Arg Cys Asp Val Pro Tyr Ala Asp Leu Asp Ala Ala Leu

          2850          2860          2870          2880          2890
           •             •             •             •             •
GAG CGG CTG GGC GAC GAG GAG GAG GTG CGC CGC CTG GTC CGG CAC GTG GTC ACC GAG
Glu Arg Leu Gly Asp Glu Glu Glu Val Arg Arg Leu Val Arg His Val Val Thr Glu
```

```
       2900           2910           2920           2930           2940           2950
        •              •              •              •              •              •
GAC GAG CGC GTC GAA CGG CTC GTC GCC CTC CTG GAG TCG GCG ACA CCC GGC GCA TCG
Asp Glu Arg Val Glu Arg Val Val Ala Leu Leu Glu Ser Ala Thr Pro Gly Ala Ser

           2960           2970           2980           2990           3000           301C
            •              •              •              •              •              •
GCG CCG TCC TGG TCG AGG GCC ACG CCT GCT GCG CGA CGA CTT CCG CAT CTC CTG CCC
Ala Pro Ser Trp Ser Arg Ala Thr Pro Ala Ala Arg Arg Leu Pro His Leu Leu Pro

           3020           3030           3040           3050           3060
            •              •              •              •              •
CGA GCT GGA CCT GGT CGT CGA CAC GGC CCT GGC CTC CGC GGC CCT CGG CGC CGG ATG
Arg Ala Gly Pro Gly Arg Arg His Gly Pro Gly Leu Arg Gly Pro Arg Arg Arg Met

3070          3080           3090           3100           3110           3120
 •             •              •              •              •              •
ACC GGC GGC GGC TTC GGC GGC TCG GCG ATC GTC CTG GTG GAG GCC GCC GCG CTG GAC
Thr Gly Gly Gly Phe Gly Gly Ser Ala Ile Val Leu Val Glu Ala Ala Ala Val Asp

           3130           3140           3150           3160           3170           3180
            •              •              •              •              •              •
GCC GTC ACC AAC GCG GTC GAG GAC GCC TTC GCC GCG GCG GGC CTC AAG CGT CCG CGG
Ala Val Thr Lys Ala Val Glu Asp Ala Phe Ala Ala Ala Gly Leu Lys Arg Pro Arg

           3190           3200           3210           3220           3230           3240
            •              •              •              •              •              •
GTG TTC GAG GCG GTG CCT CGG CGG GGC GCG GCG CCT GGT CTC ACC GTC AGC CGA GCC
Val Phe Glu Ala Val Pro Arg Arg Gly Ala Ala Pro Gly Leu Thr Val Ser Arg Ala

           3250           3260           3270           3280           3290
            •              •              •              •              •
GCT TCA CCA GCG TGT ACT CCG TGA TCC CCG GCG GGT AGT CGG GGA TCA CGC ACA TGA
Ala Ser Pro Ala Cys Thr Pro ---

3300
 •
GCT GCT AGC CGC
```

4. The molecule of any of Claims 1 to 3 which further comprises a foreign functional DNA sequence operatively linked to such operon.

5. A recombinant DNA molecule comprising a Streptomyces lividans gal operon P2 promoter expression unit located on a ca. 9kb HindIII-SphI fragment of S. lividans 1326 chromosomal DNA having an NruI site, where the P2 transcription start site is located approximately 1.25 Kb downstream of the NruI site, or any Streptomyces derivative thereof which functions in substantially the same way as the naturally occurring unit in terms of production of the galE and galK gene products.

6. The molecule of Claim 5 wherein the expression unit is a S. coelicolor gal operon P2 promoter expression unit.

7. The molecule of Claim 5 or 6 which further comprises a foreign functional DNA sequence operatively linked to such expression unit.

8. A recombinant DNA molecule comprising a Streptomyces lividans gal operon P1 promoter regulated region located on a ca. 9kb HindIII-SphI fragment of S. lividans 1326 chromosomal DNA having an NruI site, where the P1 transcription start site is located approximately 0.10 Kb downstream of the NruI site, or any Streptomyces derivative thereof which functions in substantially the same way as the naturally occurring region in terms of regulatable production of the galT, galE and galK gene products.

9. The molecule of Claim 8 wherein the region is a S. coelicolor gal operon P1 promoter regulated region.

10. The molecule of Claim 8 or 9 which further comprises a foreign functional DNA sequence operatively linked to such regulated region.

11. A recombinant DNA molecule comprising a Streptomyces lividans gal operon P2 promoter located on a ca. 9kb HindIII-SphI fragment of S. lividans 1326 chromosomal DNA having an NruI site, where the P2 transcription start site is located approximately 1.25 Kb downstream of the NruI site, or any Streptomyces derivative thereof which functions in substantially the same way as the naturally occurring promoter in terms of enabling the binding of RNA polymerase thereto and transcription of a functional DNA sequence operatively linked to such promoter.

12. The molecule of Claim 11 wherein the promoter is a S. coelicolor gal operon P2 promoter.

13. The molecule of Claim 11 or 12 which further comprises a foreign functional DNA sequence operatively linked to the P2 promoter.

14. A recombinant DNA molecule comprising a Streptomyces lividans gal operon P1 promoter located on a ca. 9kb HindIII-SphI fragment of S. lividans 1326 chromosomal DNA having an NruI site, where the P1 transcription start site is located approximately 0.10 Kb downstream of the NruI site, or any Streptomyces derivative thereof which functions in substantially the same way as the naturally occurring promoter in terms of enabling the binding of RNA polymerase thereto and transcription of a functional DNA sequence operatively linked to such promoter.

15. The molecule of Claim 14 wherein the promoter is a S. coelicolor gal operon P1 promoter.

16. The molecule of Claim 14 or 15 which further comprises a foreign functional DNA sequence operatively linked to the P1 promoter.

17. A transformed host microorganism or cell comprising the molecule of any of Claims 4, 7, 10, 13 or 16.

18. A method of preparing a transformed host microorganism or cell comprising the molecule of any of Claims 4, 7, 10, 13 or 16 which comprises transforming an appropriate host microorganism or cell with such molecule.

19. A recombinant DNA vector comprising the molecule of any of Claims 4, 7, 10, 13 or 16, and, optionally, additionally comprising a replicon.

20. A transformed host microorganism or cell comprising the recombinant DNA vector of Claim 19.

21. A method of preparing a transformed host microorganism or cell comprising the recombinant DNA vector of Claim 19 which comprises transforming an appropriate host microorganism or cell with such vector.

22. A method of expressing a foreign functional DNA sequence which comprises cultivating a transformed host microorganism or cell comprising the recombinant DNA vector of Claim 19 under suitable conditions such that the functional DNA sequence is expressed.

23. A method of regulating the expression of a foreign functional DNA sequence which comprises cultivating a transformed host microorganism or cell which contains the recombinant DNA vector of Claim 19 comprising the molecule of claim 4, 10 or 16 under appropriate conditions such that expression of the sequence is regulatable.

**Claims for the following Contracting States : AT, ES**

1. A method of preparing a recombinant DNA molecule comprising a <u>Streptomyces</u> <u>lividans</u> <u>gal</u> operon located on a ca. 9kb HindlII-SphI fragment of <u>S. lividans</u> 1326 chromosomal DNA or any <u>Streptomyces</u> derivative thereof which functions in substantially the same way as the naturally occurring <u>Strep-</u><u>tomyces</u> <u>gal</u> operon in terms of regulatable production of the <u>gal</u>T, <u>gal</u>E and <u>gal</u>K gene products, which method comprises fusing appropriate DNA fragments.

2. The method of Claim 1 wherein the operon is a <u>S. coelicolor</u> <u>gal</u> operon.

3. The method of Claim 1 wherein the operon has the following coding sequence:

```
        -120          -110          -100           -90           -80            -70
          •             •             •             •             •              •
  CTA CGC CTC CGC GTT CAC TAA TTC AAC ACT TTT GGT GAT GAA CTT TGT TTG ATT CTC


           -60           -50           -40           -30           -20
            •             •             •             •             •
  ATC TGA CAG CCC CGT CGT CGC TTC TGA TGT GTT ATG TTT GAT TGT GTT GGA TGA TTG
                                                            ·galP1

      -10            1            10            20            30            40
        •            •             •             •             •             •
  ACG CGC GTC CTG GTG ACT CAT GGG TGG GTC CAG AGG AGT GCG GCA GTG AAG AAG ACC
                  Met Thr His Gly Trp Val Gln Arg Ser Ala Ala Val Lys Lys Thr
                  galT
        50            60            70            80            90           100
         •             •             •             •             •             •
  TCG ACC CGG CTG GCC GAC GGC CGT GAG CTG GTC TAC TAC GAC CTG CGC GAC GAC ACC
  Ser Thr Arg Leu Ala Asp Gly Arg Glu Leu Val Tyr Tyr Asp Leu Arg Asp Asp Thr

          110           120           130           140           150
           •             •             •             •             •
  GTG CGC GAC GCC GTG GAC CGC CGT CCG CTC GAG CGG ACC GTC ACC ACG TCC GAG GTG
  Val Arg Asp Ala Val Asp Arg Arg Pro Leu Glu Arg Thr Val Thr Thr Ser Glu Val

  160           170           180           190           200           210
   •             •             •             •             •             •
  CGA CGC GAC CCG CTG CTC GGC GAC TCC GCG CCG TCG CGC CTC GCA CCG GCA GGG GCC
  Arg Arg Asp Pro Leu Leu Gly Asp Ser Ala Pro Ser Arg Leu Ala Pro Ala Gly Ala

        220           230           240           250           260           270
         •             •             •             •             •             •
  CAC CTA CCA TCC GCC GGC CGA CCA GTG CCC GCT GTG CCc GTC GGA CGG GGA ACG GCT
  His Leu Pro Ser Ala Gly Arg Pro Val Pro Ala Val Pro Val Gly Arg Gly Thr Ala

        280           290           300           310           320           330
         •             •             •             •             •             •
  GAG CGA GAT CCG GCC TAT GAC GTG GTG GTC TTC GAG AAT CGC TTT CCC TCG CTG GCC
  Glu Arg Asp Pro Ala Tyr Asp Val Val Val Phe Glu Asn Arg Phe Pro Ser Leu Ala
```

```
                340            350            360            370            380
                 •              •              •              •              •
GGT GAC TCC GGC CGC TGC GAG GTC GTC TGC TTC ACC TCC GAC CAC GAC GCC TCC TTC
Gly Asp Ser Gly Arg Cys Glu Val Val Cys Phe Thr Ser Asp His Asp Ala Ser Phe

       390            400            410            420            430            440
        •              •              •              •              •              •
GCC GAC CTG AGC GAG GAG CAG GCC CGG CTG GTC GTC GAC GCC TGG ACG GAC CGC ACC
Ala Asp Leu Ser Glu Glu Gln Ala Arg Leu Val Val Asp Ala Trp Thr Asp Arg Thr

          450            460            470            480            490            500
           •              •              •              •              •              •
TCC GAG CTC TCC CAT CTG CCC TCC GTT GAA CAG GTC TTC TGC TTC GAG AAC CGG GGC
Ser Glu Leu Ser His Leu Pro Ser Val Glu Gln Val Phe Cys Phe Glu Asn Arg Gly

             510            520            530            540            550
              •              •              •              •              •
GCC GAG ATC GGG GTG ACC CTG GGT CAC CCG CAC GGG CAG ATC TAC GCC TAC CCG TTC
Ala Glu Ile Gly Val Thr Leu Gly His Pro His Gly Gln Ile Tyr Ala Tyr Pro Phe

  560            570            580            590            600            610
   •              •              •              •              •              •
ACC ACC CCC CGC ACC GCC CTG ATG CTC CGT TCA CTC GCC GCC CAC AAG GAC GCG ACG
Thr Thr Pro Arg Thr Ala Leu Met Leu Arg Ser Leu Ala Ala His Lys Asp Ala Thr

     620            630            640            650            660            670
      •              •              •              •              •              •
GGC GGG GGG AAC CTG TTC GAC TCC GTG CTG GAG GAG GAG CTG GCC GGT GAG CGC GTC
Gly Gly Gly Asn Leu Phe Asp Ser Val Leu Glu Glu Glu Leu Ala Gly Glu Arg Val

        680            690            700            710            720
         •              •              •              •              •
GTC CTG GAG GGT GAG CAC TGG GCC GCC TTC GTC GCG TAC GGC GCC CAC TGG CCG TAC
Val Leu Glu Gly Glu His Trp Ala Ala Phe Val Ala Tyr Gly Ala His Trp Pro Tyr

730            740            750            760            770            780
 •              •              •              •              •              •
GAG GTG CAC CTC TAC CCG AAG CGG CGG GTG CCC GAT CTG CTC GGG CTC GAC GAG GCC
Glu Val His Leu Tyr Pro Lys Arg Arg Val Pro Asp Leu Leu Gly Leu Asp Glu Ala

   790            800            810            820            830            840
    •              •              •              •              •              •
GCT CGC ACA GAA TTC CCC AAG GTC TAC CTG GAG CTG CTG AGG CGT TTC GAC CGC ATC
Ala Arg Thr Glu Phe Pro Lys Val Tyr Leu Glu Leu Leu Arg Arg Phe Asp Arg Ile
```

```
        850          860          870          880          890          900
         •            •            •            •            •            •
TTC GGC GAG GGC GAG CCC CCG ACC CCC TAC ATC GCC GCC TGG CAC CAG GCC CCG TTC
Phe Gly Glu Gly Glu Pro Pro Thr Pro Tyr Ile Ala Ala Trp His Gln Ala Pro Phe

              9            920          930          940          950
              •            •            •            •            •
GGC CAG CTG GAG TTC GAG GGT GTC ACG CGC GAC GAC TTC GCG CTC CAC CTG GAA CTT
Gly Gln Leu Glu Phe Glu Gly Val Thr Arg Asp Asp Phe Ala Leu His Leu Glu Leu

    960          970          980          990          1000         1010
     •            •            •            •            •            •
TTC ACT TCC GCC GTA CGT CCG GCA AGC TGA AGT TCC TCG CGC GCT CCG AAT CCG GCA
Phe Thr Ser Ala Val Arg Pro Ala Ser --- ¯galP2

      1020         1030         1040         1050         1060         1070
       •            •            •            •            •            •
   TGAACC TCTTCATCAA CGACGTACCG CCGGACCGCG CGGCCGAGCG ACTGCCAGAG GTAGCGAG


1080         1090         1100         1110         1120         1130
 •            •            •            •            •            •
TTC ATC AGC GGG AAC TAC CTC GTG ACA GGT GGT GCC GGA TAC GTC GGC AGC GTC GTC
Met Ser Gly Lys Tyr Leu Val Thr Gly Gly Ala Gly Tyr Val Gly Ser Val Val
galE
   1140         1150         1160         1170         1180         1190
    •            •            •            •            •            •
GCC CAG CAC TTG GTG GAG GCG GGC AAC GAG GTC GTC GTG CTC CAC AAT CTG TCG ACC
Ala Gln His Leu Val Glu Ala Gly Asn Glu Val Val Val Leu His Asn Leu Ser Thr

      1200         1210         1220         1230         1240
       •            •            •            •            •
GGC TTC CGT GAG GTG TGC CGG CGG GTG CCT CGT TCG TCG AGG CGA CAT CCG GGA CGC
Gly Phe Arg Glu Val Cys Arg Arg Val Pro Arg Ser Ser Arg Arg His Pro Gly Arg

1250         1260         1270         1280         1290         1300
 •            •            •            •            •            •
CGC CAA GTG CGT GGA CGG CTC TCG TTC GAC GGC GTG CTG CAC TTC GCC GCC TTC TCC
Arg Gln Val Arg Gly Arg Leu Ser Phe Asp Gly Val Leu His Phe Ala Ala Phe Ser

   1310         1320         1330         1340         1350         1360
    •            •            •            •            •            •
CAG GTC GGC GAG TCG GTC GTG AAG CCC GAG AAG TAC TGG GAC AAC AAC GTC GGT GGC
Gln Val Gly Glu Ser Val Val Lys Pro Glu Lys Tyr Trp Asp Asn Asn Val Gly Gly
```

```
            1370          1380          1390          1400          1410          1420
             •             •             •             •             •             •
ACC ATC GCC CTC CTC GAC GCC ATG CGC GGC GCC GGT GTC CGC CGC CTC GTC TTC TCC
Thr Met Ala Leu Leu Glu Ala Met Arg Gly Ala Gly Val Arg Arg Leu Val Phe Ser

                1430          1440          1450          1460          1470
                 •             •             •             •             •
TCC ACG GCC GCC ACG TAC GGC GAC CCC GAG CAG GTT CCC ATC GTC GAG TCC GCG CCG
Ser Thr Ala Ala Thr Tyr Gly Glu Pro Glu Gln Val Pro Ile Val Glu Ser Ala Pro

   1480          1490          1500          1510          1520          1530
    •             •             •             •             •             •
ACG AGG CCC ACC AAT CCG TAC GGC GCC TCG AAG CTC GCC GTC GAC CAC ATG ATC ACC
Thr Arg Pro Thr Asn Pro Tyr Gly Ala Ser Lys Leu Ala Val Asp His Met Ile Thr

      1540          1550          1560          1570          1580          1590
       •             •             •             •             •             •
GGC GAG GCG GCG GCC CAC GGG CTG GGC GCG GTC TCC GTG CCG TAC TTC AAC GTC GCG
Gly Glu Ala Ala Ala His Gly Leu Gly Ala Val Ser Val Pro Tyr Phe Asn Val Ala

         1600          1610          1620          1630          1640
          •             •             •             •             •
GGC GCG TAC GGG GAG TAC GGC GAG CGC CAC GAC CCC GAG TCG CAT CTC ATT CCG CTG
Gly Ala Tyr Gly Glu Tyr Gly Glu Arg His Asp Pro Glu Ser His Leu Ile Pro Leu

1650          1660          1670          1680          1690          1700
 •             •             •             •             •             •
GTC CTT CAA GTG GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

      1710          1720          1730          1740          1750          1760
       •             •             •             •             •             •
CCG ACC CCG GAC CGA CCT GTG TGC GCG ACT ACA TCC ACG TCG CCG ACC TGG CCG AGG
Pro Thr Pro Asp Arg Pro Val Cys Ala Thr Thr Ser Thr Ser Pro Thr Trp Pro Arg

         1770          1780          1790          1800          1810
          •             •             •             •             •
CCC ACC TGC TGG CCG TGC GCC GCC GCC CCG GGC GAG CAC CTC ATC TGC AAC CTG GGC
Pro Thr Cys Trp Pro Cys Ala Ala Ala Pro Gly Glu His Leu Ile Cys Asn Leu Gly

1820          1830          1840          1850          1860          1870
 •             •             •             •             •             •
AAC GGC AAC GGC TTC TCC GTC CGC GAG GTC GTC GAG ACC GTG CGC CGC GTC ACG GGC
Asn Gly Asn Gly Phe Ser Val Arg Glu Val Val Glu Thr Val Arg Arg Val Thr Gly
```

```
        1880          1890          1900          1910          1920          1930
         •            •            •            •            •            •
CAT CCC ATC CCC GAG ATC ATG GCC CCC CGC CGC GGC CGC GAC CCG GCG CTC CTC CTC
His Pro Ile Pro Glu Ile Met Ala Pro Arg Arg Gly Arg Asp Pro Ala Val Leu Val

            1940          1950          1960          1970          1980          1990
             •            •            •            •            •            •
GCG TCC GCC GGC ACC GCC CGC GAG AAG CTC GGC TGG AAC CCG TCC CGC GCG GAC CTC
Ala Ser Ala Gly Thr Ala Arg Glu Lys Leu Gly Trp Asn Pro Ser Arg Ala Asp Leu

                2000          2010          2020          2030          2040
                 •            •            •            •            •
GCC ATC GTC TCG GAC GCG TGG GAG TTC CCG CAG CGC CGC GCG GGC CAG TAG   TA
Ala Ile Val Ser Asp Ala Trp Glu Leu Pro Gln Arg Arg Ala Gly Gln ---

            2050          2060          2070          2080          2090          2100
             •            •            •            •            •            •
ACC GCA GTT ACC GGA AAG GCG ACG GGT CAG GGC ATG GGC GAG GCT GTC GGG GAA CCG
                                            Met Gly Glu Ala Val Gly Glu Pro
                                            galK
            2110          2120          2130          2140          2150
             •            •            •            •            •
TCG GCG AGC GGT TCC GGG AGC TGT ACG GGG CGG AGC CGG AGG GGG TGT GCG CGC CGA
Ser Ala Ser Gly Ser Gly Ser Cys Thr Gly Arg Ser Arg Arg Gly Cys Gly Arg Arg

  2160          2170          2180          2190          2200          2210
   •            •            •            •            •            •
GCG GGC CCG GAG AAC CTC ATC GGG GAG CAC ACC GAC TAC AAC GAC GGC TTC GTC ATG
Ala Gly Arg Glu Asn Leu Ile Gly Glu His Thr Asp Tyr Asn Asp Gly Phe Val Met

     2220          2230          2240          2250          2260          2270
      •            •            •            •            •            •
CCT TCG CCC TGC CGC ACC AGG TCG CGG CCG TCT CCC GGC GCG AAC GAC GGC ATC CTG
Pro Ser Pro Cys Arg Thr Arg Ser Arg Pro Ser Pro Gly Ala Asn Asp Gly Ile Leu

            2280          2290          2300          2310          2320
             •            •            •            •            •
CGC CTG CAC TCG GCC GAC GTC GAC GCC GAC CCG GTC GAG CTG CGC GTC GCC GAC CTG
Arg Leu His Ser Ala Asp Val Asp Ala Asp Pro Val Glu Leu Arg Val Ala Asp Leu

  2330          2340          2350          2360          2370          2380
   •            •            •            •            •            •
GCC CCC GCG TCG GAC AAG TCC TGG ACG GCG TAC CCC TCG GGC GTC CTG TGG GCG CTG
Ala Pro Ala Ser Asp Lys Ser Trp Thr Ala Tyr Pro Ser Gly Val Leu Trp Ala Leu
```

```
      2390          2400          2410          2420          2430          2440
       •            •             •             •             ..            •
CGC GAG GCC GGA CAC GAG CTG ACC GGC GCC GAC GTC CAC CTG GCC TCG ACC GTC CCG
Arg Glu Ala Gly His Glu Leu Thr Gly Ala Asp Val His Leu Ala Ser Thr Val Pro

            2450          2460          2470          2480          2490
             •            •             •             •             •
TCC GGG GCG GGG CTC TCC TCC TCC GCG GCC CTG GAG GTC CGT CCC CTG GCG ATG AAC
Ser Gly Ala Gly Leu Ser Ser Ser Ala Ala Leu Glu Val Arg Pro Leu Ala Met Asn

 2500          2510          2520          2530          2540          2550
  •            •             •             •             •             •
GAC CTC TAC GCC CTC GCC CTG CGC GGC TGG CAG CTG GCC CGG CTG TGC CAG CGC GCG
Asp Leu Tyr Ala Leu Ala Leu Arg Gly Trp Gln Leu Ala Arg Leu Cys Gln Arg Ala

    2560          2570          2580          2590          2600          2610
     •            •             •             •             •             •
GAG AAC GTC TAC GTC GGC GCC CCC GTC GGC ATC ATG GAC CAG ACG GCG TCC GCC TGC
Glu Asn Val Tyr Val Gly Ala Pro Val Gly Ile Met Asp Gln Thr Ala Ser Ala Cys

       2620          2630          2640          2650          2660          2670
        •             •             •             •             •             •
TGC GAG GCG GGC ACC CCC TCT TCC TCG ACA CCC GCG ACC TCT CCC AGC GGC AGA TCC
Cys Glu Ala Gly Thr Pro Ser Ser Ser Thr Pro Ala Thr Ser Pro Ser Gly Arg Ser

          2680          2690          2700          2710          2720
           •             •             •             •             •
CCT TCG ACC TCG CCG CCG AGG GGA TGC GCC TGC TGG TCG TCG ACA CCC GGG TCA AGC
Pro Ser Thr Ser Pro Pro Arg Gly Cys Ala Cys Trp Ser Ser Thr Pro Gly Ser Ser

 2730          2740          2750          2760          2770          2780
  •            •             •             /•            •             •
ACT CCC ACA GCG AGG GCG AGT ACG GCA AGC GCC GCG CGC GCT CCG AGA AGG GCG CCG
Thr Pro Thr Ala Arg Ala Ser Thr Ala Ser Ala Ala Arg Ala Ala Arg Arg Ala Pro

       2790          2800          2810          2820          2830          2840
        •             •             •             •             •             •
CGC TGC TGG GCC TCG ACG CGC TGC GAC GTG CCC TAC GCC GAC CTC GAC GCG GCG CTG
Arg Cys Trp Ala Ser Thr Arg Cys Asp Val Pro Tyr Ala Asp Leu Asp Ala Ala Leu

          2850          2860          2870          2880          2890
           •             •             •             •             •
GAG CGG CTG GGC GAC GAG GAG GAG-GTG CGC CGC CTG GTG CGG CAC GTG GTG ACC GAG
Glu Arg Leu Gly Asp Glu Glu Glu Val Arg Arg Leu Val Arg His Val Val Thr Glu
```

```
         2900            2910            2920            2930            2940            2950
          •               •               •               •               •               •
      GAC GAG CGC GTC GAA CGG GTC GTC CCC CTG CTC GAG TCC GCC ACA CCC GGC GCA TCC
      Asp Glu Arg Val Glu Arg Val Val Ala Leu Leu Glu Ser Ala Thr Pro Gly Ala Ser

             2960            2970            2980            2990            3000            3010
              •               •               •               •               •               •
      GCG CCC TCC TGG TCC AGG GCC ACG CCT GCT GCG CGA CGA CTT CCG CAT CTC CTG CCC
      Ala Pro Ser Trp Ser Arg Ala Thr Pro Ala Ala Arg Arg Leu Pro His Leu Leu Pro

             3020            3030            3040            3050            3060
              •               •               •               •               •
      CGA GCT GGA CCT GGT CCT CGA CAC GGC CCT GGC CTC CGC GGC CCT CGC CGC CGG ATG
      Arg Ala Gly Pro Gly Arg Arg His Gly Pro Gly Leu Arg Gly Pro Arg Arg Arg Met

      3070            3080            3090            3100            3110            3120
       •               •               •               •               •               •
      ACC GGC GGC GGC TTC GGC GGC TCG GCG ATC GTC CTG GTG GAG GCC GCC GCG GTG GAC
      Thr Gly Gly Gly Phe Gly Gly Ser Ala Ile Val Leu Val Glu Ala Ala Ala Val Asp

         3130            3140            3150            3160            3170            3180
          •               •               •               •               •               •
      GCC GTC ACC AAG GCG GTC GAG GAC GCC TTC GCC GCG GCG GGC CTC AAC CGT CCG CGG
      Ala Val Thr Lys Ala Val Glu Asp Ala Phe Ala Ala Ala Gly Leu Lys Arg Pro Arg

             3190            3200            3210            3220            3230            3240
              •               •               •               •               •               •
      GTG TTC GAG GCG GTG CCT CGG CCG GGC GCG GCG CCT GGT CTG ACG GTC AGC CGA GCC
      Val Phe Glu Ala Val Pro Arg Arg Gly Ala Ala Pro Gly Leu Thr Val Ser Arg Ala

             3250            3260            3270            3280            3290
              •               •               •               •               •
      GCT TCA CCA GCG TGT ACT CCC TGA TCC CCG GCG GGT AGT CGG GGA TCA CGC ACA TGA
      Ala Ser Pro Ala Cys Thr Pro ---

      3300
       •
      GCT GCT ACC CGC
```

which comprises transforming an appropriate host microorganism or cell with such molecule.

4. The method of any of Claims 1 to 3 wherein the molecule further comprises a foreign functional DNA sequence operatively linked to such operon.

5. A method of preparing a recombinant DNA molecule comprising a Streptomyces lividans gal operon P2 promoter expression unit located on a ca. 9kb HindIII-SphI fragment of S. lividans 1326 chromosomal DNA having an NruI site, where the P2 transcription start site is located approximately 1.25 Kb downstream of the NruI site, or any derivative thereof which functions in substantially the same way as the naturally occurring unit in terms of production of the galE and galK gene products, which method comprises fusing appropriate DNA fragments.

6. The method of Claim 5 wherein the expression unit is a S. coelicolor gal operon P2 promoter expression unit.

7. The method of Claim 5 or 6 wherein the molecule further comprises a foreign functional DNA sequence operatively linked to such expression unit.

8. A method of preparing a recombinant DNA molecule comprising a Streptomyces lividans gal operon P1 promoter regulated region located on a ca. 9kb HindIII-SphI fragment of S. lividans 1326 chromosomal DNA having an NruI site, where the P1 transcription start site is located approximately 0.10Kb

downstream of the NruI site, or any Streptomyces derivative thereof which functions in substantially the same way as the naturally occurring region in terms of regulatable production of the galT, galE and galK gene products, which method comprises fusing appropriate DNA fragments.

9. The method of Claim 8 wherein the region is a S. coelicolor gal operon P1 promoter regulated region.

10. The method of Claim 8 or 9 wherein the molecule further comprises a foreign functional DNA sequence operatively linked to such regulated region.

11. A method of preparing a recombinant DNA molecule comprising a Streptomyces lividans gal operon P2 promoter located on a ca. 9kb HindIII-SphI fragment of S. lividans 1326 chromosomal DNA having an NruI site, where the P2 transcription start site is located approximately 1.25Kb downstream of the NruI site, or any Streptomyces derivative thereof which functions in substantially the same way as the naturally occurring promoter in terms of enabling the binding of RNA polymerase thereto and transcription of a functional DNA sequence operatively linked to such promoter, which method comprises fusing appropriate DNA fragments.

12. The method of Claim 11 wherein the promoter is a S. coelicolor gal operon P2 promoter.

13. The method of Claim 11 or 12 wherein the molecule further comprises a foreign functional DNA sequence operatively linked to the P2 promoter.

14. A method of preparing a recombinant DNA molecule comprising a Streptomyces lividans gal operon P1 promoter located on a ca. 9kb HindIII-SphI fragment of S. lividans 1326 chromosomal DNA having an NruI site, where the P1 transcription start site is located approximately 0.10Kb downstream of the NruI site, or any Streptomyces derivative thereof which functions in substantially the same way as the naturally occurring promoter in terms of enabling the binding of RNA polymerase thereto and transcription of a functional DNA sequence operatively linked to such promoter, which method comprises fusing appropriate DNA fragments.

15. The method of Claim 14 wherein the promoter is a S. coelicolor gal operon P1 promoter.

16. The method of Claim 14 or 15 wherein the molecule further comprises a foreign functional DNA sequence operatively linked to the P1 promoter.

17. A method of preparing a transformed host microorganism or cell comprising the molecule of any of Claims 4, 7, 10, 13 or 16 which comprises transforming an appropriate host microorganism or cell with such molecule.

18. A method of preparing a recombinant DNA vector comprising the molecule of any of Claims 4, 7, 10, 13 or 16, and, optionally, additionally comprising a replicon, which method comprises fusing appropriate DNA fragments.

19. A method of preparing a transformed host microorganism or cell comprising the recombinant DNA vector of Claim 18 which comprises transforming an appropriate host microorganism with such vector.

20. A method of expressing a foreign functional DNA sequence which comprises cultivating a transformed host microorganism or cell comprising the recombinant DNA vector of Claim 18 under suitable conditions such that the functional DNA sequence is expressed.

21. A method of regulating the expression of a foreign functional DNA sequence which comprises cultivating a transformed host microorganism or cell which contains the recombinant DNA vector of Claim 18 comprising the molecule of Claim 4, 10 or 16 under appropriate conditions such that expression of the sequence is regulatable.

# EP 0 235 112 B1

**Patentansprüche**
**Patentansprüche für folgende Vertragsstaaten : BE, CH, DE, FR, GB, IT, LI, LU, NL, SE, GR**

1. Rekombinantes DNA-Molekül, umfassend ein Streptomyces lividans-gal-Operon, das sich auf einem etwa 9 kb großen HindIII-SphI-Fragment der chromosomalen DNA von S. lividans 1326 befindet, oder ein beliebiges Streptomyces-Derivat davon, das in bezug auf die regulierbare Produktion der galT-, galE- und galK-Genprodukte im wesentlichen in der gleichen Weise funktioniert wie das natürlich vorkommende Streptomyces-gal-Operon.

2. Molekül nach Anspruch 1, wobei das Operon ein S. coelicolor-gal-Operon ist.

3. Molekül nach Anspruch 1 mit der folgenden codierenden Sequenz:

```
        -120        -110        -100        -90        -80        -70
         •           •           •           •          •          •
CTA CGC CTC CGC GTT CAC TAA TTC AAC ACT TTT GGT GAT GAA CTT TCT TTC ATT GTG


              -60         -50         -40        -30        -20
               •           •           •          •          •
ATG TGA CAC CCG CGT GGT GCC TTC TGA TGT GTT ATG TTT GAT TGT GTT GGA TGA TTG
                                                       -galP1


  -10           1          10          20         30         40
   •            •           •           •          •          •
ACG GGC GTC CTC GTG ACT CAT GGG TGG GTG CAC AGG AGT GCG GCA GTG AAG AAG ACC
                Met Thr His Gly Trp Val Gln Arg Ser Ala Ala Val Lys Lys Thr
                galT
       50          60          70         80         90        100
        •           •           •          •          •          •
TCG ACC CGG CTC GCC GAC GGC CGT GAG CTC GTC TAC TAC GAC CTG CGC GAC GAC ACC
Ser Thr Arg Leu Ala Asp Gly Arg Glu Leu Val Tyr Tyr Asp Leu Arg Asp Asp Thr

          110         120         130        140        150
           •           •           •          •          •
GTC CGC GAC GCC GTG GAC CGC CGT CCG CTC GAG CGC ACC GTC ACC ACG TCC GAG GTC
Val Arg Asp Ala Val Asp Arg Arg Pro Leu Glu Arg Thr Val Thr Thr Ser Glu Val

160         170         180         190        200        210
 •           •           •           •          •          •
CGA CGC GAC CCG CTG CTC GGC GAC TCC GCG CCG TCC CGC CTC GCA CCG GCA GGC GCC
Arg Arg Asp Pro Leu Leu Gly Asp Ser Ala Pro Ser Arg Leu Ala Pro Ala Gly Ala

   220         230         240        250        260        270
    •           •           •          •          •          •
CAC CTA CCA TCC GCC GGC CGA CCA GTG CCC GCT GTC CCc GTC GGA CGC GGA ACG GCT
His Leu Pro Ser Ala Gly Arg Pro Val Pro Ala Val Pro Val Gly Arg Gly Thr Ala

      280         290         300        310        320        330
       •           •           •          •          •          •
GAG CGA GAT CCG GCC TAT GAC GTC GTG GTC TTC GAG AAT CGC TTT CCC TCG CTC GCC
Glu Arg Asp Pro Ala Tyr Asp Val Val Val Phe Glu Asn Arg Phe Pro Ser Leu Ala
```

43

```
        340           350           360           370           380
         •             •             •             •             •
GGT GAC TCC GGG CGC TGC GAG GTC CTC TGC TTC ACC TCC GAC CAC GAC GCC TCC TTC
Gly Asp Ser Gly Arg Cys Glu Val Val Cys Phe Thr Ser Asp His Asp Ala Ser Phe

     390           400           410           420           430           440
      •             •             •             •             •             •
GCC GAC CTG ACC GAG GAG CAG GCC CGG CTG GTC GTC GAC GCC TGG ACG GAC CGC ACC
Ala Asp Leu Ser Glu Glu Gln Ala Arg Leu Val Val Asp Ala Trp Thr Asp Arg Thr

        450           460           470           480           490           500
         •             •             •             •             •             •
TCC GAG CTC TCC CAT CTG CCC TCC GTT GAA CAG GTC TTC TGC TTC GAG AAC CGG GCC
Ser Glu Leu Ser His Leu Pro Ser Val Glu Gln Val Phe Cys Phe Glu Asn Arg Gly

           510           520           530           540           550
            • •           •             •             •             •
GCC GAG ATC GGG GTG ACG CTG GGT CAC CCG CAC GGG CAG ATC TAC GCC TAC CCG TTC
Ala Glu Ile Gly Val Thr Leu Gly His Pro His Gly Gln Ile Tyr Ala Tyr Pro Phe

     560           570           580           590           600           610
      •             •             •             •             •             •
ACC ACC CCC CGC ACC GCC CTG ATG CTC CGT TCA CTC GCC GCC CAC AAG GAC GCG ACG
Thr Thr Pro Arg Thr Ala Leu Met Leu Arg Ser Leu Ala Ala His Lys Asp Ala Thr

        620           630           640           650           660           670
         •             •             •             •             •             •
GGC GGG GGG AAC CTC TTC GAC TCC GTC CTC GAG GAG GAG CTG GCC GGT GAG CGG GTC
Gly Gly Gly Asn Leu Phe Asp Ser Val Leu Glu Glu Glu Leu Ala Gly Glu Arg Val

        680           690           700           710           720
         •             •             •             •             •
CTC CTG GAG GGT GAG CAC TGG GCC GCC TTC GTC GCG TAC GGC GCG CAC TGG CCG TAC
Val Leu Glu Gly Glu His Trp Ala Ala Phe Val Ala Tyr Gly Ala His Trp Pro Tyr

 730           740           750           760           770           780
  •             •             •             •             •             •
GAG GTG CAC CTC TAC CCG AAG CGG CGG GTG CCC GAT CTG CTC GGG CTC GAC GAG GCG
Glu Val His Leu Tyr Pro Lys Arg Arg Val Pro Asp Leu Leu Gly Leu Asp Glu Ala

     790           800           810           820           830           840
      •             •             •             •             •             •
GCT CGC ACA GAA TTC CCC AAG GTC TAC CTC GAG CTG CTG AGG CGT TTC GAC CGC ATC
Ala Arg Thr Glu Phe Pro Lys Val Tyr Leu Glu Leu Leu Arg Arg Phe Asp Arg Ile
```

44

```
        850              850              870              880              890              900
         •                •                •                •                •                •
   TTC GGC CAG CGC GAG CCC CCG ACC CCC TAC ATC GCC GCC TGG CAC CAG GCC CCC TTC
   Phe Gly Glu Gly Glu Pro Pro Thr Pro Tyr Ile Ala Ala Trp His Gln Ala Pro Phe

              9.               920              930              940              950
               •                •                •                •                •
   GGC CAG CTG GAG TTC GAG GGT GTC ACG CGC GAC GAC TTC GCG CTC CAC CTG GAA CTT
   Gly Gln Leu Glu Phe Glu Gly Val Thr Arg Asp Asp Phe Ala Leu His Leu Glu Leu

      960              970              980              990              1000             1010
       •                •                •                •                •                •
   TTC ACT TCC GCC GTA CGT CCC GCA AGC TGA AGT TCC TCG CGC GCT CCC AAT CCG GCA
   Phe Thr Ser Ala Val Arg Pro Ala Ser ---              ⁻galP2

          1020     1030     1040     1050     1060     1070
           •        •        •        •        •        •
        TCAACC TGTTCATCAA CGACGTACCC CCCGAGCGCC CGGCCCAGCG ACTCCGAGAG GTAGCGAG

       1080             1090             1100             1110             1120             1130
        •                •                •                •                •                •
   TTC ATC AGC GGG AAG TAC CTG GTG ACA GGT GGT GCC GGA TAC GTC GGC AGC GTC CTC
       Met Ser Gly Lys Tyr Leu Val Thr Gly Gly Ala Gly Tyr Val Gly Ser Val Val
       galE
       1140             1150             1160             1170             1180             1190
        •                •                •                •                •                •
   GCC CAG CAC TTG GTG GAG GCG GGC AAC GAG GTC GTG GTG CTC CAC AAT CTG TCG ACC
   Ala Gln His Leu Val Glu Ala Gly Asn Glu Val Val Val Leu His Asn Leu Ser Thr

          1200             1210             1220             1230             1240
           •                •                •                •                •
   GGC TTC CGT GAG GTG TGC CGG CGG GTC CCT CGT TCG TCC AGG CGA CAT CCC GGA CGC
   Gly Phe Arg Glu Val Cys Arg Arg Val Pro Arg Ser Ser Arg Arg His Pro Gly Arg

   1250             1260             1270             1280             1290             1300
    •                •                •                •                •                •
   CGC CAA GTG CGT GGA CGG CTC TCC TTC GAC GGC GTG CTC CAC TTC GCC GCC TTC TCC
   Arg Gln Val Arg Gly Arg Leu Ser Phe Asp Gly Val Leu His Phe Ala Ala Phe Ser

       1310             1320             1330             1340             1350             1360
        •                •                •                •                •                •
   CAG CTC GGC GAG TCG GTC GTG AAG CCC GAG AAG TAC TGG GAC AAC AAC GTC GGT GGC
   Gln Val Gly Glu Ser Val Val Lys Pro Glu Lys Tyr Trp Asp Asn Asn Val Gly Gly
```

```
        1370          1380          1390          1400          1410          1420
          .             .             .             .             .             .
ACC ATC GCC CTC CTC GAC GCC ATC CGC GGC CCC GGT GTC CGC CGC CTC CTC TTC TCC
Thr Met Ala Leu Leu Glu Ala Met Arg Gly Ala Gly Val Arg Arg Leu Val Phe Ser

              1430          1440          1450          1460          1470
                .             .             .             .             .
TCC ACC GCC GCC ACG TAC GGC GAG CCC GAG CAG GTT CCC ATC GTC GAG TCC GCC CCG
Ser Thr Ala Ala Thr Tyr Gly Glu Pro Glu Gln Val Pro Ile Val Glu Ser Ala Pro

    1480          1490          1500          1510          1520          1530
      .             .             .             .             .             .
ACG ACG CCC ACC AAT CCG TAC GGC GCC TCC AAG CTC GCC GTC GAC CAC ATG ATC ACC
Thr Arg Pro Thr Asn Pro Tyr Gly Ala Ser Lys Leu Ala Val Asp His Met Ile Thr

        1540          1550          1560          1570          1580          1590
          .             .             .             .             .             .
GGC GAG GCG GCC GCC CAC GGG CTC GGC GCC GTC TCC GTC CCG TAC TTC AAC GTC GCG
Gly Glu Ala Ala Ala His Gly Leu Gly Ala Val Ser Val Pro Tyr Phe Asn Val Ala

              1600          1610          1620          1630          1640
                .             .             .             .             .
GGC GCG TAC GGC GAG TAC GGC GAG CGC CAC GAC CCC GAG TCG CAT CTC ATT CCC CTG
Gly Ala Tyr Gly Glu Tyr Gly Glu Arg His Asp Pro Glu Ser His Leu Ile Pro Leu

    1650          1660          1670          1680          1690          1700
      .             .             .             .             .             .
GTC CTT CAA GTC GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

        1710          1720          1730          1740          1750          1760
          .             .             .             .             .             .
CCG ACC CCG GAC CGA CCT GTG TGC GGG ACT ACA TCC ACG TCG CCG ACC TCG CCG AGG
Pro Thr Pro Asp Arg Pro Val Cys Ala Thr Thr Ser Thr Ser Pro Thr Trp Pro Arg

        1770          1780          1790          1800          1810
          .             .             .             .             .
CCC ACC TGC TGG CCG TGC GCC GCC GCC CCG GGC GAG CAC CTC ATC TGC AAC CTG GGC
Pro Thr Cys Trp Pro Cys Ala Ala Ala Pro Gly Glu His Leu Ile Cys Asn Leu Gly

1820          1830          1840          1850          1860          1870
  .             .             .             .             .             .
AAC GGC AAC GGC TTC TCC GTC CGC GAG GTC GTC GAG ACC GTG CGC CGC GTG ACG GGC
Asn Gly Asn Gly Phe Ser Val Arg Glu Val Val Glu Thr Val Arg Arg Val Thr Gly
```

46

```
        1880        1890        1900        1910        1920        1930
         •           •           •           •           •           •
CAT CCC ATC CCC GAG ATC ATG GCC CCC CGC CGC GGG CGC GAC CCG GCC CTC CTC CTC
His Pro Ile Pro Glu Ile Met Ala Pro Arg Arg Gly Arg Asp Pro Ala Val Leu Val


            1940        1950        1960        1970        1980        1990
             •           •           •           •           •           •
GCG TCC GCC GGC ACC GCC CGC GAG AAG CTC GGC TGG AAC CCG TCC CGC GCG GAC CTC
Ala Ser Ala Gly Thr Ala Arg Glu Lys Leu Gly Trp Asn Pro Ser Arg Ala Asp Leu


                2000        2010        2020        2030        2040
                 •           •           •           •           •
GCC ATC GTG TCC GAC GCC TGG GAG TTG CCC CAG CGC CGC GCG CGC CAC TAC  TA
Ala Ile Val Ser Asp Ala Trp Glu Leu Pro Gln Arg Arg Ala Gly Gln ---


            2050        2060        2070        2080        2090        2100
             •           •           •           •           •           •
ACC GCA GTT ACC GGA AAG GCG ACG GGT CAG GGC ATG GGC GAG GCT GTC GGG GAA CCC
                                            Met Gly Glu Ala Val Gly Glu Pro
                                            galK

            2110        2120        2130        2140        2150
             •           •           •           •           •
TCC GCG AGC GGT TCC GGG AGC TGT ACG GGG CGG ACC CGG AGG GGG TGT GGG CGC CGA
Ser Ala Ser Gly Ser Gly Ser Cys Thr Gly Arg Ser Arg Arg Gly Cys Gly Arg Arg

2160        2170        2180        2190        2200        2210
 •           •           •           •           •           •
GCG GGC CGG GAG AAC CTC ATC GGG GAG CAC ACC GAC TAC AAC GAC GGC TTC GTC ATG
Ala Gly Arg Glu Asn Leu Ile Gly Glu His Thr Asp Tyr Asn Asp Gly Phe Val Met

        2220        2230        2240        2250        2260        2270
         •           •           •           •           •           •
CCT TCC CCC TGC CGC ACC AGG TCG CCC CCG TCT CCC GGC GCG AAC GAC GGC ATC CTG
Pro Ser Pro Cys Arg Thr Arg Ser Arg Pro Ser Pro Gly Ala Asn Asp Gly Ile Leu


            2280        2290        2300        2310        2320
             •           •           •           •           •
CGC CTC CAC TCC GCC GAC GTC GAC GCC GAC CCG GTC GAG CTG CGC GTC GCC GAC CTG
Arg Leu His Ser Ala Asp Val Asp Ala Asp Pro Val Glu Leu Arg Val Ala Asp Leu


2330        2340        2350        2360        2370        2380
 •           •           •           •           •           •
GCC CCC GCG TCG GAC AAG TCC TGG ACG GCG TAC CCC TCC GGC GTC CTG TGG GCG CTG
Ala Pro Ala Ser Asp Lys Ser Trp Thr Ala Tyr Pro Ser Gly Val Leu Trp Ala Leu
```

```
      2390            2400            2410            2420            2430            2440
        •               •               •               •               •               •
   CCC CAG CCC CGA CAC GAG CTC ACC GGC CCC GAC GTC CAC CTC GCC TCC ACC GTC CCC
   Arg Glu Ala Gly His Glu Leu Thr Gly Ala Asp Val His Leu Ala Ser Thr Val Pro

              2450            2460            2470            2480            2490
                •               •               •               •               •
   TCC GGG GCG GGC CTC TCC TCC TCC GCG GCC CTG GAG GTC CGT CCC CTG GCG ATG AAC
   Ser Gly Ala Gly Leu Ser Ser Ser Ala Ala Leu Glu Val Arg Pro Leu Ala Met Asn

    2500            2510            2520            2530            2540            2550
      •               •               •               •               •               •
   GAC CTG TAC GCC CTC GCG CTG CGC GGC TGG CAG CTG GCC CGG CTG TGC CAG CGC GCC
   Asp Leu Tyr Ala Leu Ala Leu Arg Gly Trp Gln Leu Ala Arg Leu Cys Gln Arg Ala

      2560            2570            2580            2590            2600            2610
        •               •               •               •               •               •
   GAG AAC GTC TAC GTC GGC GCC CCC GTC GGC ATC ATG GAC CAG ACG GCG TCC GCC TGC
   Glu Asn Val Tyr Val Gly Ala Pro Val Gly Ile Met Asp Gln Thr Ala Ser Ala Cys

              2620            2630            2640            2650            2660            2670
                •               •               •               •               •               •
   TGC GAG GCG GGC ACG CCC TCT TCC TCG ACA CCC GCG ACC TCT CCC ACC GGC AGA TCC
   Cys Glu Ala Gly Thr Pro Ser Ser Ser Thr Pro Ala Thr Ser Pro Ser Gly Arg Ser

              2680            2690            2700            2710            2720
                •               •               •               •               •
   CCT TCG ACC TCG CCG CCG AGG GGA TGC GCC TGC TGG TCC TCG ACA CCC GGC TCA AGC
   Pro Ser Thr Ser Pro Pro Arg Gly Cys Ala Cys Trp Ser Ser Thr Pro Gly Ser Ser

    2730            2740            2750            2760            2770            2780
      •               •               •               •               •               •
   ACT CCC ACA CCC AGG GCG AGT ACG GCA AGC GCC GCG CGG GCT GCG AGA AGC GCG CCG
   Thr Pro Thr Ala Arg Ala Ser Thr Ala Ser Ala Ala Arg Ala Ala Arg Arg Ala Pro

      2790            2800            2810            2820            2830            2840
        •               •               •               •               •               •
   CCC TGC TGG GCG TCG ACG CGC TGC GAC GTG CCC TAC GCC GAC CTG GAC GCC GCG CTG
   Arg Cys Trp Ala Ser Thr Arg Cys Asp Val Pro Tyr Ala Asp Leu Asp Ala Ala Leu

              2850            2860            2870            2880            2890
                •               •               •               •               •
   GAG CGG CTG GGC GAC GAG GAG GAG GTG CGC CGC CTG GTC CGG CAC GTG GTG ACC GAG
   Glu Arg Leu Gly Asp Glu Glu Glu Val Arg Arg Leu Val Arg His Val Val Thr Glu
```

48

```
        2900            2910            2920            2930            2940            2950
         •               •               •               •               •               •
    GAC GAG CGC GTC GAA CGC GTC GTC GCC CTC CTC GAG TCC GCC ACA CCC GGC GCA TCC
    Asp Glu Arg Val Glu Arg Val Val Ala Leu Leu Glu Ser Ala Thr Pro Gly Ala Ser

            2960            2970            2980            2990            3000            3010
             •               •               •               •               •               •
        GCC CCC TCC TGG TCG ACG GCC ACG CCT GCT GCG CGA CGA CTT CCG CAT CTC CTC CCC
        Ala Pro Ser Trp Ser Arg Ala Thr Pro Ala Ala Arg Arg Leu Pro His Leu Leu Pro

                3020            3030            3040            3050            3060
                 •               •               •               •               •
        CGA CCT GGA CCT GGT CGT CGA CAC GGC CCT CGC CTC CGC GGC CCT CGG CGC CGG ATG
        Arg Ala Gly Pro Gly Arg Arg His Gly Pro Gly Leu Arg Gly Pro Arg Arg Arg Met

    3070            3080            3090            3100            3110            3120
     •               •               •               •               •               •
    ACC GGC GGC GGC TTC GGC GGC TCC GCG ATC GTC CTC GTC GAG GCC GCC GCG GTC GAC
    Thr Gly Gly Gly Phe Gly Gly Ser Ala Ile Val Leu Val Glu Ala Ala Ala Val Asp

        3130            3140            3150            3160            3170            3180
         •               •               •               •               •               •
    GCC GTC ACC AAG GCG GTC GAG GAC GCC TTC GCG GCG GCG GGC CTC AAG CGT CCG CGG
    Ala Val Thr Lys Ala Val Glu Asp Ala Phe Ala Ala Ala Gly Leu Lys Arg Pro Arg

            3190            3200            3210            3220            3230            3240
             •               •               •               •               •               •
    GTG TTC GAG GCG GTG CCT CGG CGC GGC GCG GCG CCT GGT CTC ACC GTC AGC CGA GCC
    Val Phe Glu Ala Val Pro Arg Arg Gly Ala Ala Pro Gly Leu Thr Val Ser Arg Ala

                3250            3260            3270            3280            3290
                 •               •               •               •               •
    GCT TCA CCA GCG TGT ACT CCC TGA TCC CCG GCG GGT AGT CGG GGA TCA CGC ACA TGA
    Ala Ser Pro Ala Cys Thr Pro ---

    3300
     •
    GGT GGT AGC CGC
```

4. Molekül nach einem der Ansprüche 1 bis 3, das außerdem eine fremde funktionelle DNA-Sequenz umfaßt, die funktionell mit einem solchen Operon verknüpft ist.

5. Rekombinantes DNA-Molekül, umfassend eine Streptomyces lividans-gal-Operon-P2-Promotor-Expressionseinheit, die sich auf einem etwa 9 kb großen HindIII-SphI-Fragment der chromosomalen DNA von S. lividans 1326 mit einer NruI-Stelle befindet, wobei die P2-Transkriptionsstartstelle etwa 1,25 kb stromabwärts der NruI-Stelle liegt, oder ein beliebiges Streptomyces-Derivat davon, das in bezug auf die Produktion der galE- und galK-Genprodukte im wesentlichen in der gleichen Weise wie die natürlich vorkommende Einheit funktioniert.

6. Molekül nach Anspruch 5, wobei die Expressionseinheit eine S. coelicolor-gal-Operon-P2-Promotor-Expressionseinheit ist.

7. Molekül nach Anspruch 5 oder 6, das außerdem eine fremde funktionelle DNA-Sequenz umfaßt, die funktionell mit einer solchen Expressionseinheit verknüpft ist.

8. Rekombinantes DNA-Molekül, umfassend einen durch einen Streptomyces lividans-gal-Operon-P1-Promotor regulierten Bereich, der sich auf einem etwa 9 kb großen HindIII-SphI-Fragment der chromosomalen DNA von S. lividans 1326 mit einer NruI-Stelle befindet, wobei die P1-Transkriptionsstartstelle etwa 0,10 kb stromabwärts der NruI-Stelle liegt, oder ein beliebiges Streptomyces-Derivat davon, das in bezug auf die regulierbare Produktion der galT-, galE- und galK-Genprodukte im wesentlichen in der gleichen Weise wie der natürlich vorkommende Bereich funktioniert.

9. Molekül nach Anspruch 8, wobei der Bereich ein vom S. coelicolor-gal-Operon-P1-Promotor regulierter Bereich ist.

10. Molekül nach Anspruch 8 oder 9, das außerdem eine fremde funktionelle DNA-Sequenz umfaßt, die funktionell mit dem regulierten Bereich verknüpft ist.

11. Rekombinantes DNA-Molekül, umfassend einen Streptomyces lividans-gal-Operon-P2-Promotor, der sich auf einem etwa 9 kb großen HindIII-SphI-Fragment der chromosomalen DNA von S. lividans 1326, mit einer NruI-Stelle befindet, wobei die P2-Transkriptionsstartstelle etwa 1,25 kb stromabwärts der NruI-Stelle liegt, oder ein beliebiges Streptomyces-Derivat davon, das in bezug auf die Ermöglichung der Bindung von RNA-Polymerase daran und die Transkription einer funktionellen DNA-Sequenz, die mit dem Promotor funktionell verknüpft ist, im wesentlichen in der gleichen Weise wie der natürlich vorkommende Promotor funktioniert.

12. Molekül nach Anspruch 11, wobei der Promotor ein S. coelicolor gal-Operon-P2-Promotor ist.

13. Molekül nach Anspruch 11 oder 12, das außerdem eine fremde funktionelle DNA-Sequenz umfaßt, die funktionell mit dem P2-Promotor verknüpft ist.

14. Rekombinantes DNA-Molekül, umfassend einen Streptomyces lividans-gal-Operon-P1-Promotor, der sich auf einem etwa 9 kb großen HindIII-SphI-Fragment der chromosomalen DNA von S. lividans 1326 mit einer NruI-Stelle befindet, wobei die P1-Transkriptionsstartstelle etwa 0,10 kb stromabwärts der NruI-Stelle liegt, oder ein beliebiges Streptomyces-Derivat davon, das in bezug auf die Ermöglichung der Bindung von RNA-Polymerase daran und der Transkription einer funktionellen DNA-Sequenz, die funktionell mit dem Promotor verknüpft ist, im wesentlichen in der gleichen Weise wie der natürlich vorkommende Promotor funktioniert.

15. Molekül nach Anspruch 14, wobei der Promotor ein S. coelicolor-gal-Operon-P1-Promotor ist.

16. Molekül nach Anspruch 14 oder 15, das außerdem eine fremde funktionelle DNA-Sequenz umfaßt, die funktionell mit dem P1-Promotor verknüpft ist.

17. Transformierte(r) Wirtsorganismus oder -zelle, umfassend das Molekül nach einem der Ansprüche 4, 7, 10, 13 oder 16.

18. Verfahren zur Herstellung eines (einer) transformierten Wirtsorganismus oder -zelle, der (die) das Molekül nach einem der Ansprüche 4, 7, 10, 13 oder 16 enthält, umfassend die Transformation eines (einer) geeigneten Wirtsorganismus oder -zelle mit einem solchen Molekül.

19. Rekombinanter DNA-Vektor, umfassend das Molekül nach einem der Ansprüche 4, 7, 10, 13 oder 16 und gegebenenfalls zusätzlich ein Replikon.

20. Transformierte(r) Wirtsorganismus oder -zelle, umfassend den rekombinanten DNA-Vektor nach Anspruch 19.

21. Verfahren zur Herstellung eines (einer) transformierten Wirtsorganismus oder -zelle, der (die) den rekombinanten DNA-Vektor nach Anspruch 19 enthält, umfassend die Transformation eines (einer) geeigneten Wirtsorganismus oder -zelle mit einem solchen Vektor.

22. Verfahren zur Expression einer fremden funktionellen DNA-Sequenz, umfassend die Züchtung eines (einer) transformierten Wirtsorganismus oder -zelle, der (die) den rekombinanten DNA-Vektor nach Anspruch 19 enthält, unter geeigneten Bedingungen, so daß die funktionelle DNA-Sequenz exprimiert wird.

**23.** Verfahren zur Regulation der Expression einer fremden funktionellen DNA-Sequenz, umfassend die Züchtung eines (einer) transformierten Wirtsorganismus oder -zelle, der (die) den rekombinanten DNA-Vektor nach Anspruch 19 enthält, umfassend das Molekül nach Anspruch 4, 10 oder 16, unter geeigneten Bedingungen, so daß die Expression der Sequenz regulierbar ist.

**Patentansprüche für folgende Vertragsstaaten : AT, ES**

**1.** Verfahren zur Herstellung eines rekombinanten DNA-Moleküls, umfassend ein Streptomyces lividans-gal-Operon, das sich auf einem etwa 9 kb großen HindIII-SphI-Fragment der chromosomalen DNA von S. lividans 1326 befindet, oder ein beliebiges Streptomyces-Derivat davon, das in bezug auf die regulierbare Produktion der galT-, galE- und galK-Genprodukte im wesentlichen in der gleichen Weise funktioniert wie das natürlich vorkommende Streptomyces-gal-Operon, wobei das Verfahren die Fusion geeigneter DNA-Fragmente umfaßt.

**2.** Verfahren nach Anspruch 1, wobei das Operon ein S. coelicolor-gal-Operon ist.

**3.** Verfahren nach Anspruch 1, wobei das Operon die folgende codierende Sequenz hat:

```
         -120          -110          -100           -90           -80           -70
          •             •             •             •             •             •
    CTA CCC CTC CCC CTT CAC TAA TTC AAC ACT TTT CCT CAT CAA CTT TCT TTC ATT CTC


          -60           -50           -40           -30           -20
           •             •             •             •             •
    ATC TCA CAC CCC CCT CCT CCC TTC TCA TCT CTT ATC TTT CAT TCT CTT CCA TCA TTC
                                                                    ·galP1

    -10            1            10            20            30            40
     •             •             •             •             •             •
    ACC CCC CTC CTC CTC ACT CAT CCC TCC CTC CAC ACC ACT CCC CCA CTC AAC AAC ACC
                    Met Thr His Gly Trp Val Gln Arg Ser Ala Ala Val Lys Lys Thr
                    galT
         50            60            70            80            90            100
          •             •             •             •             •             •
    TCC ACC CCC CTC CCC CAC CCC CCT CAC CTC CTC TAC TAC CAC CTC CCC CAC CAC ACC
    Ser Thr Arg Leu Ala Asp Gly Arg Glu Leu Val Tyr Tyr Asp Leu Arg Asp Asp Thr

         110           120           130           140           150
          •             •             •             •             •
    CTC CCC CAC CCC CTC CAC CCC CCT CCC CTC CAC CCC ACC CTC ACC ACC TCC CAC CTC
    Val Arg Asp Ala Val Asp Arg Arg Pro Leu Glu Arg Thr Val Thr Thr Ser Glu Val

    160           170           180           190           200           210
     •             •             •             •             •             •
    CCA CCC CAC CCC CTC CTC CCC CAC TCC CCC CCG TCC CCC CTC CCA CCC CCA CCC CCC
    Arg Arg Asp Pro Leu Leu Gly Asp Ser Ala Pro Ser Arg Leu Ala Pro Ala Gly Ala

         220           230           240           250           260           270
          •             •             •             •             •             •
    CAC CTA CCA TCC CCC CCC CCA CCA CTC CCC CCT CTC CCc CTC CCA CCC CCA ACC CCT
    His Leu Pro Ser Ala Gly Arg Pro Val Pro Ala Val Pro Val Gly Arg Gly Thr Ala

         280           290           300           310           320           330
          •             •             •             •             •             •
    CAC CCA CAT CCC CCC TAT CAC CTC CTC CTC TTC CAC AAT CCC TTT CCC TCC CTC CCC
    Glu Arg Asp Pro Ala Tyr Asp Val Val Val Phe Glu Asn Arg Phe Pro Ser Leu Ala
```

```
          340          350          360          370          380
           •            •            •            •            •
CGT GAC TCC GGC CGC TGC GAG GTC GTC TGC TTC ACC TCC GAC CAC GAC GCC TCC TTC
Gly Asp Ser Gly Arg Cys Glu Val Val Cys Phe Thr Ser Asp His Asp Ala Ser Phe

          390          400          410          420          430          440
           •            •            •            •            •            •
GCC GAC CTG AGC GAG GAG CAG GCC CGG CTG GTC GTC GAC GCC TGG ACG GAC CGC ACC
Ala Asp Leu Ser Glu Glu Gln Ala Arg Leu Val Val Asp Ala Trp Thr Asp Arg Thr

          450          460          470          480          490          500
           •            •            •            •            •            •
TCC GAG CTG TCC CAT CTG CCC TCC GTT GAA CAG GTG TTC TGC TTC GAG AAC CGC GGC
Ser Glu Leu Ser His Leu Pro Ser Val Glu Gln Val Phe Cys Phe Glu Asn Arg Gly

          510          520          530          540          550
           ••           •            •            •            •
GCC GAG ATC GGC GTG ACG CTG GGT CAC CCG CAC GGC CAG ATC TAC GCC TAC CCG TTC
Ala Glu Ile Gly Val Thr Leu Gly His Pro His Gly Gln Ile Tyr Ala Tyr Pro Phe

   560          570          580          590          600          610
    •            •            •            •            •            •
ACC ACC CCC CGC ACC GCC CTG ATG CTC CGT TCA CTC GCC GCC CAC AAG GAC GCG ACG
Thr Thr Pro Arg Thr Ala Leu Met Leu Arg Ser Leu Ala Ala His Lys Asp Ala Thr

          620          630          640          650          660          670
           •            •            •            •            •            •
GGC GGG GGG AAC CTG TTC GAC TCC GTG CTG GAG GAC GAG CTG GCC GGT GAG CGC GTC
Gly Gly Gly Asn Leu Phe Asp Ser Val Leu Glu Glu Glu Leu Ala Gly Glu Arg Val

          680          690          700          710          720
           •            •            •            •            •
GTC CTG GAG GGT GAG CAC TGG GCC GCC TTC GTC GCG TAC GGC GCC CAC TGG CCG TAC
Val Leu Glu Gly Glu His Trp Ala Ala Phe Val Ala Tyr Gly Ala His Trp Pro Tyr

730          740          750          760          770          780
 •            •            •            •            •            •
GAG GTG CAC CTC TAC CCG AAG CGC CGG GTG CCC GAT CTG CTC GGG CTC GAC GAG GCG
Glu Val His Leu Tyr Pro Lys Arg Arg Val Pro Asp Leu Leu Gly Leu Asp Glu Ala

   790          800          810          820          830          840
    •            •            •            •            •            •
GCT CGC ACA GAA TTC CCC AAG GTC TAC CTG GAG CTG CTG AGG CGT TTC GAC CGC ATC
Ala Arg Thr Glu Phe Pro Lys Val Tyr Leu Glu Leu Leu Arg Arg Phe Asp Arg Ile
```

```
        850         860          870          880          890          900
         .           .            .            .            .            .
TTC CGC GAG GGC GAG CCC CCC ACC CCC TAC ATC GCG GCC TGG CAC CAG GCC CCC TTC
Phe Gly Glu Gly Glu Pro Pro Thr Pro Tyr Ile Ala Ala Trp His Gln Ala Pro Phe

           9            920          930          940          950
           .            .            .            .            .
GGC CAG CTC GAG TTC GAG GGT GTC ACC CGC GAC GAC TTC GCG CTC CAC CTC GAA CTT
Gly Gln Leu Glu Phe Glu Gly Val Thr Arg Asp Asp Phe Ala Leu His Leu Glu Leu

 960          970          980          990          1000         1010
  .            .            .            .            .            .
TTC ACT TCC GCC GTA CGT CCG GCA AGC TGA AGT TCC TCG CGC GCT CCG AAT CCG GCA
Phe Thr Ser Ala Val Arg Pro Ala Ser ---              ‾galP2

    1020        1030         1040         1050         1060        1070
     .           .            .            .            .           .
  TGAACG TGTTCATCAA CGACGTACCC CCGGAGCGCC CGGCCGAGCG ACTGCGAGAG GTAGCGAG


  1080         1090          1100         1110         1120         1130
   .            .             .            .            .            .
TTC ATG AGC GGC AAC TAC CTG GTG ACA GGT GGT GCC GGA TAC GTC GGC AGC GTC GTC
    Met Ser Gly Lys Tyr Leu Val Thr Gly Gly Ala Gly Tyr Val Gly Ser Val Val
    galE
  1140         1150          1160         1170         1180         1190
   .            .             .            .            .            .
GCC CAG CAC TTG GTG GAG GCG GGG AAC GAG GTC GTG GTG CTG CAC AAT CTG TCG ACC
Ala Gln His Leu Val Glu Ala Gly Asn Glu Val Val Val Leu His Asn Leu Ser Thr

        1200         1210         1220         1230         1240
         .            .            .            .            .
GGC TTC CGT GAG GTG TGC CGG CGG GTG CCT CGT TCG TCG ACG CGA CAT CCG GGA CGC
Gly Phe Arg Glu Val Cys Arg Arg Val Pro Arg Ser Ser Arg Arg His Pro Gly Arg

 1250        1260         1270         1280         1290         1300
  .           .            .            .            .            .
CGC CAA GTG CGT GGA CGG CTC TCG TTC GAC GGC GTG CTG CAC TTC GCC GCC TTC TCC
Arg Gln Val Arg Gly Arg Leu Ser Phe Asp Gly Val Leu His Phe Ala Ala Phe Ser

    1310        1320         1330         1340         1350         1360
     .           .            .            .            .            .
CAG CTC GGC GAG TCC GTC GTG AAG CCC GAG AAG TAC TGG GAC AAC AAC GTC GGT GGC
Gln Val Gly Glu Ser Val Val Lys Pro Glu Lys Tyr Trp Asp Asn Asn Val Gly Gly
```

```
            1370            1380            1390            1400            1410            1420
             •               •               •               •               •               •
ACC ATC GCG CTC CTC GAC GCC ATC CGG GGC GCC GGT GTG CGC CGC CTC GTC TTC TCC
Thr Met Ala Leu Leu Glu Ala Met Arg Gly Ala Gly Val Arg Arg Leu Val Phe Ser

                    1430            1440            1450            1460            1470
                     •               •               •               •               •
TCC ACG GCC GCC ACG TAC GGC GAG CCC GAG CAG GTT CCC ATC GTC GAG TCC GCG CCC
Ser Thr Ala Ala Thr Tyr Gly Glu Pro Glu Gln Val Pro Ile Val Glu Ser Ala Pro

1480            1490            1500            1510            1520            1530
 •               •               •               •               •               •
ACG AGG CCC ACC AAT CCG TAC GGC GCC TCG AAG CTC GCC GTC GAC CAC ATC ATC ACC
Thr Arg Pro Thr Asn Pro Tyr Gly Ala Ser Lys Leu Ala Val Asp His Met Ile Thr

        1540            1550            1560            1570            1580            1590
         •               •               •               •               •               •
GGC GAG GCG CCG GCC CAC GGG CTG GGC GCC GTC TCC GTG CCG TAC TTC AAC GTC GCG
Gly Glu Ala Ala Ala His Gly Leu Gly Ala Val Ser Val Pro Tyr Phe Asn Val Ala

            1600            1610            1620            1630            1640
             •               •               •               •               •
GGC GCC TAC GGG GAG TAC GGC GAG CGC CAC GAC CCC GAG TCG CAT CTG ATT CCG CTG
Gly Ala Tyr Gly Glu Tyr Gly Glu Arg His Asp Pro Glu Ser His Leu Ile Pro Leu

1650            1660            1670            1680            1690            1700
 •               •               •               •               •               •
GTC CTT CAA GTG GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

        1710            1720            1730            1740            1750            1760
         •               •               •               •               •               •
CGG ACC CCG GAC CGA CCT GTG TGC GCG ACT ACA TCC ACG TCG CCG ACC TGG CCG AGG
Pro Thr Pro Asp Arg Pro Val Cys Ala Thr Thr Ser Thr Ser Pro Thr Trp Pro Arg

            1770            1780            1790            1800            1810
             •               •               •               •               •
CCC ACC TGC TGG CCG TGC GCC GCC GCC CCG GGC GAG CAC CTC ATC TGC AAC CTC GGC
Pro Thr Cys Trp Pro Cys Ala Ala Ala Pro Gly Glu His Leu Ile Cys Asn Leu Gly

1820            1830            1840            1850            1860            1870
 •               •               •               •               •               •
AAC GGC AAC GGC TTC TCC GTC CGC GAG GTC GTC GAG ACC GTG CGG CGC GTG ACG GGC
Asn Gly Asn Gly Phe Ser Val Arg Glu Val Val Glu Thr Val Arg Arg Val Thr Gly
```

```
        1880          1890          1900          1910          1920          1930
         •            •            •            •            •            •
CAT CCC ATC CCC GAG ATC ATC GCC CCC CGC CGC GGC CGC GAC CCG GCC GTC CTC CTC
His Pro Ile Pro Glu Ile Met Ala Pro Arg Arg Gly Arg Asp Pro Ala Val Leu Val

        1940          1950          1960          1970          1980          1990
         •            •            •            •            •            •
GCG TCG GCC GGC ACC GCC CGC GAG AAC CTC GGC TGG AAC CCC TCC CGC GCG GAC CTC
Ala Ser Ala Gly Thr Ala Arg Glu Lys Leu Gly Trp Asn Pro Ser Arg Ala Asp Leu

           2000          2010          2020          2030          2040
            •            •            •            •            •
CCC ATC GTC TCG GAC GCG TGG GAG TTC CCG CAG CGC CGC GCG GGC CAG TAG  TA
Ala Ile Val Ser Asp Ala Trp Glu Leu Pro Gln Arg Arg Ala Gly Gln ---


        2050          2060          2070          2080          2090          2100
         •            •            •            •            •            •
ACC GCA GTT ACC GGA AAG GCG ACG GGT CAG GGC ATC GGC GAG GCT GTC GGG GAA CCC
                                          Met Gly Glu Ala Val Gly Glu Pro
                                          galK
        2110          2120          2130          2140          2150
         •            •            •            •            •
TCG GCG AGC GGT TCC GGG AGC TGT ACG GGG CGG ACC CGG AGC GGG TGT GGG CGC CGA
Ser Ala Ser Gly Ser Gly Ser Cys Thr Gly Arg Ser Arg Arg Gly Cys Gly Arg Arg

  2160          2170          2180          2190          2200          2210
   •            •            •            •            •            •
GCG GGC CGG GAG AAC CTC ATC GGG GAG CAC ACC GAC TAC AAC GAC GGC TTC GTC ATG
Ala Gly Arg Glu Asn Leu Ile Gly Glu His Thr Asp Tyr Asn Asp Gly Phe Val Met

   2220          2230          2240          2250          2260          2270
    •            •            •            •            •            •
CCT TCG CCC TGC CGC ACC AGG TCG CGG CCG TCT CCC GGC GCC AAC GAC GGC ATC CTG
Pro Ser Pro Cys Arg Thr Arg Ser Arg Pro Ser Pro Gly Ala Asn Asp Gly Ile Leu

        2280          2290          2300          2310          2320
         •            •            •            •            •
CGC CTC CAC TCG GCC GAC GTC GAC GCC GAC CCG GTC GAG CTC CGC GTC GCC GAC CTG
Arg Leu His Ser Ala Asp Val Asp Ala Asp Pro Val Glu Leu Arg Val Ala Asp Leu

2330          2340          2350          2360          2370          2380
 •            •            •            •            •            •
GCC GCC GCG TCG GAC AAG TCC TGG ACG GCG TAC CCC TCG GGC GTC CTG TGG GCG CTG
Ala Pro Ala Ser Asp Lys Ser Trp Thr Ala Tyr Pro Ser Gly Val Leu Trp Ala Leu
```

EP 0 235 112 B1

```
        2390           2400          2410          2420          2430          2440
         •             •             •             •             ·.            •
CGC GAG GCC GGA CAC GAG CTG ACC GGC GCC GAC CTC CAC CTG GCC TCC ACC GTC CCC
Arg Glu Ala Gly His Glu Leu Thr Gly Ala Asp Val His Leu Ala Ser Thr Val Pro

            2450          2460          2470          2480          2490
             •             •             •             •             •
TCC GGC GCC GGG CTC TCC TCC TCC GCC GCC CTG GAG GTC CGT CCC CTG GCG ATG AAC
Ser Gly Ala Gly Leu Ser Ser Ser Ala Ala Leu Glu Val Arg Pro Leu Ala Met Asn

 2500          2510          2520          2530          2540          2550
  •             •             •             •             •             •
GAC CTG TAC GCC CTC GCC CTG CGC GGC TGG CAG CTG GCC CGG CTG TGC CAG CGC GCG
Asp Leu Tyr Ala Leu Ala Leu Arg Gly Trp Gln Leu Ala Arg Leu Cys Gln Arg Ala

    2560          2570          2580          2590          2600          2610
     •             •             •             •             •             •
GAG AAC CTC TAC GTC GGC GCC CCC GTC GGC ATC ATG GAC CAG ACG GCG TCC GCC TGC
Glu Asn Val Tyr Val Gly Ala Pro Val Gly Ile Met Asp Gln Thr Ala Ser Ala Cys

        2620          2630          2640          2650          2660          2670
         •             •             •             •             •             •
TGC GAG GCG GGC ACC CCC TCT TCC TCG ACA CCC GCG ACC TCT CCC ACC GGC AGA TCC
Cys Glu Ala Gly Thr Pro Ser Ser Ser Thr Pro Ala Thr Ser Pro Ser Gly Arg Ser

            2680          2690          2700          2710          2720
             •             •             •             •             •
CCT TCG ACC TCG CCG CCC AGG GGA TGC GCC TGC TGG TCG TCG ACA CCC GGC TCA ACC
Pro Ser Thr Ser Pro Pro Arg Gly Cys Ala Cys Trp Ser Ser Thr Pro Gly Ser Ser

 2730          2740          2750          2760          2770          2780
  •             •             •             •             •             •
ACT CCC ACA GCC AGG GCG AGT ACG GCA AGC GCC GCG CGG GCT GCG AGA AGG GCG CCC
Thr Pro Thr Ala Arg Ala Ser Thr Ala Ser Ala Ala Arg Ala Ala Arg Arg Ala Pro

        2790          2800          2810          2820          2830          2840
         •             •             •             •             •             •
CGC TGC TGG GCG TCC ACC CGC TGC CAC GTC CCC TAC GCC GAC CTC GAC GCC GCC CTC
Arg Cys Trp Ala Ser Thr Arg Cys Asp Val Pro Tyr Ala Asp Leu Asp Ala Ala Leu

            2850          2860          2870          2880          2890
             •             •             •             •             •
GAG CGG CTG GGC GAC GAG GAG GAC GTC CGC CGC CTG GTC CGG CAC GTG GTG ACC GAG
Glu Arg Leu Gly Asp Glu Glu Glu Val Arg Arg Leu Val Arg His Val Val Thr Glu
```

56

```
     2900          2910          2920          2930          2940          2950
      •             •             •             •             •             •
 CAC CAC CGC GTC CAA CGC CTC CTC CCC CTC CTC CAC TCC CCC ACA CCC CGC GCA TCC
 Asp Glu Arg Val Glu Arg Val Val Ala Leu Leu Glu Ser Ala Thr Pro Gly Ala Ser


           2960          2970          2980          2990          3000          3010
            •             •             •             •             •             •
 GCC CCC TCC TGG TCC AGG CCC ACC CCT GCT GCC CGA CGA CTT CCC CAT CTC CTC CCC
 Ala Pro Ser Trp Ser Arg Ala Thr Pro Ala Ala Arg Arg Leu Pro His Leu Leu Pro


           3020          3030          3040          3050          3060
            •             •             •             •             •
 CGA CCT GGA CCT GGT CGT CCA CAC GGC CCT GGC CTC CGC GGC CCT CGG CGC CGG ATC
 Arg Ala Gly Pro Gly Arg Arg His Gly Pro Gly Leu Arg Gly Pro Arg Arg Arg Met


 3070          3080          3090          3100          3110          3120
  •             •             •             •             •             •
 ACC GGC GGC GGC TTC GGC GGC TCG GCG ATC GTC CTC GTG GAC GCC GCC GCG GTG CAC
 Thr Gly Gly Gly Phe Gly Gly Ser Ala Ile Val Leu Val Glu Ala Ala Ala Val Asp


           3130          3140          3150          3160          3170          3180
            •             •             •             •             •             •
 GCC GTC ACC AAG GCG GTC GAG GAC GCC TTC GCC GCG GCG GGC CTC AAG CGT CCG CGG
 Ala Val Thr Lys Ala Val Glu Asp Ala Phe Ala Ala Ala Gly Leu Lys Arg Pro Arg


           3190          3200          3210          3220          3230          3240
            •             •             •             •             •             •
 GTC TTC GAG GCG GTG CCT CGG CGG GGC GCG GCG CCT GGT CTC ACG GTC AGC CGA GCC
 Val Phe Glu Ala Val Pro Arg Arg Gly Ala Ala Pro Gly Leu Thr Val Ser Arg Ala


           3250          3260          3270          3280          3290
            •             •             •             •             •
 GCT TCA CCA GCG TGT ACT CCC TGA TCC CCG GCG GGT AGT CGG GGA TCA CGC ACA TGA
 Ala Ser Pro Ala Cys Thr Pro ---


 3300
  •
 GCT GGT ACC CGC
```

umfassend die Transformation eines (einer) geeigneten Wirtsorganismus oder -zelle mit einem solchen Molekül.

4. Verfahren nach einem der Ansprüche 1 bis 3, wobei das Molekül außerdem eine fremde funktionelle DNA-Sequenz umfaßt, die funktionell mit einem solchen Operon verknüpft ist.

5. Verfahren zur Herstellung eines rekombinanten DNA-Moleküls, umfassend eine Streptomyces lividans-gal-Operon-P2-Promotor-Expressionseinheit, die sich auf einem etwa 9 kb großen HindIII-SphI-Fragment der chromosomalen DNA von S. lividans 1326 mit einer NruI-Stelle befindet, wobei die P2-Transkriptionsstartstelle etwa 1,25 kb stromabwärts der NruI-Stelle liegt, oder ein beliebiges Derivat davon, das in bezug auf die Produktion der galE-und galK-Genprodukte im wesentlichen in der gleichen Weise wie die natürlich vorkommende Einheit funktioniert, wobei das Verfahren die Fusion geeigneter DNA-Fragmente umfaßt.

6. Verfahren nach Anspruch 5, wobei die Expressionseinheit eine S. coelicolor-gal-Operon-P2-Promotor-Expressionseinheit ist.

7. Verfahren nach Anspruch 5 oder 6, wobei das Molekül außerdem eine fremde funktionelle DNA-Sequenz umfaßt, die funktionell mit einer solchen Expressionseinheit verknüpft ist.

**8.** Verfahren zur Herstellung eines rekombinanten DNA-Moleküls, umfassend einen durch einen Streptomyces lividans-gal-Operon-P1-Promotor regulierten Bereich, der sich auf einem etwa 9 kb großen HindIII-SphI-Fragment der chromosomalen DNA von S. lividans 1326 mit einer NruI-Stelle befindet, wobei die P1-Transkriptionsstartstelle etwa 0,10 kb stromabwärts der NruI-Stelle liegt, oder ein beliebiges Streptomyces-Derivat davon, das in bezug auf die regulierbare Produktion der galT-, galE- und galK-Genprodukte im wesentlichen in der gleichen Weise wie der natürlich vorkommende Bereich funktioniert, wobei das Verfahren die Fusion geeigneter DNA-Fragmente umfaßt.

**9.** Verfahren nach Anspruch 8, wobei der Bereich ein vom S. coelicolor-gal-Operon-P1-Promotor regulierter Bereich ist.

**10.** Verfahren nach Anspruch 8 oder 9, wobei das Molekül außerdem eine fremde funktionelle DNA-Sequenz umfaßt, die funktionell mit dem regulierten Bereich verknüpft ist.

**11.** Verfahren zur Herstellung eines rekombinanten DNA-Moleküls, umfassend einen Streptomyces lividans-gal-Operon-P2-Promotor, der sich auf einem etwa 9 kb großen HindIII-SphI-Fragment der chromosomalen DNA von S. lividans 1326, mit einer NruI-Stelle befindet, wobei die P2-Transkriptionsstartstelle etwa 1,25 kb stromabwärts der NruI-Stelle liegt, oder ein beliebiges Streptomyces-Derivat davon, das in bezug auf die Ermöglichung der Bindung von RNA-Polymerase daran und die Transkription einer funktionellen DNA-Sequenz, die mit dem Promotor funktionell verknüpft ist, im wesentlichen in der gleichen Weise wie der natürlich vorkommende Promotor funktioniert, wobei das Verfahren die Fusion geeigneter DNA-Fragmente umfaßt.

**12.** Verfahren nach Anspruch 11, wobei der Promotor ein S. coelicolor gal-Operon-P2-Promotor ist.

**13.** Verfahren nach Anspruch 11 oder 12, wobei das Molekül außerdem eine fremde funktionelle DNA-Sequenz umfaßt, die funktionell mit dem P2-Promotor verknüpft ist.

**14.** Verfahren zur Herstellung eines rekombinanten DNA-Moleküls, umfassend einen Streptomyces lividans-gal-Operon-P1-Promotor, der sich auf einem etwa 9 kb großen HindIII-SphI-Fragment der chromosomalen DNA von S. lividans 1326 mit einer NruI-Stelle befindet, wobei die P1-Transkriptionsstartstelle etwa 0,10 kb stromabwärts der NruI-Stelle liegt, oder ein beliebiges Streptomyces-Derivat davon, das in bezug auf die Ermöglichung der Bindung von RNA-Polymerase daran und der Transkription einer funktionellen DNA-Sequenz, die funktionell mit dem Promotor verknüpft ist, im wesentlichen in der gleichen Weise wie der natürlich vorkommende Promotor funktioniert, wobei das Verfahren die Fusion geeigneter DNA-Fragmente umfaßt.

**15.** Verfahren nach Anspruch 14, wobei der Promotor ein S. coelicolor-gal-Operon-P1-Promotor ist.

**16.** Verfahren nach Anspruch 14 oder 15, wobei das Molekül außerdem eine fremde funktionelle DNA-Sequenz umfaßt, die funktionell mit dem P1-Promotor verknüpft ist.

**17.** Verfahren zur Herstellung eines (einer) transformierte(n) Wirtsorganismus oder -zelle, umfassend das Molekül nach einem der Ansprüche 4, 7, 10, 13 oder 16, das die Transformation eines (einer) geeigneten Wirtsorganismus oder -zelle mit einem solchen Molekül umfaßt.

**18.** Verfahren zur Herstellung eines rekombinanten DNA-Vektors, umfassend das Molekül nach einem der Ansprüche 4, 7, 10, 13 oder 16 und gegebenenfalls zusätzlich ein Replikon, wobei das Verfahren die Fusion geeigneter DNA-Fragmente umfaßt.

**19.** Verfahren zur Herstellung eines (einer) transformierten Wirtsorganismus oder -zelle, der (die) den rekombinanten DNA-Vektor nach Anspruch 18 enthält, umfassend die Transformation eines geeigneten Wirtsorganismus mit einem solchen Vektor.

**20.** Verfahren zur Expression einer fremden funktionellen DNA-Sequenz, umfassend die Züchtung eines (einer) transformierten Wirtsorganismus oder -zelle, der (die) den rekombinanten DNA-Vektor nach Anspruch 18 enthält, unter geeigneten Bedingungen, so daß die funktionelle DNA-Sequenz exprimiert wird.

**21.** Verfahren zur Regulation der Expression einer fremden funktionellen DNA-Sequenz, umfassend die Züchtung eines (einer) transformierten Wirtsorganismus oder -zelle, der (die) den rekombinanten DNA-Vektor nach Anspruch 18 enthält, umfassend das Molekül nach Anspruch 4, 10 oder 16, unter geeigneten Bedingungen, so daß die Expression der Sequenz regulierbar ist.

**Revendications**
**Revendications pour les Etats contractants suivants : BE, CH, DE, FR, GB, IT, LI, LU, NL, SE, GR**

**1.** Molécule d'ADN recombinant comprenant un opéron gal de Streptomyces lividans situé sur un fragment HindIII-SphI d'environ 9kb de l'ADN chromosomique de S. lividans 1326, ou l'un quelconque de ses dérivés de Streptomyces qui agit pratiquement de la même manière que l'opéron gal de Streptomyces naturel en termes de production régulable des produits des gènes galT, galE et galK.

**2.** Molécule suivant la revendication 1, dans laquelle l'opéron est un opéron gal de S. coelicolor.

**3.** Molécule suivant la revendication 1, qui comprend la séquence codante suivante :

```
        -120         -110         -100          -90          -80          -70
          •            •            •            •            •            •
   CTA CCC CTC CCC CTT CAC TAA TTC AAC ACT TTT CCT CAT CAA CTT TCT TTC ATT CTC


           -60          -50          -40          -30          -20
            •            •            •            •            •
   ATC TCA CAC CCC CCT CCT CCC TTC TCA TCT CTT ATC TTT CAT TCT CTT CCA TCA TTC
                                                              ˙galP1


    -10           1           10           20           30           40
     •            •            •            •            •            •
   ACC CCC CTC CTC CTC ACT CAT CCC TCC CTC CAC ACC ACT CCC CCA CTC AAC AAC ACC
                   Met Thr His Gly Trp Val Gln Arg Ser Ala Ala Val Lys Lys Thr
                   galT
          50           60           70           80           90          100
           •            •            •            •            •            •
   TCC ACC CCC CTC CCC CAC CCC CCT CAC CTC CTC TAC TAC CAC CTC CCC CAC CAC ACC
   Ser Thr Arg Leu Ala Asp Gly Arg Glu Leu Val Tyr Tyr Asp Leu Arg Asp Asp Thr


          110          120          130          140          150
           •            •            •            •            •
   CTC CCC CAC CCC CTC CAC CCC CCT CCC CTC CAC CCC ACC CTC ACC ACC TCC CAC CTC
   Val Arg Asp Ala Val Asp Arg Arg Pro Leu Glu Arg Thr Val Thr Thr Ser Glu Val

   160          170          180          190          200          210
    •            •            •            •            •            •
   CCA CCC CAC CCC CTC CTC CCC CAC TCC CCC CCC TCC CCC CTC CCA CCC CCA CCC CCC
   Arg Arg Asp Pro Leu Leu Gly Asp Ser Ala Pro Ser Arg Leu Ala Pro Ala Gly Ala

           220          230          240          250          260          270
            •            •            •            •            •            •
   CAC CTA CCA TCC CCC CCC CCA CCA CTC CCC CCT CTC CCc CTC CCA CCC CCA ACC CCT
   His Leu Pro Ser Ala Gly Arg Pro Val Pro Ala Val Pro Val Gly Arg Gly Thr Ala

           280          290          300          310          320          330
            •            •            •            •            •            •
   CAC CCA CAT CCC CCC TAT CAC CTC CTC CTC TTC CAC AAT CCC TTT CCC TCC CTC CCC
   Clu Arg Asp Pro Ala Tyr Asp Val Val Val Phe Glu Asn Arg Phe Pro Ser Leu Ala
```

```
            340          350          360          370          380
             •            •            •            •            •
GCT GAC TCC GGG CGC TGC GAG GTC CTC TGC TTC ACC TCC GAC CAC GAC GCC TCC TTC
Gly Asp Ser Gly Arg Cys Glu Val Val Cys Phe Thr Ser Asp His Asp Ala Ser Phe

     390          400          410          420          430          440
      •            •            •            •            •            •
GCC GAC CTG ACC GAG GAG CAG GCC CGG CTG GTC GTC GAC GCC TGG ACG GAC CGC ACC
Ala Asp Leu Ser Glu Glu Gln Ala Arg Leu Val Val Asp Ala Trp Thr Asp Arg Thr

            450          460          470          480          490          500
             •            •            •            •            •            •
TCC GAG CTG TCC CAT CTC CCC TCC GTT GAA CAG GTG TTC TGC TTC GAG AAC CGC GGC
Ser Glu Leu Ser His Leu Pro Ser Val Glu Gln Val Phe Cys Phe Glu Asn Arg Gly

                 510          520          530          540          550
                  • •          •            •            •            •
GCC GAG ATC GGG GTC ACG CTG GGT CAC CCG CAC GGG CAG ATC TAC GCC TAC CCG TTC
Ala Glu Ile Gly Val Thr Leu Gly His Pro His Gly Gln Ile Tyr Ala Tyr Pro Phe

     560          570          580          590          600          610
      •            •            •            •            •            •
ACC ACC CCC CGC ACC GCC CTG ATG CTC CGT TCA CTC GCC GCC CAC AAG GAC GCG ACG
Thr Thr Pro Arg Thr Ala Leu Met Leu Arg Ser Leu Ala Ala His Lys Asp Ala Thr

       620          630          640          650          660          670
        •            •            •            •            •            •
GGC GGG GGG AAC CTC TTC GAC TCC GTG CTG GAG GAG GAG CTG GCC GGT GAG CGC GTC
Gly Gly Gly Asn Leu Phe Asp Ser Val Leu Glu Glu Glu Leu Ala Gly Glu Arg Val

            680          690          700          710          720
             •            •            •            •            •
GTC CTG GAG GGT GAG CAC TGG GCC GCC TTC GTC GCG TAC GGC GCG CAC TGG CCG TAC
Val Leu Glu Gly Glu His Trp Ala Ala Phe Val Ala Tyr Gly Ala His Trp Pro Tyr

   730          740          750          760          770          780
    •            •            •            •            •            •
GAG GTC CAC CTC TAC CCG AAG CGG CGG GTC CCC GAT CTG CTC GGG CTC GAC GAG GCG
Glu Val His Leu Tyr Pro Lys Arg Arg Val Pro Asp Leu Leu Gly Leu Asp Glu Ala

       790          800          810          820          830          840
        •            •            •            •            •            •
GCT CGC ACA GAA TTC CCC AAG GTC TAC CTC GAG CTC CTG AGG CGT TTC GAC CGC ATC
Ala Arg Thr Glu Phe Pro Lys Val Tyr Leu Glu Leu Leu Arg Arg Phe Asp Arg Ile
```

60

```
        850           860           870           880           890           900
         •             •             •             •             •             •
TTC GGC GAG GGC GAG CCC CCG ACC CCC TAC ATC GCC GCC TGG CAC CAG GCC CCG TTC
Phe Gly Glu Gly Glu Pro Pro Thr Pro Tyr Ile Ala Ala Trp His Gln Ala Pro Phe

              9           920           930           940           950
              •             •             •             •             •
GGC CAG CTG GAG TTC GAG GGT GTG ACG CGC GAC GAC TTC GCG CTC GAC CTG GAA CTT
Gly Gln Leu Glu Phe Glu Gly Val Thr Arg Asp Asp Phe Ala Leu His Leu Glu Leu

   960           970           980           990           1000          1010
    •             •             •             •             •             •
TTC ACT TCC GCC CTA CGT CCG GCA AGC TGA AGT TCC TCG GCG GCT CCG AAT GCG GCA
Phe Thr Ser Ala Val Arg Pro Ala Ser ---              ⁻galP2

      1020          1030          1040          1050          1060          1070
       •             •             •             •             •             •
     TGAACG TGTTCATCAA CGACGTACCC CGGGAGCGCG CGCCCGAGCG ACTGCGAGAG GTAGCGAC


 1080          1090          1100          1110          1120          1130
  •             •             •             •             •             •
TTC ATG AGC GGG AAG TAC CTG GTG ACA GGT GGT GCC GGA TAC GTC GGC AGC GTC GTC
   Met Ser Gly Lys Tyr Leu Val Thr Gly Gly Ala Gly Tyr Val Gly Ser Val Val
   galE
   1140          1150          1160          1170          1180          1190
    •             •             •             •             •             •
GCC CAG CAC TTG GTG GAG GCG GGG AAC GAG GTC GTG GTG CTG CAC AAT CTG TCG ACC
Ala Gln His Leu Val Glu Ala Gly Asn Glu Val Val Val Leu His Asn Leu Ser Thr

      1200          1210          1220          1230          1240
       •             •             •             •             •
GGC TTC CGT GAG GTG TGC CGG CGC GTC CCT CCT TCG TCC AGG CGA CAT CCG GGA CGC
Gly Phe Arg Glu Val Cys Arg Arg Val Pro Arg Ser Ser Arg Arg His Pro Gly Arg

 1250          1260          1270          1280          1290          1300
  •             •             •             •             •             •
CGC CAA GTG CGT GGA CGG CTC TCG TTC GAC GGC GTG CTC CAC TTC GCC GCC TTC TCC
Arg Gln Val Arg Gly Arg Leu Ser Phe Asp Gly Val Leu His Phe Ala Ala Phe Ser

   1310          1320          1330          1340          1350          1360
    •             •             •             •             •             •
CAG GTC GGC GAG TCG GTC GTG AAG CCC GAG AAG TAC TGG GAC AAC AAC GTC GGT GGC
Gln Val Gly Glu Ser Val Val Lys Pro Glu Lys Tyr Trp Asp Asn Asn Val Gly Gly
```

```
              1370        1380        1390        1400        1410        1420
               •           •           •           •           •           •
ACC ATG GCG CTG CTC GAG GCC ATG CGG GGC GCG GGT GTC CGG CGG CTC GTC TTC TCC
Thr Met Ala Leu Leu Glu Ala Met Arg Gly Ala Gly Val Arg Arg Leu Val Phe Ser

                   1430        1440        1450        1460        1470
                    •           •           •           •           •
TCC ACG CCC GCC ACG TAC GGC GAG CCC GAG CAG GTT CCC ATC GTC GAG TCC GCC CCG
Ser Thr Ala Ala Thr Tyr Gly Glu Pro Glu Gln Val Pro Ile Val Glu Ser Ala Pro

        1480        1490        1500        1510        1520        1530
         •           •           •           •           •           •
ACG AGG CCC ACC AAT CCG TAC GGC GCC TCC AAG CTC GCC GTC GAC CAC ATG ATC ACC
Thr Arg Pro Thr Asn Pro Tyr Gly Ala Ser Lys Leu Ala Val Asp His Met Ile Thr

            1540        1550        1560        1570        1580        1590
             •           •           •           •           •           •
GGC GAG GCG GCC GCC CAC GGG CTC GGC GCG GTC TCC GTC CCG TAC TTC AAC GTC GCG
Gly Glu Ala Ala Ala His Gly Leu Gly Ala Val Ser Val Pro Tyr Phe Asn Val Ala

                1600        1610        1620        1630        1640
                 •           •           •           •           •
GGC GCG TAC GGC GAG TAC GGC GAG CGC CAC GAC CCC GAG TCG CAT CTG ATT CCC CTG
Gly Ala Tyr Gly Glu Tyr Gly Glu Arg His Asp Pro Glu Ser His Leu Ile Pro Leu

    1650        1660        1670        1680        1690        1700
     •           •           •           •           •           •
GTC CTT CAA GTC GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

        1710        1720        1730        1740        1750        1760
         •           •           •           •           •           •
CCG ACC CCG GAC CGA CCT GTC TGC GCG ACT ACA TCC ACG TCG CCG ACC TCG CCG AGG
Pro Thr Pro Asp Arg Pro Val Cys Ala Thr Thr Ser Thr Ser Pro Thr Trp Pro Arg

            1770        1780        1790        1800        1810
             •           •           •           •           •
CCC ACC TGC TGG CCG TCC GCC GCC GCC CCG GGC GAG CAC CTC ATC TGC AAC CTG GGC
Pro Thr Cys Trp Pro Cys Ala Ala Ala Pro Gly Glu His Leu Ile Cys Asn Leu Gly

1820        1830        1840        1850        1860        1870
 •           •           •           •           •           •
AAC GGC AAC GGC TTC TCC GTC CGC GAG GTC GTC GAG ACC GTG CGG CGG GTG ACG GGC
Asn Gly Asn Gly Phe Ser Val Arg Glu Val Val Glu Thr Val Arg Arg Val Thr Gly
```

```
        1880        1890        1900        1910        1920        1930
         •           •           •           •           •           •
CAT CCC ATC CCC GAG ATC ATG GCC CCG CGC CGC GGC CGC GAC CCG GCG CTC CTC CTC
His Pro Ile Pro Glu Ile Met Ala Pro Arg Arg Gly Arg Asp Pro Ala Val Leu Val

            1940        1950        1960        1970        1980        1990
             •           •           •           •           •           •
GCG TCG GCC GGC ACC GCC CGC GAG AAG CTG GGC TGG AAC CCG TCC CGC GCG GAC CTC
Ala Ser Ala Gly Thr Ala Arg Glu Lys Leu Gly Trp Asn Pro Ser Arg Ala Asp Leu

            2000        2010        2020        2030        2040
             •           •           •           •           •
GCC ATC GTC TCG GAC GCG TGG GAC TTC CCG CAG CGC CGC GCC CGC CAC TAG  TA
Ala Ile Val Ser Asp Ala Trp Glu Leu Pro Gln Arg Arg Ala Gly Gln ---

            2050        2060        2070        2080        2090        2100
             •           •           •           •           •           •
ACC GCA GTT ACC GGA AAG GCG AGG GGT CAG GGC ATG GGC GAG GCT GTC GGG GAA CCG
                                            Met Gly Glu Ala Val Gly Glu Pro
                                            galK
            2110        2120        2130        2140        2150
             •           •           •           •           •
TCG GCC AGC GGT TCC GGG AGC TCT ACG GGG CGG AGC CGG ACG GGG TGT GGG CGC CGA
Ser Ala Ser Gly Ser Gly Ser Cys Thr Gly Arg Ser Arg Arg Gly Cys Gly Arg Arg

2160        2170        2180        2190        2200        2210
 •           •           •           •           •           •
GCG GGC CGG GAG AAC CTC ATC GGG GAG CAC ACC GAC TAC AAC GAC GGC TTC GTC ATC
Ala Gly Arg Glu Asn Leu Ile Gly Glu His Thr Asp Tyr Asn Asp Gly Phe Val Met

    2220        2230        2240        2250        2260        2270
     •           •           •           •           •           •
CCT TCG CCC TGC CGC ACC ACG TCG CGC CCG TCT CCC GGC GCG AAC GAC GGC ATC CTG
Pro Ser Pro Cys Arg Thr Arg Ser Arg Pro Ser Pro Gly Ala Asn Asp Gly Ile Leu

            2280        2290        2300        2310        2320
             •           •           •           •           •
CGC CTG CAC TCG GCC GAC GTC GAC GCC GAC CCC GTC GAG CTC CGC GTC GCC GAC CTC
Arg Leu His Ser Ala Asp Val Asp Ala Asp Pro Val Glu Leu Arg Val Ala Asp Leu

2330        2340        2350        2360        2370        2380
 •           •           •           •           •           •
GCC CCC GCC TCG GAC AAG TCC TGG ACG GCC TAC CCC TCG GGC GTC CTG TGG GCC CTG
Ala Pro Ala Ser Asp Lys Ser Trp Thr Ala Tyr Pro Ser Gly Val Leu Trp Ala Leu
```

```
        2390          2400          2410          2420          2430          2440
          •             •             •             •             •             •
CGC GAG GCC GGA CAC GAG CTG ACC GGC GCC GAC GTC CAC CTG GCC TCG ACC GTC CCG
Arg Glu Ala Gly His Glu Leu Thr Gly Ala Asp Val His Leu Ala Ser Thr Val Pro

              2450          2460          2470          2480          2490
                •             •             •             •             •
TCC GGG GCG GGG CTC TCC TCC TCC GCG GCC CTG GAG GTC CGT CCC CTG GCG ATG AAC
Ser Gly Ala Gly Leu Ser Ser Ser Ala Ala Leu Glu Val Arg Pro Leu Ala Met Asn

  2500          2510          2520          2530          2540          2550
    •             •             •             •             •             •
GAC CTC TAC GCC CTC GCG CTG CGC GGC TGG CAG CTG GCC CGC CTG TGC CAG CGC GCG
Asp Leu Tyr Ala Leu Ala Leu Arg Gly Trp Gln Leu Ala Arg Leu Cys Gln Arg Ala

      2560          2570          2580          2590          2600          2610
        •             •             •             •             •             •
GAG AAC GTC TAC GTC GGC GCC CCC CTC GGC ATC ATG GAC CAG ACG GCG TCC GCC TGC
Glu Asn Val Tyr Val Gly Ala Pro Val Gly Ile Met Asp Gln Thr Ala Ser Ala Cys

          2620          2630          2640          2650          2660          2670
            •             •             •             •             •             •
TGC GAG GCG GGC ACC CCC TCT TCC TCG ACA CCC GCG ACC TCT CCC ACC GGC AGA TCC
Cys Glu Ala Gly Thr Pro Ser Ser Ser Thr Pro Ala Thr Ser Pro Ser Gly Arg Ser

              2680          2690          2700          2710          2720
                •             •             •             •             •
CCT TCG ACC TCG CCG CCG AGG GGA TGC GCC TGC TGG TCC TCG ACA CCC GGC TCA AGC
Pro Ser Thr Ser Pro Pro Arg Gly Cys Ala Cys Trp Ser Ser Thr Pro Gly Ser Ser

  2730          2740          2750           2760          2770          2780
    •             •             •             •             •             •
ACT CCC ACA GCG AGG GCC AGT ACC GCA AGC GCC GCG CGG GCT GCG ACA AGC GCC CCG
Thr Pro Thr Ala Arg Ala Ser Thr Ala Ser Ala Ala Arg Ala Ala Arg Arg Ala Pro

          2790          2800          2810          2820          2830          2840
            •             •             •             •             •             •
CGC TGC TGG GCG TCG ACG CGC TGC GAC GTG CCG TAC GCC GAC CTG GAC GCC GCG CTG
Arg Cys Trp Ala Ser Thr Arg Cys Asp Val Pro Tyr Ala Asp Leu Asp Ala Ala Leu

          2850          2860          2870          2880          2890
            •             •             •             •             •
GAG CGG CTG GGC GAC GAG GAG GAG GTG CCG CGC CTG GTC CGC CAC GTG GTG ACC GAG
Glu Arg Leu Gly Asp Glu Glu Glu Val Arg Arg Leu Val Arg His Val Val Thr Glu
```

```
       2900          2910          2920          2930          2940          2950
        •             •             •             •             •             •
    GAC CAC CGC CTC GAA CGC CTC GTC GCC CTC CTC GAG TCC GCC ACA CCC GGC CCA TCC
    Asp Glu Arg Val Glu Arg Val Val Ala Leu Leu Glu Ser Ala Thr Pro Gly Ala Ser

           2960          2970          2980          2990          3000          3010
            •             •             •             •             •             •
    GCC CCG TCC TCG TCG AGG GCC ACG CCT GCT GCG CGA CGA CTT CCG CAT CTC CTC CCC
    Ala Pro Ser Trp Ser Arg Ala Thr Pro Ala Ala Arg Arg Leu Pro His Leu Leu Pro

           3020          3030          3040          3050          3060
            •             •             •             •             •
    CCA GCT CGA CCT GGT CCT CGA CAC GGC CCT GGC CTC CGC GGC CCT CGG CGC CGG ATC
    Arg Ala Gly Pro Gly Arg Arg His Gly Pro Gly Leu Arg Gly Pro Arg Arg Arg Met

     3070          3080          3090          3100          3110          3120
      •             •             •             •             •             •
    ACC GGC GGC GGC TTC GGC GGC TCC GCG ATC GTC CTG GTG GAG GCC GCC GCG GTC GAC
    Thr Gly Gly Gly Phe Gly Gly Ser Ala Ile Val Leu Val Glu Ala Ala Ala Val Asp

           3130          3140          3150          3160          3170          3180
            •             •             •             •             •             •
    GCC GTC ACC AAG GCG GTC GAG GAC GCC TTC GCC GCC GCG GGC CTC AAG CGT CCG CGG
    Ala Val Thr Lys Ala Val Glu Asp Ala Phe Ala Ala Ala Gly Leu Lys Arg Pro Arg

           3190          3200          3210          3220          3230          3240
            •             •             •             •             •             •
    GTC TTC GAG GCG GTG CCT CGG CGC GGC GCG GCG CCT GGT CTC ACG GTC AGC CGA GCC
    Val Phe Glu Ala Val Pro Arg Arg Gly Ala Ala Pro Gly Leu Thr Val Ser Arg Ala

           3250          3260          3270          3280          3290
            •             •             •             •             •
    GCT TCA CCA GCG TGT ACT CCG TGA TCC CCG GCG GGT AGT CGG GGA TCA CGC ACA TGA
    Ala Ser Pro Ala Cys Thr Pro ---

    3300
     •
    GCT GCT AGC CGC
```

4. Molécule suivant l'une quelconque des revendications 1 à 3, qui comprend en outre une séquence d'ADN fonctionnelle étrangère liée de manière fonctionnelle à cet opéron.

5. Molécule d'ADN recombinant comprenant une unité d'expression de promoteur P2 d'opéron gal de Streptomyces lividans située sur un fragment HindIII-SphI d'environ 9kb d'ADN chromosomique de S. lividans 1326 ayant un site NruI, le site d'initiation de transcription de P2 étant situé approximativement 1,25 kb en aval du site NruI, ou de l'un quelconque de ses dérivés de Streptomyces qui agit pratiquement de la même manière que l'unité naturelle en termes de production des produits des gènes galE et galK.

6. Molécule suivant la revendication 5, dans laquelle l'unité d'expression est une unité d'expression de promoteur P2 d'opéron gal de S. coelicolor.

7. Molécule suivant la revendication 5 ou 6, qui comprend en outre une séquence d'ADN fonctionnelle étrangère liée de manière fonctionnelle à une telle unité d'expression.

8. Molécule d'ADN recombinant comprenant une région régulée par le promoteur P1 d'opéron gal de Streptomyces lividans, située sur un fragment HindIII-SphI d'environ 9kb d'ADN chromosomique de S. lividans 1326 ayant un site NruI, le site d'initiation de transcription de P1 étant situé approximativement 0,10 kb en aval du site NruI, ou l'un quelconque de ses dérivés de <u>Streptomyces</u> qui agit pratiquement de la même manière que la région naturelle en termes de production régulable des produits de gènes <u>gal</u>T, <u>gal</u>E et <u>gal</u>K.

9. Molécule suivant la revendication 8, dans laquelle la région est une région régulée par le promoteur P1 d'opéron <u>gal</u> de <u>S</u>. <u>coelicolor</u>.

10. Molécule suivant la revendication 8 ou 9, qui comprend en outre une séquence d'ADN fonctionnelle étrangère liée de manière fonctionnelle à une telle région régulée.

11. Molécule d'ADN recombinant comprenant un promoteur P2 d'opéron <u>gal</u> de <u>Streptomyces</u> <u>lividans</u> situé sur un fragment HindIII-SphI d'environ 9kb d'ADN chromosomique de <u>S</u>. <u>lividans</u> 1326 ayant un site NruI, le site d'initiation de transcription de P2 étant situé approximativement 1,25 kb en aval du site NruI, ou l'un quelconque de ses dérivés de <u>Streptomyces</u> qui agit pratiquement de la même manière que le promoteur naturel en termes de possibilité de liaison de l'ARN-polymérase à ce promoteur et de transcription d'une séquence d'ADN fonctionnelle liée de manière fonctionnelle à ce promoteur.

12. Molécule suivant la revendication 11, dans laquelle le promoteur est un promoteur P2 d'opéron <u>gal</u> de <u>S</u>. <u>coelicolor</u>.

13. Molécule suivant la revendication 11 ou 12, qui comprend en outre une séquence d'ADN fonctionnelle étrangère liée de manière fonctionnelle au promoteur P2.

14. Molécule d'ADN recombinant comprenant un promoteur P1 d'opéron <u>gal</u> de <u>Streptomyces</u> <u>lividans</u> situé sur un fragment HindIII-SphI d'environ 9kb d'ADN chromosomique de <u>S</u>. <u>lividans</u> 1326 ayant un site NruI, le site d'initiation de transcription de P1 étant situé approximativement 0,10 kb en aval du site NruI, ou l'un quelconque de ses dérivés de <u>Streptomyces</u> qui agit pratiquement de la même manière que le promoteur naturel en termes de possibilité de liaison de l'ARN-polymérase à ce promoteur et de transcription d'une séquence d'ADN fonctionnelle liée de manière fonctionnelle à ce promoteur.

15. Molécule suivant la revendication 14, dans laquelle le promoteur est un promoteur P1 d'opéron <u>gal</u> de <u>S</u>. <u>coelicolor</u>.

16. Molécule suivant la revendication 14 ou 15, qui comprend en outre une séquence d'ADN fonctionnelle étrangère liée de manière fonctionnelle au promoteur P1.

17. Micro-organisme ou cellule hôte transformé comprenant la molécule suivant l'une quelconque des revendications 4, 7, 10, 13 et 16.

18. Procédé de préparation d'un micro-organisme ou d'une cellule hôte transformé comprenant la molécule suivant l'une quelconque des revendications 4, 7, 10, 13 et 16, qui comprend la transformation d'un micro-organisme ou d'une cellule hôte approprié avec une telle molécule.

19. Vecteur d'ADN recombinant comprenant la molécule suivant l'une quelconque des revendications 4, 7, 10, 13 et 16 et comprenant en outre, facultativement, un réplicon.

20. Micro-organisme ou cellule hôte transformé comprenant le vecteur d'ADN recombinant suivant la revendication 19.

21. Procédé de préparation d'un micro-organisme ou d'une cellule hôte transformé comprenant le vecteur d'ADN recombinant suivant la revendication 19, qui comprend la transformation d'un micro-organisme ou d'une cellule hôte approprié avec un tel vecteur.

22. Procédé pour l'expression d'une séquence d'ADN fonctionnelle étrangère, qui comprend la culture d'un micro-organisme ou d'une cellule hôte transformé comprenant le vecteur d'ADN recombinant suivant la revendication 19 dans des conditions convenables de telle sorte que la séquence d'ADN fonctionnelle soit exprimée.

**23.** Procédé pour réguler l'expression d'une séquence d'ADN fonctionnelle étrangère, qui comprend la culture d'un micro-organisme ou d'une cellule hôte transformé qui contient le vecteur d'ADN recombinant suivant la revendication 19 comprenant la molécule suivant la revendication 4, 10 ou 16 dans des conditions appropriées de telle sorte que l'expression de la séquence puisse être régulée.

**Revendications pour les Etats contractants suivants : AT, ES**

**1.** Procédé de préparation d'une molécule d'ADN recombinant comprenant un opéron gal de Streptomyces lividans situé sur un fragment HindIII-SphI d'environ 9kb d'ADN chromosomique de S. lividans 1326, ou l'un quelconque de ses dérivés de Streptomyces qui agit pratiquement de la même manière que l'opéron gal de Streptomyces naturel en termes de production régulable des produits des gènes galT, galE et galK, procédé qui comprend la fusion de fragments d'ADN appropriés.

**2.** Procédé suivant la revendication 1, dans lequel l'opéron est un opéron gal de S. coelicolor.

**3.** Procédé suivant la revendication 1, dans lequel l'opéron comprend la séquence codante suivante :

```
          -120         -110         -100         -90          -80          -70
           •            •            •            •            •            •
   CTA CGC CTC CGC GTT CAC TAA TTC AAC ACT TTT GCT CAT GAA CTT TGT TTC ATT GTC


          -60          -50          -40          -30          -20
           •            •            •            •            •
   ATC TGA CAC GCC CGT GGT GGC TTC TGA TGT GTT ATC TTT GAT TGT GTT CGA TGA TTC
                                                                    ˙galP1

   -10           1           10          20          30          40
    •            •            •           •           •           •
   ACC GGC GTC CTG GTG ACT CAT GGG TGG GTG CAG AGG AGT GCG GCA GTG AAG AAG ACC
               Met Thr His Gly Trp Val Gln Arg Ser Ala Ala Val Lys Lys Thr
               galT
     50          60          70          80          90          100
      •           •           •           •           •           •
   TCG ACC CGG CTG GCC GAC GGC CGT GAG CTG GTC TAC TAC GAC CTG CGC GAC GAC ACC
   Ser Thr Arg Leu Ala Asp Gly Arg Glu Leu Val Tyr Tyr Asp Leu Arg Asp Asp Thr

        110         120         130         140         150
         •           •           •           •           •
   GTG CGC GAC GCC GTG GAC CGC CGT CCG CTG GAG CGG ACC GTC ACC ACG TCC GAG GTC
   Val Arg Asp Ala Val Asp Arg Arg Pro Leu Glu Arg Thr Val Thr Thr Ser Glu Val

   160         170         180         190         200         210
    •           •           •           •           •           •
   CGA CGC GAC CCG CTC CTC GGC GAC TCC GCG CCG TCG CGC CTC GCA CCG GCA GGC GCG
   Arg Arg Asp Pro Leu Leu Gly Asp Ser Ala Pro Ser Arg Leu Ala Pro Ala Gly Ala

        220         230         240         250         260         270
         •           •           •           •           •           •
   CAC CTA CCA TCC GCC GGC CGA CCA GTG CCC GCT GTG CCc GTC GGA CGC GGA ACG GC:
   His Leu Pro Ser Ala Gly Arg Pro Val Pro Ala Val Pro Val Gly Arg Gly Thr Al:

        280         290         300         310         320         3˙
         •           •           •           •           •           •
   GAG CGA GAT CCG GCC TAT GAC GTG GTG GTC TTC GAG AAT CGC TTT CCC TCG CTG GC
   Glu Arg Asp Pro Ala Tyr Asp Val Val Val Phe Glu Asn Arg Phe Pro Ser Leu Al
```

```
           340         350         360         370         380
           •           •           •           •           •
CGT GAC TCC GGC CGC TGC GAG GTC GTC TGC TTC ACC TCC GAC CAC GAC GCC TCC TTC
Gly Asp Ser Gly Arg Cys Glu Val Val Cys Phe Thr Ser Asp His Asp Ala Ser Phe

        390         400         410         420         430         440
         •           •           •           •           •           •
GCC GAC CTG ACC GAG GAG CAG GCC CGG CTG GTC GTC GAC GCC TGG ACG GAC CGC ACC
Ala Asp Leu Ser Glu Glu Gln Ala Arg Leu Val Val Asp Ala Trp Thr Asp Arg Thr

           450         460         470         480         490         500
            •           •           •           •           •           •
TCC GAG CTG TCC CAT CTG CCC TCC GTT GAA CAG GTC TTC TGC TTC GAG AAC CGG GGC
Ser Glu Leu Ser His Leu Pro Ser Val Glu Gln Val Phe Cys Phe Glu Asn Arg Gly

              510         520         530         540         550
               •           •           •           •           •
GCC GAG ATC GGG GTC ACG CTG GGT CAC CCG CAC GGG CAG ATC TAC GCC TAC CCG TTC
Ala Glu Ile Gly Val Thr Leu Gly His Pro His Gly Gln Ile Tyr Ala Tyr Pro Phe

  560         570         580         590         600         610
   •           •           •           •           •           •
ACC ACC CCC CGC ACC GCC CTG ATG CTC CGT TCA CTC GCC GCC CAC AAG GAC GCG ACG
Thr Thr Pro Arg Thr Ala Leu Met Leu Arg Ser Leu Ala Ala His Lys Asp Ala Thr

     620         630         640         650         660         670
      •           •           •           •           •           •
GGC GGG GGG AAC CTG TTC GAC TCC GTC CTG GAG GAG GAG CTG GCC GGT GAG CGC GTC
Gly Gly Gly Asn Leu Phe Asp Ser Val Leu Glu Glu Glu Leu Ala Gly Glu Arg Val

        680         690         700         710         720
         •           •           •           •           •
GTC CTC GAG GGT GAG CAC TGG GCC GCC TTC GTC GCC TAC GGC GCC CAC TGG CCG TAC
Val Leu Glu Gly Glu His Trp Ala Ala Phe Val Ala Tyr Gly Ala His Trp Pro Tyr

730         740         750         760         770         780
 •           •           •           •           •           •
GAG GTG CAC CTC TAC CCC AAG CGG CGG GTG CCC GAT CTG CTC GGG CTC GAC GAG GCG
Glu Val His Leu Tyr Pro Lys Arg Arg Val Pro Asp Leu Leu Gly Leu Asp Glu Ala

        790         800         810         820         830         840
         •           •           •           •           •           •
GCT CGC ACA GAA TTC CCC AAG GTC TAC CTG GAG CTG CTG AGG CGT TTC GAC CGG ATC
Ala Arg Thr Glu Phe Pro Lys Val Tyr Leu Glu Leu Leu Arg Arg Phe Asp Arg Ile
```

```
          850          860          870          880          890          900
           •            •            •            •            •            •
TTC GGC GAG GGC GAG CCC CCG ACC CCC TAC ATC GCC GCC TCC CAC CAG GCG CCC TTC
Phe Gly Glu Gly Glu Pro Pro Thr Pro Tyr Ile Ala Ala Trp His Gln Ala Pro Phe

                     9:           920          930          940          950
                      •            •            •            •            •
GGC CAG CTC GAG TTC GAG GGT GTC ACG CGC GAC GAC TTC GCC CTC CAC CTG GAA CTT
Gly Gln Leu Glu Phe Glu Gly Val Thr Arg Asp Asp Phe Ala Leu His Leu Glu Leu

    960          970          980          990          1000         1010
     •            •            •            •            •            •
TTC ACT TCC GCC GTA CGT CCG GCA AGC TGA AGT TCC TCG CGC GCT CCG AAT CCG GCA
Phe Thr Ser Ala Val Arg Pro Ala Ser ---              ¯galP2

    1020         1030         1040         1050         1060         1070
     •            •            •            •            •            •
    TGAACG TGTTCATCAA CGACGTACCC CCGGAGCGCG CGGCCGAGCG ACTGCGAGAG GTAGCGAG


1080         1090         1100         1110         1120         1130
 •            •            •            •            •            •
TTC ATC AGC GGG AAG TAC CTG GTG ACA GGT GGT GCC GGA TAC CTC GGC AGC GTC GTC
Met Ser Gly Lys Tyr Leu Val Thr Gly Gly Ala Gly Tyr Val Gly Ser Val Val
galE
    1140         1150         1160         1170         1180         1190
     •            •            •            •            •            •
GCC CAG CAC TTG GTG GAG GCG GGG AAC GAG GTC GTG GTC CTG CAC AAT CTG TCC ACC
Ala Gln His Leu Val Glu Ala Gly Asn Glu Val Val Val Leu His Asn Leu Ser Thr

        1200         1210         1220         1230         1240
         •            •            •            •            •
GGC TTC CGT GAG GTG TGC CGG CGG GTG CCT CGT TCG TCG AGG CGA CAT CCG GGA CGC
Gly Phe Arg Glu Val Cys Arg Arg Val Pro Arg Ser Ser Arg Arg His Pro Gly Arg

1250         1260         1270         1280         1290         1300
 •            •            •            •            •            •
CGC CAA GTC CGT GGA CGG CTC TCG TTC GAC GGC GTG CTC CAC TTC GCC GCC TTC TCC
Arg Gln Val Arg Gly Arg Leu Ser Phe Asp Gly Val Leu His Phe Ala Ala Phe Ser

    1310         1320         1330         1340         1350         1360
     •            •            •            •            •            •
CAG GTC GGC GAG TCC GTC GTG AAG CCC GAG AAG TAC TGG GAC AAC AAC GTC GGT GGC
Gln Val Gly Glu Ser Val Val Lys Pro Glu Lys Tyr Trp Asp Asn Asn Val Gly Gly
```

```
         1370          1380          1390          1400          1410          1420
          •             •             •             •             •             •
ACC ATG GCG CTG CTG GAG GCC ATG CGG GGC GCG GGT GTG CGG CGG CTC GTC TTC TCC
Thr Met Ala Leu Leu Glu Ala Met Arg Gly Ala Gly Val Arg Arg Leu Val Phe Ser

                1430          1440          1450          1460          1470
                 •             •             •             •             •
TCC ACG GCC GCC ACG TAC GGC GAG CCC GAG CAG GTT CCC ATC GTC GAC TCC GCG CCG
Ser Thr Ala Ala Thr Tyr Gly Glu Pro Glu Gln Val Pro Ile Val Glu Ser Ala Pro

1480          1490          1500          1510          1520          1530
 •             •             •             •             •             •
ACG AGG CCC ACC AAT CCG TAC GGC GCC TCG AAG CTC GCC GTC GAC CAC ATG ATC ACC
Thr Arg Pro Thr Asn Pro Tyr Gly Ala Ser Lys Leu Ala Val Asp His Met Ile Thr

      1540          1550          1560          1570          1580          1590
       •             •             •             •             •             •
GGC GAG GCC GCG GCC CAC GGC CTC GGC GCC GTC TCC GTC CCG TAC TTC AAC GTC GCG
Gly Glu Ala Ala Ala His Gly Leu Gly Ala Val Ser Val Pro Tyr Phe Asn Val Ala

            1600          1610          1620          1630          1640
             •             •             •             •             •
GGC GCC TAC GGG GAG TAC GGC GAG CGC CAC GAC CCC GAG TCG CAT CTC ATT CCC CTG
Gly Ala Tyr Gly Glu Tyr Gly Glu Arg His Asp Pro Glu Ser His Leu Ile Pro Leu

1650          1660          1670          1680          1690          1700
 •             •             •             •             •             •
GTC CTT CAA GTG GCG CAG GGC AGG CGG GAG GCC ATC TCC GTC TAC GGC GAC GAC TAC
Val Leu Gln Val Ala Gln Gly Arg Arg Glu Ala Ile Ser Val Tyr Gly Asp Asp Tyr

      1710          1720          1730          1740          1750          1760
       •             •             •             •             •             •
CCC ACC CCG GAC CGA CCT GTG TGC GCG ACT ACA TCC ACG TCG CCG ACC TGG CCG AGG
Pro Thr Pro Asp Arg Pro Val Cys Ala Thr Thr Ser Thr Ser Pro Thr Trp Pro Arg

            1770          1780          1790          1800          1810
             •             •             •             •             •
CCC ACC TGC TGG CCG TGC GCC GCC CCC CCG GGC GAG CAC CTC ATC TGC AAC CTC GGC
Pro Thr Cys Trp Pro Cys Ala Ala Ala Pro Gly Glu His Leu Ile Cys Asn Leu Gly

1820          1830          1840          1850          1860          1870
 •             •             •             •             •             •
AAC GGC AAC GGC TTC TCC GTC CGC GAG GTC GTC GAG ACC GTG CGG CGG GTG ACG GGC
Asn Gly Asn Gly Phe Ser Val Arg Glu Val Val Glu Thr Val Arg Arg Val Thr Gly
```

```
        1880          1890          1900          1910          1920          1930
          .             .             .             .             .             .
CAT CCG ATC CCC GAG ATC ATG GCC CCC CGC CGC GGG CGC GAC CCG GCG GTC CTG GTC
His Pro Ile Pro Glu Ile Met Ala Pro Arg Arg Gly Arg Asp Pro Ala Val Leu Val

         1940          1950          1960          1970          1980          1990
           .             .             .             .             .             .
GCG TCC GCC GGC ACC GCC CGC GAG AAG CTC GGC TGG AAC CCG TCC CGC GCG GAC CTC
Ala Ser Ala Gly Thr Ala Arg Glu Lys Leu Gly Trp Asn Pro Ser Arg Ala Asp Leu

           2000          2010          2020          2030          2040
             .             .             .             .             .
GCC ATC GTC TCC GAC GCG TGG GAG TTC CCG CAG CGG CGC GCG GGC CAG TAG  TA
Ala Ile Val Ser Asp Ala Trp Glu Leu Pro Gln Arg Arg Ala Gly Gln ---

         2050          2060          2070          2080          2090          2100
           .             .             .             .             .             .
ACC GCA GTT ACC GGA AAG GCG ACG GGT CAC GGC ATG GGC GAG GCT GTC GGG GAA CCG
                                              Met Gly Glu Ala Val Gly Glu Pro
                                              galK
          2110          2120          2130          2140          2150
            .             .             .             .             .
TCG GCC AGC GGT TCC GGG AGC TGT ACG GGG CGG AGC CGG AGG GGG TGT GGG CGC CGA
Ser Ala Ser Gly Ser Gly Ser Cys Thr Gly Arg Ser Arg Arg Gly Cys Gly Arg Arg

2160          2170          2180          2190          2200          2210
  .             .             .             .             .             .
GCG GGC CGG GAG AAC CTC ATC GGG GAG CAC ACC GAC TAC AAC GAC GCG TTC GTC ATG
Ala Gly Arg Glu Asn Leu Ile Gly Glu His Thr Asp Tyr Asn Asp Gly Phe Val Met

    2220          2230          2240          2250          2260          2270
      .             .             .             .             .             .
CCT TCG CCC TGC CGC ACC AGG TCG CGG CCG TCT CCC GGC GCG AAC GAC GGC ATC CTG
Pro Ser Pro Cys Arg Thr Arg Ser Arg Pro Ser Pro Gly Ala Asn Asp Gly Ile Leu

          2280          2290          2300          2310          2320
            .             .             .             .             .
CGC CTG CAC TCG GCC GAC GTC GAC GCC GAC CCG GTC GAG CTG CGC GTC GCC GAC CTG
Arg Leu His Ser Ala Asp Val Asp Ala Asp Pro Val Glu Leu Arg Val Ala Asp Leu

2330          2340          2350          2360          2370          2380
  .             .             .             .             .             .
GCC CCC GCG TCG GAC AAG TCC TGG ACG GCG TAC CCC TCG GGC GTC CTG TGG GCG CTC
Ala Pro Ala Ser Asp Lys Ser Trp Thr Ala Tyr Pro Ser Gly Val Leu Trp Ala Leu
```

```
        2390          2400          2410          2420          2430          2440
         •             •             •             •             ••            •
CGC GAG GCC GGA CAC GAG CTG ACC GGC GCC GAC GTC CAC CTG GCC TCG ACC GTC CCG
Arg Glu Ala Gly His Glu Leu Thr Gly Ala Asp Val His Leu Ala Ser Thr Val Pro

           2450          2460          2470          2480          2490
            •             •             •             •             •
TCC GCG GCG GGC CTC TCC TCC TCC GCG GCC CTC GAG GTC CGT CCC CTC GCG ATG AAC
Ser Gly Ala Gly Leu Ser Ser Ser Ala Ala Leu Glu Val Arg Pro Leu Ala Met Asn

 2500          2510          2520          2530          2540          2550
  •             •             •             •             •             •
GAC CTC TAC GCC CTC GCC CTC CGC GGC TGG CAG CTC GCC CGG CTG TGC CAG CGC GCG
Asp Leu Tyr Ala Leu Ala Leu Arg Gly Trp Gln Leu Ala Arg Leu Cys Gln Arg Ala

    2560          2570          2580          2590          2600          2610
     •             •             •             •             •             •
GAG AAC GTC TAC GTC GGC GCC CCC GTC GGC ATC ATG GAC CAG ACG GCG TCC GCC TGC
Glu Asn Val Tyr Val Gly Ala Pro Val Gly Ile Met Asp Gln Thr Ala Ser Ala Cys

       2620          2630          2640          2650          2660          2670
        •             •             •             •             •             •
TGC GAG GCG GGC ACG CCC TCT TCC TCG ACA CCC GCG ACC TCT CCC AGC GGC AGA TCC
Cys Glu Ala Gly Thr Pro Ser Ser Ser Thr Pro Ala Thr Ser Pro Ser Gly Arg Ser

          2680          2690          2700          2710          2720
           •             •             •             •             •
CCT TCG ACC TCG CCG CCG AGG GGA TGC GCC TGC TGG TCC TCG ACA CCC GGC TCA AGC
Pro Ser Thr Ser Pro Pro Arg Gly Cys Ala Cys Trp Ser Ser Thr Pro Gly Ser Ser

 2730          2740          2750         ,2760          2770          2780
  •             •             •             •             •             •
ACT CCC ACA GCC AGG GCG AGT ACC GCA AGC GCC GCG CGC CCT GCC AGA AGG GCG CCC
Thr Pro Thr Ala Arg Ala Ser Thr Ala Ser Ala Ala Arg Ala Ala Arg Arg Ala Pro

          2790          2800          2810          2820          2830          2840
           •             •             •             •             •             •
CGC TGC TGG GCC TCG ACG CGC TGC GAC GTC CCG TAC GCC GAC CTC GAC GCC GCC CTC
Arg Cys Trp Ala Ser Thr Arg Cys Asp Val Pro Tyr Ala Asp Leu Asp Ala Ala Leu

          2850          2860          2870          2880          2890
           •             •             •             •             •
GAG CGG CTG GGC GAC GAG GAG GAG GTG CGC CGC CTC GTC CGG CAC GTC GTC ACC GAG
Glu Arg Leu Gly Asp Glu Glu Glu Val Arg Arg Leu Val Arg His Val Val Thr Glu
```

```
      2900          2910          2920          2930          2940          2950
       •            •            •            •            •            •
GAC GAG CCC GTC GAA CGG GTC GTC GCC CTC CTC GAG TCG GCG ACA CCC GGC GCA TCG
Asp Glu Arg Val Glu Arg Val Val Ala Leu Leu Glu Ser Ala Thr Pro Gly Ala Ser

          2960          2970          2980          2990          3000          3010
           •            •            •            •            •            •
GCC CCG TCC TGG TCC AGG GCC ACG CCT GCT GCC CGA CGA CTT CCG CAT CTC CTG CCC
Ala Pro Ser Trp Ser Arg Ala Thr Pro Ala Ala Arg Arg Leu Pro His Leu Leu Pro

             3020          3030          3040          3050          3060
              •            •            •            •            •
CGA CCT GGA CCT GGT CGT CGA CAC GGC CCT GGC CTC CGC GGC CCT CGC CGC CGG ATC
Arg Ala Gly Pro Gly Arg Arg His Gly Pro Gly Leu Arg Gly Pro Arg Arg Arg Met

3070          3080          3090          3100          3110          3120
 •            •            •            •            •            •
ACC GGC GGC GGC TTC GGC GGC TCG GCG ATC GTC CTC GTC GAG GCC GCC GCG GTG GAC
Thr Gly Gly Gly Phe Gly Gly Ser Ala Ile Val Leu Val Glu Ala Ala Ala Val Asp

          3130          3140          3150          3160          3170          3180
           •            •            •            •            •            •
GCC GTC ACC AAG GCG GTC GAG GAC GCC TTC GCC GCG GCG GGC CTC AAG CGT CCG CGG
Ala Val Thr Lys Ala Val Glu Asp Ala Phe Ala Ala Ala Gly Leu Lys Arg Pro Arg

             3190          3200          3210          3220          3230          3240
              •            •            •            •            •            •
GTG TTC GAG GCG GTG CCT CGG CGG GGC GGC GGG CCT GGT CTG ACG GTC AGC CGA GCC
Val Phe Glu Ala Val Pro Arg Arg Gly Ala Ala Pro Gly Leu Thr Val Ser Arg Ala

             3250          3260          3270          3280          3290
              •            •            •            •            •
GCT TCA CCA GCG TGT ACT CCG TGA TCC CCG GCG GGT AGT CGG GGA TCA CGC ACA TGA
Ala Ser Pro Ala Cys Thr Pro ---

3300
 •
GCT GCT ACC CGC
```

qui comprend la transfomation d'un micro-organisme ou d'une cellule hôte approprié avec une telle molécule.

4. Procédé suivant l'une quelconque des revendications 1 à 3, dans lequel la molécule comprend en outre une séquence d'ADN fonctionnelle étrangère liée de manière fonctionnelle à un tel opéron.

5. Procédé de préparation d'une molécule d'ADN recombinant comprenant une unité d'expression de promoteur P2 d'opéron gal de Streptomyces lividans située sur un fragment HindIII-SpHI d'environ 9kb d'ADN chromosomique de S. lividans 1326 comprenant un site NruI, le site d'initiation de transcription de P2 étant situé approximativement 1,25 kb en aval du site NruI, ou l'un quelconque de ses dérivés de Streptomyces qui agit pratiquement de la même manière que l'unité naturelle en termes de production des produits de gènes galE et galK, procédé qui comprend la fusion de fragments d'ADN appropriés.

6. Procédé suivant la revendication 5, dans lequel l'unité d'expression est une unité d'expression de promoteur P2 d'opéron gal de S. coelicolor.

7. Procédé suivant la revendication 5 ou 6, dans lequel la molécule comprend en outre une séquence d'ADN fonctionnelle étrangère liée de manière fonctionnelle à une telle unité d'expression.

8. Procédé de préparation d'une molécule d'ADN recombinant comprenant une région régulée par le promoteur P1 d'opéron gal de Streptomyces lividans, située sur un fragment HindIII-SphI d'environ 9kb d'ADN chromosomique de S. lividans 1326 comprenant un site NruI, le site d'initiation de transcription de P1 étant situé approximativement 0,10 kb en aval du site NruI, ou l'un quelconque de ses dérivés de Streptomyces qui agit pratiquement de la même manière que la région naturelle en termes de production régulable des produits des gènes galT, galE et galK, procédé qui comprend la fusion de fragments d'ADN appropriés.

9. Procédé suivant la revendication 8, dans lequel la région est une région régulée par le promoteur P1 d'opéron gal de S. coelicolor.

10. Procédé suivant la revendication 8 ou 9, dans lequel la molécule comprend en outre une séquence d'ADN fonctionnelle étrangère liée de manière fonctionnelle à une telle région régulée.

11. Procédé de préparation d'une molécule d'ADN recombinant comprenant un promoteur P2 d'opéron gal de Streptomyces lividans situé sur un fragment HindIII-SphI d'environ 9kb d'ADN chromosomique de S. lividans 1326 comprenant un site NruI, le site d'initiation de transcription de P2 étant situé approximativement 1,25 kb en aval du site NruI, ou l'un quelconque de ses dérivés de Streptomyces qui agit pratiquement de la même manière que le promoteur naturel en termes de possibilité de liaison de l'ARN-polymérase à ce promoteur et de transcription d'une séquence d'ADN fonctionnelle liée de manière fonctionnelle à un tel promoteur, procédé qui comprend la fusion de fragments d'ADN appropries.

12. Procédé suivant la revendication 11, dans lequel le promoteur est un promoteur P2 d'opéron gal de S. coelicolor.

13. Procédé suivant la revendication 11 ou 12, dans lequel la molécule comprend en outre une séquence d'ADN fonctionnelle étrangère liée de manière fonctionnelle au promoteur P2.

14. Procédé de préparation d'une molécule d'ADN recombinant comprenant un promoteur P1 d'opéron gal de Streptomyces lividans situé sur un fragment HindIII-SphI d'environ 9kb d'ADN chromosomique de S. lividans 1326 comprenant un site NruI, le site d'initiation de transcription de P1 étant situé approximativement 0,10 kb en aval du site NruI, ou l'un quelconque de ses dérivés de Streptomyces qui agit pratiquement de la même manière que le promoteur naturel en termes de possibilité de liaison de l'ARN-polymérase à ce promoteur et de transcription d'une séquence d'ADN fonctionnelle liée de manière fonctionnelle à ce promoteur, procédé qui comprend la fusion de fragments d'ADN appropries.

15. Procédé suivant la revendication 14, dans lequel le promoteur est un promoteur P1 d'opéron gal de S. coelicolor.

16. Procédé suivant la revendication 14 ou 15, dans lequel la molécule comprend en outre une séquence d'ADN fonctionnelle étrangère liée de manière fonctionnelle au promoteur P1.

17. Procédé de préparation d'un micro-organisme ou d'une cellule hôte transformé comprenant la molécule suivant l'une quelconque des revendications 4, 7, 10, 13 et 16, qui comprend la transformation d'un micro-organisme ou d'une cellule hôte approprié avec une telle molécule.

18. Procédé de préparation d'un vecteur d'ADN recombinant comprenant la molécule suivant l'une quelconque des revendications 4, 7, 10, 13 et 16 et comprenant en outre, facultativement, un réplicon, procédé qui comprend la fusion de fragments d'ADN appropriés.

19. Procédé de préparation d'un micro-organisme ou d'une cellule hôte transformé comprenant le vecteur d'ADN recombinant suivant la revendication 18, qui comprend la transformation d'un micro-organisme hôte approprié avec ce vecteur.

20. Procédé pour l'expression d'une séquence d'ADN fonctionnelle étrangère, qui comprend la culture d'un micro-organisme ou d'une cellule hôte transformé comprenant le vecteur d'ADN recombinant suivant la revendication 18 dans des conditions convenables de telle sorte que la séquence d'ADN fonctionnelle soit exprimée.

**21.** Procédé pour réguler l'expression d'une séquence d'ADN fonctionnelle étrangère, qui comprend la culture d'un micro-organisme ou d'une cellule hôte transformé qui contient le vecteur d'ADN recombinant suivant la revendication 18 comprenant la molécule suivant la revendication 4, 10 ou 16 dans des conditions appropriées de telle sorte que l'expression de la séquence puisse être régulée.

# FIG. I

FIG. 2

EP 0 235 112 B1

# FIG. 3

S. lividans galoperon

S. coelicolor